# EUROPEAN PATENT APPLICATION

(11) **EP 3 909 982 A1**
(43) Date of publication of application: **17.11.2021**
(21) Application number: 20738698.8
(22) Date of filing: 13.01.2020
(51) Int. Cl.: C07K 16/28, C07K 19/00, C12N 15/13

(54) **CD73 ANTIBODY, PREPARATION METHOD THEREFOR AND APPLICATION THEREOF**

(30) Priority: 11.01.2019 CN 201910028562
(71) Applicant: Shanghai Pharmaexplorer Co., Ltd., Shanghai 201210 (CN)
(72) Inventor: WANG, Dongxu, Shanghai 201210 (CN); DUAN, Qing, Shanghai 201210 (CN); LIU, Lile, Shanghai 201210 (CN); YANG, Tatchi Teddy, Shanghai 201210 (CN); LIU, Hu, Shanghai 201210 (CN); HAN, Ye, Shanghai 201210 (CN); XIE, Rongrong, Shanghai 201210 (CN); SHAO, Xiaohui, Shanghai 201210 (CN); WANG, Peng, Shanghai 201210 (CN); ZHONG, Qin, Shanghai 201210 (CN); HUANG, Yajun, Shanghai 201210 (CN); WU, Jian, Shanghai 201210 (CN); WANG, Meiling, Shanghai 201210 (CN); WANG, Yuandong, Shanghai 201210 (CN)
(74) Representative: Cabinet Laurent & Charras
(86) International application number: PCT/CN2020/071838
(87) International publication number: WO 2020/143836

(57) **Abstract**

An antibody targeting CD73, a preparation method therefor and a use thereof. The provided monoclonal antibody can bind to a CD73 antigen with high specificity, and has high affinity and significant antitumor activity.

## Description

### Technical field

The invention belongs to the field of biomedicine, in particular to a CD73 antibody and the preparation method and application thereof.

### Background

In recent years, tumor immunotherapy has become the focus in the field of tumor therapy, among them, therapeutic monoclonal antibodies against immune checkpoints have shown anti-tumor activity in the treatment of some tumor types such as melanoma and non-small cell lung cancer. Immune checkpoint antibodies targeting cytotoxic T lymphocyte-associated antigen-4 (CTLA-4) and programmed cell death 1/programmed cell death ligand 1 (PD-1/PD-L1) have been approved by FDA.

However, the low response rate of single drug is the main problem of existing tumor immunotherapy. In 2000, CTLA4 antibody was tested clinically, showing toxicity (causing tissue-specific inflammatory reaction) and low response rate. The most obvious clinical effect occurred in the treatment of melanoma, but the objective response rate was only 15%. In the clinical trials of PD-1 and PD-L1, Hodgkin lymphoma, Merkel cell carcinoma and connective tissue proliferative melanoma have the highest objective response rate, reaching 50-90%; the response rate of melanoma treatment is 35-40%; the response rate of non-small cell lung cancer, head and neck cancer, bladder cancer, renal cancer and hepatocellular carcinoma is only 15-25%. Tumor is a multi-channel and multi-target disease, and the objective response rate of a single therapeutic drug is low, which is probably due to the fact that tumor cells choose other compensatory pathways to meet the growth when a certain signal pathway is inhibited by drugs. In order to improve the existing therapeutic effect and reduce the dosage of toxic antibodies, tumor immune combination therapy will become an important development trend.

Tumors use various means to escape immune elimination, so it is necessary to better understand the immunosuppression of tumor microenvironment. In tumor microenvironment, there is low oxygen content, lacking nutrients, and usually acidic pH value. Tumor cells have a variety of regulatory mechanisms to adapt to the harsh living environment, one of the most important ways is to change purine metabolism by up-regulating the expression of CD73 (exo-5'-nucleotidase). CD73 is a 70KD protein, which forms a dimer with non-covalent bonds, and its C-terminal is anchored to the cell membrane through glycosyl phosphatidylinositol (GPI). CD73 dephosphorylates extracellular monophosphate nucleotide (AMP) to produce adenosine. Extracellular adenosine binds to a variety of cell surface-specific adenosine receptors (A1, A2A, A2B and A3) to activate adenosine pathway, which play an important role in immunosuppression and angiogenesis.

Studies have shown that CD73 is highly expressed on the surface of various tumor cells, including bladder cancer, blood cancer, glioma, malignant glioma, melanoma, ovarian cancer, colon cancer and breast cancer. Up-regulation of CD73 expression is associated with cancer cell proliferation, metastasis, angiogenesis, and shorter patient survival. Therefore, CD73 can be used as a new drug target and biomarker to treat cancer.

### Summary of the invention

In order to overcome the current lack of safe and highly specific CD73 antibodies, the present invention provides a CD73 antibody with high affinity and strong specificity, and a preparation method and application thereof.

In the first aspect of the present invention, there is provided a heavy chain variable region of an antibody having complementary determining regions or CDRs selected from the group consisting of:
VH-CDR1 as shown in SEQ ID NO. 10n +3,
VH-CDR2 as shown in SEQ ID NO. 10n +4, and
VH-CDR3 as shown in SEQ ID NO. 10n +5;
wherein, each n is independently 0, 1, 2, 3, 4, 5, 6, 7, 8, or 9;
wherein any one of the above amino acid sequences further comprises a derivative sequence that is optionally with at least one amino acid added, deleted, modified and/or substituted, and is capable of retaining the binding affinity to CD73.

In another preferred embodiment, the heavy chain variable region has the amino acid sequence as shown in SEQ ID NO. 10 n +1, wherein n is 0, 1, 2, 3, 4, 5, 6, 7, 8, or 9.

In another preferred embodiment, the heavy chain variable region has the amino acid sequence as shown in SEQ ID NO: 1 or 101.

In another preferred embodiment, the heavy chain variable region has the amino acid sequence as shown in SEQ ID NO: 11.

In another preferred embodiment, the heavy chain variable region has the amino acid sequence as shown in SEQ ID NO: 21.

In the second aspect of the present invention, there is provided a heavy chain of an antibody having heavy chain variable regions according to the first aspect of the present invention.

In another preferred embodiment, the heavy chain further comprises a heavy chain constant region.

In another preferred embodiment, the heavy chain constant region is of human or murine origin.

In another preferred embodiment, the heavy chain constant region is a human antibody heavy chain IgG1 constant region.

In another preferred embodiment, the heavy chain constant region is a human antibody heavy chain IgG1-TM constant region.

In another preferred embodiment, the IgG1-TM constant region is IgG1 and contains three site mutations of L234F, L235E and P331S.

In the third aspect of the present invention, there is provided a light chain variable region of an antibody having complementary determining regions or CDRs selected from the group consisting of:
VL-CDR1 as shown in SEQ ID NO. 10n +8,
VL-CDR2 as shown in SEQ ID NO. 10n +9, and
VL-CDR3 as shown in SEQ ID NO. 10n +10;
wherein, each n is independently 0, 1, 2, 3, 4, 5, 6, 7, 8, or 9;
wherein any one of the above amino acid sequences further comprises a derivative sequence that is optionally with at least one amino acid added, deleted, modified and/or substituted, and is capable of retaining the binding affinity to CD73.

In another preferred example, the light chain variable region has the amino acid sequence as shown in SEQ ID NO. 10n +6 or SEQ ID NO. 103, wherein n is 0, 1, 2, 3, 4, 5, 6, 7, 8, or 9.

In another preferred embodiment, the light chain variable region has the amino acid sequence as shown in SEQ ID NO: 6 or 103.

In another preferred embodiment, the light chain variable region has the amino acid sequence as shown in SEQ ID NO: 16.

In another preferred embodiment, the light chain variable region has the amino acid sequence shown in SEQ ID NO: 26.

In the fourth aspect of the present invention, there is provided a light chain of an antibody having light chain variable regions according to the third aspect of the present invention.

In another preferred embodiment, the light chain further comprises a light chain constant region.

In another preferred embodiment, the light chain constant region is of human or murine origin.

In another preferred embodiment, the light chain constant region is human antibody light chain kappa constant region.

In the fifth aspect of the present invention, there is provided an antibody having:
(1) the heavy chain variable region according to the first aspect of the present invention; and/or
(2) the light chain variable region according to the third aspect of the present invention.

Alternatively, the antibody has: the heavy chain according to the second aspect of the present invention; and/or the light chain according to the fourth aspect of the present invention.
wherein any one of the above amino acid sequences further comprises a derivative sequence that is optionally with at least one amino acid added, deleted, modified and/or substituted, and is capable of retaining the binding affinity to CD73.

In another preferred embodiment, the amino acid sequence of any one of the above-mentioned CDRs comprises a derivative CDR sequence with 1, 2 or 3 amino acids added, deleted, modified and/or substituted, and the derivative antibody consisting of VH and VL containing the derivative CDR sequence is capable of retaining the binding affinity to CD73.

In another preferred embodiment, the ratio (F1/F0) of the affinity F1 for the derivative antibody binding to CD73 to the affinity F0 for the corresponding non-derived antibody binding to CD73 is 0.5-2, preferably 0.7-1.5, and more preferably 0.8-1.2.

In another preferred embodiment, the number of added, deleted, modified and/or substituted amino acids is 1-5 (such as 1-3, preferably 1-2, more preferably 1).

In another preferred embodiment, the derivative sequence with at least one amino acid added, deleted, modified, and/or substituted, which can retain the binding affinity to CD73, is an amino acid sequence having a homology or sequence identity of at least 96%.

In another preferred embodiment, the antibody further comprises a heavy chain constant region and/or a light chain constant region.

In another preferred embodiment, the heavy chain constant region is of human, and/or the light chain constant region is of human.

In another preferred embodiment, the heavy chain constant region is a human antibody heavy chain IgG1 constant region, and the light chain constant region is a human antibody light chain kappa constant region.

In another preferred embodiment, the heavy chain constant region is a human antibody heavy chain IgG1-TM constant region, and the light chain constant region is a human antibody light chain kappa constant region.

In another preferred embodiment, the heavy chain variable region of the antibody further comprises a human framework region, and/or the light chain variable region of the antibody further comprises a human framework region.

In another preferred embodiment, the heavy chain variable region of the antibody further comprises a murine framework region, and/or the light chain variable region of the antibody further comprises a murine framework region.

In another preferred embodiment, the antibody is selected from the group consisting of an animal-derived antibody, a chimeric antibody, a humanized antibody, a fully human antibody, or a combination thereof.

In another preferred embodiment, the ratio (Z1/Z0) of the immunogenicity Z1 of the chimeric antibody in humans to the immunogenicity Z0 of a non-chimeric antibody (e.g., a murine antibody) in humans is from 0 to 0.5, preferably from 0 to 0.2, and more preferably from 0 to 0.05 (e.g., 0.001 to 0.05).

In another preferred embodiment, the antibody is a partially or fully humanized, or a fully human monoclonal antibody.

In another preferred embodiment, the antibody is a double-chain antibody or a single-chain antibody.

In another preferred embodiment, the antibody is a full-length protein of an antibody, or an antigen binding fragment.

In another preferred embodiment, the antibody is a bispecific antibody or a multispecific antibody.

In another preferred embodiment, the antibody is in the form of a drug conjugate.

In another preferred embodiment, the antibody has one or more characteristics selected from the group consisting of:
(a) inhibiting the enzyme activity of CD73;
(b) promoting endocytosis of CD73;
(c) restoring the proliferation of T cells mediated by AMP;
(d) improving tumor microenvironment and activating tumor-specific immune response
(e) inhibiting the migration or metastasis of tumor cells;
(b) inhibiting tumor growth.

In another preferred embodiment, the antibody has a heavy chain variable region according to the first aspect of the invention and a light chain variable region according to the third aspect of the invention;
wherein, the heavy chain variable region and the light chain variable region comprise CDRs selected from the group consisting of:

| VH-CDR1 | VH-CDR2 | VH-CDR3 | VL-CDR1 | VL-CDR2 | VL-CDR3 |
|---|---|---|---|---|---|
| Sequence number | Sequence number | Sequence number | Sequence number | Sequence number | Sequence number |
| 3 | 4 | 5 | 8 | 9 | 10 |
| 13 | 14 | 15 | 18 | 19 | 20 |
| 23 | 24 | 25 | 28 | 29 | 30 |
| 33 | 34 | 35 | 38 | 39 | 40 |
| 43 | 44 | 45 | 48 | 49 | 50 |
| 53 | 54 | 55 | 58 | 59 | 60 |
| 63 | 64 | 65 | 68 | 69 | 70 |
| 73 | 74 | 75 | 78 | 79 | 80 |
| 83 | 84 | 85 | 88 | 89 | 90 |
| 93 | 94 | 95 | 98 | 99 | 100 |

wherein any one of the above amino acid sequences further comprises a derivative sequence that is optionally with at least one amino acid added, deleted, modified and/or substituted, and is capable of retaining the binding affinity to CD73.

In another preferred embodiment, the antibody has a heavy chain variable region according to the first aspect of the invention and a light chain variable region according to the third aspect of the invention; wherein,
the heavy chain variable region comprises the following three complementary determining regions or CDRs:
   VH-CDR1 as shown in SEQ ID NO. 3,
   VH-CDR2 as shown in SEQ ID NO. 4, and
   VH-CDR3 as shown in SEQ ID NO. 5;
the light chain variable region comprises the following three complementary determining regions or CDRs:
   VL-CDR1 as shown in SEQ ID NO. 8,
   VL-CDR2 as shown in SEQ ID NO. 9, and
   VL-CDR3 as shown in SEQ ID NO. 10;
or
the heavy chain variable region comprises the following three complementary determining regions or CDRs:
   VH-CDR1 as shown in SEQ ID NO. 13,
   VH-CDR2 as shown in SEQ ID NO. 14, and
   VH-CDR3 as shown in SEQ ID NO. 15;
the light chain variable region comprises the following three complementary determining regions or CDRs:
   VL-CDR1 as shown in SEQ ID NO. 18,
   VL-CDR2 as shown in SEQ ID NO. 19, and
   VL-CDR3 as shown in SEQ ID NO. 20;
or
the heavy chain variable region comprises the following three complementary determining regions or CDRs:
   VH-CDR1 as shown in SEQ ID NO. 23,
   VH-CDR2 as shown in SEQ ID NO. 24, and
   VH-CDR3 as shown in SEQ ID NO. 25;
the light chain variable region comprises the following three complementary determining regions or CDRs:
   VL-CDR1 as shown in SEQ ID NO. 28,
   VL-CDR2 as shown in SEQ ID NO. 29, and
   VL-CDR3 as shown in SEQ ID NO. 30.

In another preferred embodiment, the heavy chain variable region of the antibody contains the amino acid sequence as shown in SEQ ID NO. 1, 11, 21, 31, 41, 51, 61, 71, 81, 91 or 101; and/or the light chain variable region of the antibody contains the amino acid sequence as shown in SEQ ID NO. 6, 16, 26, 36, 46, 56, 66, 76, 86, 96, or 103.

In another preferred embodiment, the heavy chain variable region of the antibody contains the amino acid sequence as shown in SEQ ID NO. 1; and the light chain variable region of the antibody contains the amino acid sequence as shown in SEQ ID NO. 6.

In another preferred embodiment, the heavy chain variable region of the antibody contains the amino acid sequence as shown in SEQ ID NO. 101; and the light chain variable region of the antibody contains the amino acid sequence as shown in SEQ ID NO. 103.

In another preferred embodiment, the heavy chain variable region of the antibody contains the amino acid sequence as shown in SEQ ID NO. 11; and the light chain variable region of the antibody contains the amino acid sequence as shown in SEQ ID NO. 16.

In another preferred embodiment, the heavy chain variable region of the antibody contains the amino acid sequence shown in SEQ ID NO. 21; and the light chain variable region of the antibody contains the amino acid sequence as shown in SEQ ID NO. 26.

In another preferred embodiment, the antibody is selected from the group consisting of:

| Antibody number | Clone number | VH Sequence number | VL Sequence number |
|---|---|---|---|
| 1 | 42A5A7 | 1 | 6 |
| 2 | 56F12H8 | 11 | 16 |
| 3 | 66H6C12 | 21 | 26 |
| 4 | 24D6B4 | 31 | 36 |
| 5 | 60G1C8 | 41 | 46 |
| 6 | 69C9E12 | 51 | 56 |
| 7 | 71E10B3 | 61 | 66 |
| 8 | 77B9A3 | 71 | 76 |
| 9 | 80H7D6 | 81 | 86 |
| 10 | 125A4E10 | 91 | 96 |
| 11 | Hu030-2 | 101 | 103. |

In another preferred embodiment, the amino acid sequence of the heavy chain variable region has at least 80%, 85%, 90%, 91%, 92%, 93%, 94%, 95%, 96%, 97%, 98% or 99% sequence homology or sequence identity with the amino acid sequence as shown in SEQ ID NO. 1, 11, 21, 31, 41, 51, 61, 71, 81, 91 or 101 in the sequence listing.

In another preferred embodiment, the amino acid sequence of the light chain variable region has at least 80%, 85%, 90%, 91%, 92%, 93%, 94%, 95%, 96%, 97%, 98% or 99% sequence homology or sequence identity with the amino acid sequence as shown in SEQ ID NO. 6, 16, 26, 36, 46, 56, 66, 76, 86, 96, or 103 in the sequence listing.

In the sixth aspect of the present invention, there is provided a recombinant protein comprising:
(i) the heavy chain variable region according to the first aspect of the invention, the heavy chain according to the second aspect of the invention, the light chain variable region according to the third aspect of the invention, the light chain according to the fourth aspect of the invention, or the antibody according to the fifth aspect of the invention; and
(ii) an optional tag sequence to assist expression and/or purification.

In another preferred embodiment, the tag sequence comprises a 6His tag.

In another preferred embodiment, the recombinant protein (or polypeptide) comprises a fusion protein.

In another preferred embodiment, the recombinant protein is a monomer, dimer, or multimer.

In another preferred embodiment, the recombinant protein comprises:
(i) an antibody selected from the group consisting of,

| Antibody number | Clone number | VH Sequence number | VL Sequence number |
|---|---|---|---|
| 1 | 42A5A7 | 1 | 6 |
| 2 | 56F12H8 | 11 | 16 |
| 3 | 66H6C12 | 21 | 26 |
| 4 | 24D6B4 | 31 | 36 |
| 5 | 60G1C8 | 41 | 46 |
| 6 | 69C9E12 | 51 | 56 |
| 7 | 71E10B3 | 61 | 66 |
| 8 | 77B9A3 | 71 | 76 |
| 9 | 80H7D6 | 81 | 86 |
| 10 | 125A4E10 | 91 | 96 |
| 11 | Hu030-2 | 101 | 103 |

and
(ii) an optional tag sequence to assist expression and/or purification.

In the seventh aspect of the present invention, there is provided a polynucleotide encoding a polypeptide selected from the group consisting of:
(1) the heavy chain variable region according to the first aspect of the invention, the heavy chain according to the second aspect of the invention, the light chain variable region according to the third aspect of the invention, the light chain according to the fourth aspect of the invention, or the antibody according to the fifth aspect of the invention; and
(2) the recombinant protein according to the sixth aspect of the present invention.

In another preferred embodiment, the polynucleotide encoding the heavy chain variable region is as shown in SEQ ID NO. 2, 12, 22, 32, 42, 52, 62, 72, 82, 92, or 102; and/or, the polynucleotide encoding the light chain variable region is as shown in SEQ ID NO. 7, 17, 27, 37, 47, 57, 67, 77, 87, 97, or 104.

In another preferred embodiment, the polynucleotide encoding the heavy chain variable region sequence and the polynucleotide encoding the light chain variable region sequence are selected from the group consisting of:

| Clone number | Sequence number of polynucleotide encoding VH | Sequence number of polynucleotide encoding VL |
|---|---|---|
| 42A5A7 | 2 | 7 |
| 56F12H8 | 12 | 17 |
| 66H6C12 | 22 | 27 |
| 24D6B4 | 32 | 37 |
| 60G1C8 | 42 | 47 |
| 69C9E12 | 52 | 57 |
| 71E10B3 | 62 | 67 |
| 77B9A3 | 72 | 77 |
| 80H7D6 | 82 | 87 |
| 125A4E10 | 92 | 97 |
| Hu030-2 | 102 | 104. |

In the eighth aspect of the present invention, there is provided a vector comprising the polynucleotide according to any one of the seventh aspect of the present invention.

In another preferred embodiment, the vector comprises a bacterial plasmid, a phage, a yeast plasmid, a plant cell virus, a mammalian cell virus such as an adenovirus, a retrovirus, or other vectors.

In the ninth aspect of the present invention, there is provided a genetically engineered host cell comprising the vector according to the eighth aspect of the present invention or having the polynucleotide according to the seventh aspect of the present invention integrated in the genome.

In the tenth aspect of the present invention, there is provided an antibody conjugate comprising:
(a) an antibody moiety, which is selected from the group consisting of the heavy chain variable region according to the first aspect of the invention, the heavy chain according to the second aspect of the invention, the light chain variable region according to the third aspect of the invention, the light chain according to the fourth aspect of the invention, or the antibody according to the fifth aspect of the invention, or a combination thereof; and
(b) a coupling moiety coupled to the antibody moiety, which is selected from the group consisting of a detectable label, a drug, a toxin, a cytokine, a radionuclide, an enzyme, or a combination thereof.

In another preferred embodiment, the antibody moiety is coupled to the coupling moiety by a chemical bond or linker.

In the eleventh aspect of the present invention, there is provided an immune cell expressing or exposing the antibody according to the fifth aspect of the present invention outside the cell membrane.

In another preferred embodiment, the immune cell comprises a NK cell, a T cell.

In another preferred embodiment, the immune cell is derived from human or non-human mammals (such as mice).

In the twelfth aspect of the present invention, there is provided a pharmaceutical composition comprising:
(i) an active ingredient selected from the group consisting of: the heavy chain variable region according to the first aspect of the present invention, the heavy chain according to the second aspect of the invention, the light chain variable region according to the third aspect of the invention, the light chain according to the fourth aspect of the invention, or the antibody according to the fifth aspect of the invention, the recombinant protein according to the sixth aspect of the invention, the antibody conjugate according to the tenth aspect of the invention, the immune cell according to the eleventh aspect of the invention, or a combination thereof; and
(ii) a pharmaceutically acceptable carrier.

In another preferred embodiment, the pharmaceutical composition is a liquid formulation.

In another preferred embodiment, the pharmaceutical composition is an injection.

In another preferred embodiment, the pharmaceutical composition comprising 0.01 to 99.99% of the antibody according to the fifth aspect of the present invention, the recombinant protein according to the sixth aspect of the present invention, the antibody conjugate according to the tenth aspect of the present invention, the immune cell according to the eleventh aspect of the present invention, or a combination thereof, and 0.01 to 99.99% of the pharmaceutical carrier, wherein the percentage is the mass percentage of the pharmaceutical composition.

In the thirteenth aspect of the invention, there is provided a use of an active ingredient selected from the group consisting of: the heavy chain variable region according to the first aspect of the present invention, the heavy chain according to the second aspect of the invention, the light chain variable region according to the third aspect of the invention, the light chain according to the fourth aspect of the invention, or the antibody according to the fifth aspect of the invention, the recombinant protein according to the sixth aspect of the invention, the antibody conjugate according to the tenth aspect of the invention, the immune cell according to the eleventh aspect of the invention, or a combination thereof, wherein the active ingredient is used for (a) preparing a diagnostic reagent or kit; and/or (b) preparing a medicament for the prevention and/or treatment of diseases associated with abnormal CD73 expression or function.

In another preferred embodiment, the diagnostic reagent is a detection sheet or a detection plate.

In another preferred embodiment, the disease associated with abnormal CD73 expression or function is a tumor.

In another preferred embodiment, the tumor is selected from the group consisting of bladder cancer, blood cancer, glioma, malignant glioma, melanoma, ovarian cancer, colon cancer, breast cancer, lung cancer, head and neck cancer, prostate cancer, pancreatic cancer.

In another preferred embodiment, the diagnostic reagent or kit is used for:
(1) detecting CD73 protein in samples; and/or
(2) detecting endogenous CD73 protein in tumor cells; and/or
(3) detecting tumor cells expressing CD73 protein;
wherein the medicament is used for preventing and/or treating diseases associated with abnormal CD73 expression or function, and the diseases associated with abnormal CD73 expression or function are tumors.

In another preferred embodiment, the tumor is selected from the group consisting of bladder cancer, blood cancer, glioma, malignant glioma, melanoma, ovarian cancer, colon cancer, breast cancer, lung cancer, head and neck cancer, prostate cancer, pancreatic cancer.

In another preferred embodiment, the antibody is in the form of a drug conjugate (ADC).

In another preferred embodiment, the diagnostic reagent or kit is used to diagnose CD73-related diseases.

In another preferred embodiment, the diagnostic reagent or kit is used to detect CD73 protein in a sample.

In the fourteenth aspect of the present invention, there is provided a method for *in vitro* detection (including diagnostic or non-diagnostic) of CD73 protein in a sample, comprising the steps of:
(1) contacting the sample with the antibody according to the fifth aspect of the present invention *in vitro;*
(2) detecting whether an antigen-antibody complex is formed, wherein the formation of the complex indicates the presence of CD73 protein in the sample.

In the fifteenth aspect of the invention, there is provided a composition for detecting CD73 protein in a sample *in vitro,* which comprises the antibody according to the fifth aspect of the present invention, the recombinant protein according to the sixth aspect of the present invention, the antibody conjugate according to the tenth aspect of the present invention, the immune cell according to the eleventh aspect of the present invention, or a combination thereof as an active ingredient.

In the sixteenth aspect of the invention, there is provided a detection plate comprising a substrate (support plate) and a test strip containing the antibody according to the fifth aspect of the present invention, the recombinant protein according to the sixth aspect of the present invention, the antibody conjugate according to the tenth aspect of the present invention, the immune cell according to the eleventh aspect of the present invention, or a combination thereof.

In the seventeenth aspect of the present invention, there is provided a kit comprising:
(1) a first container containing the antibody of the present invention; and/or
(2) a second container containing a secondary antibody against the antibody of the present invention;
or,
the kit comprises the detection plate according to the sixteenth aspect of the present invention.

In the eighteenth aspect of the present invention, there is provided a method for preparing a recombinant polypeptide, comprising:
(a) culturing the host cell according to the ninth aspect of the present invention under the condition suitable for expression;
(b) isolating a recombinant polypeptide from the culture, which is the antibody according to the fifth aspect of the present invention or the recombinant protein according to the sixth aspect of the present invention.

In the nineteenth aspect of the present invention, there is provided a drug combination comprising:
(i) a first active ingredient comprising the antibody 1 according to the fifth aspect of the present invention, or the recombinant protein according to the sixth aspect of the present invention, or the antibody conjugate according to the tenth aspect of the present invention, or the immune cell according to the eleventh aspect of the present invention, or the pharmaceutical composition according to the twelfth aspect of the present invention, or a combination thereof;
(ii) a second active ingredient comprising a second antibody, or a chemotherapeutic agent.

In another preferred embodiment, the second antibody is selected from the group consisting of a CTLA4 antibody, a PD-1 antibody, a PD-L1 antibody.

In another preferred embodiment, the second antibody is a PD-1 antibody.

In another preferred embodiment, the second active ingredient is an A2AR inhibitor.

In another preferred embodiment, the chemotherapeutic agent is selected from the group consisting of docetaxel, carboplatin, or a combination thereof.

In the twentieth aspect of the invention, there is provided a use of a combination for preparation of a medicine for the treatment of diseases associated with abnormal CD73 expression or function, wherein the combination comprises the antibody according to the fifth aspect of the present invention, or the recombinant protein according to the sixth aspect of the present invention, or the antibody conjugate according to the tenth aspect of the present invention, or the immune cell according to the eleventh aspect of the present invention, and/or the pharmaceutical composition according to the twelfth aspect of the present invention, as well as a second antibody or a chemotherapeutic agent.

In another preferred embodiment, the second antibody is selected from the group consisting of a CTLA4 antibody, a PD-1 antibody, a PD-L1 antibody.

In another preferred embodiment, the second antibody is a PD-1 antibody.

In another preferred embodiment, the second active ingredient is an A2AR inhibitor.

In the twenty-first aspect of the invention, there is provided a method for treating a disease associated with abnormal CD73 expression or function, which comprises administering an effective amount of the antibody according to the fifth aspect of the present invention, or the recombinant protein according to the sixth aspect of the present invention, or the antibody conjugate according to the tenth aspect of the present invention, or the immune cell according to the eleventh aspect of the present invention, or the pharmaceutical composition according to the twelfth aspect of the present invention, or a combination thereof, to a subject in need.

In another preferred embodiment, the disease associated with abnormal CD73 expression or function is a tumor.

In another preferred embodiment, the tumor is selected from the group consisting of bladder cancer, blood cancer, glioma, malignant glioma, melanoma, ovarian cancer, colon cancer, breast cancer, lung cancer, head and neck cancer, prostate cancer, pancreatic cancer.

In another preferred embodiment, the method further comprises administering a safe and effective amount of a second antibody to the subject before, during and/or after the administration of the first active ingredient.

In another preferred embodiment, the second antibody is selected from the group consisting of a CTLA4 antibody, a PD-1 antibody, a PD-L1 antibody.

In another preferred embodiment, the second antibody is a PD-1 antibody.

It should be understood that within the scope of the present invention, each technical features of the present invention described above and the technical features in the following (as examples) may be combined with each other to form a new or preferred technical solution, which is not listed here due to space limitations.

### Description of Figures

Figure. 1 Flow cytometry (FACS) detects binding of murine antibody to human CD73, cynomolgus monkey CD73 and murine CD73. Among them, mIgG1 is the isotype control; MFI is mean fluorescence intensity.
Figure. 2 The anti-CD73 murine antibody inhibits the enzyme activity of human CD73. Among them, mIgG1 is the isotype control;
Figure. 3 CD73 endocytosis is mediated by anti-CD73 murine antibody. Among them, mIgG1 is the isotype control;
Figure. 4 Anti-CD73 murine antibody restores AMP-mediated inhibition of T cell proliferation. Among them, Activated is the proliferation percentage of T cells without AMP and antibody; Activated + AMP 500 uM is the proliferation percentage of T cells with AMP and without antibody; mIgG1 is the isotype control;
Figure. 5 Flow cytometry (FACS) detects the binding of chimeric antibodies to human CD73, cynomolgus monkey CD73 and murine CD73. Among them, hIgG1 is the control; MFI is mean fluorescence intensity
Figure. 6 The anti-CD73 chimeric antibody inhibits the enzyme activity of human CD73. Among them, hIgG1 is the control;
Figure. 7 CD73 endocytosis is mediated by anti-CD73 chimeric antibody. Among them, hIgG1 is the control;
Figure. 8 Anti-CD73 chimeric antibody restores CD4 + T cells proliferation. Among them, Activated is the proliferation percentage of T cells without AMP and antibody; Activated + AMP 800 uM is the proliferation percentage of T cells with AMP and without antibody; hIgG1 is control; Tab2 is an anti-human CD73 antibody 11E1 of Innate Pharma.

### Detailed Description

Through extensive and intensive studies, the inventors have obtained a variety of antibody sequences using different immunization strategies (different mouse strains, multiple antigens, and different administration routes), and specific anti-CD73 monoclonal antibodies that bind to human CD73 were selected from them with better properties (e.g., excellent in various aspects *of in vitro* activity). Specifically, the present invention adopts techniques such as immunizing SJL mice, hybridomas, molecular biology (sequencing, constructing vectors) and the like, and provides a group of human-mouse chimeric antibodies binding to CD73, which contain heavy chain and light chain variable regions of mouse antibodies and constant regions of human antibodies. All variable regions contain three complementary determining regions or hypervariable regions, CDR1, CDR2 and CRR3. The variable region of the antibody can be humanized and combined with the constant region of the human antibody to form a fully human antibody molecule. The obtained CD73 antibody was proved by CD73 enzyme activity assay, endocytosis assay and T cell proliferation assay that it had excellent biological activity; compared with MEDI9447, it can obviously inhibit the enzyme activity of CD73 and promote the endocytosis of CD73; compared with BMS-986179, it can more effectively restore the proliferation of T cells mediated by AMP. In addition, the present invention also provides the use of the anti-CD73 monoclonal antibody, including improving tumor microenvironment, activating tumor specific immune response, inhibiting tumor growth, and being applied alone or in combination with other anti-tumor drugs for tumor immunotherapy. The present invention also provides the use of the anti-CD73 monoclonal antibody combined with a plurality of immune checkpoint antibodies or chemotherapeutic agents to effectively inhibit tumor growth, thereby being used for preparing drugs for treating diseases related to abnormal CD73 expression or function. On this basis, the present invention has been completed.

### The terms

In the present invention, "VH-CDR1" and "CDR-H1" can be used interchangeably, and both refer to CDR1 of heavy chain variable region; "VH-CDR2" and "CDR-H2" can be used interchangeably and both refer to CDR2 of heavy chain variable region; "VH-CDR3" and "CDR-H3" can be used interchangeably and both refer to CDR3 of heavy chain variable region. "VL-CDR1" and "CDR-L1" can be used interchangeably, and both refer to CDR1 of light chain variable region; "VL-CDR2" and "CDR-L2" can be used interchangeably and both refer to CDR2 of light chain variable region; "VL-CDR3" and "CDR-L3" can be used interchangeably and both refer to CDR3 of light chain variable region.

### Antibody

As used herein, the term "antibody" or "immunoglobulin" is a heterotetrameric glycoprotein of about 150,000 Da having the same structural characteristics, which consists of two identical light chains (L) and two identical heavy chains (H). Each light chain is connected to the heavy chain through a covalent disulfide bond, and the numbers of disulfide bonds between heavy chains of different immunoglobulin isotypes are different. Each heavy and light chain also has regularly spaced intrachain disulfide bonds. Each heavy chain has a variable region (VH) at one end, followed by multiple constant regions. Each light chain has a variable region (VL) at one end and a constant region at the other end; the constant region of the light chain is opposite to the first constant region of the heavy chain, and the variable region of the light chain is opposite to the variable region of the heavy chain. Special amino acid residues form an interface between the variable regions of the light and heavy chains.

As used herein, the term "variable" means that certain parts of the variable region of an antibody differ in sequence, which forms the binding and specificity of various specific antibodies for their specific antigens. However, the variability is not evenly distributed throughout the variable region of the antibody. It is concentrated in three segments called complementary determining regions (CDRs) or hypervariable regions in the light chain and heavy chain variable regions. The more conserved part of the variable region is called the framework region (FR). The variable regions of the natural heavy and light chains each contain four FR regions, which are roughly in the β-folded configuration, connected by the three CDRs that form the connecting loop, and in some cases may form a partly β folded structure. The CDRs in each chain get close through the FR regions and together with the CDRs of the other chain form the antigen-binding site of the antibody (see Kabat et al., NIH Publ. No. 91-3242, Volume I, pages 647-669 (1991)). The constant regions are not directly involved in the binding of antibodies to antigens, but they exhibit different effector functions, such as involved in the antibody-dependent cytotoxicity of antibodies.

The light chains of vertebrate antibodies (immunoglobulins) can be classified into one of two distinct classes (referred to as κ and λ) based on the amino acid sequence of their constant regions. Immunoglobulins can be divided into different types, according to the amino acid sequence of the constant region of the heavy chain. There are five main classes of immunoglobulins: IgA, IgD, IgE, IgG, and IgM, some of which can be further divided into subclasses (isotypes), such as IgG1, IgG2, IgG3, IgG4, IgA, and IgA2. The heavy chain constant regions corresponding to different classes of immunoglobulins are called δ, ε, γ, α, and µ, respectively. The subunit structures and three-dimensional configurations of different classes of immunoglobulins are well known to those skilled in the art.

In general, the antigen-binding properties of an antibody can be described by the three specific regions located in the variable regions of the heavy and light chains, called complementary determining regions (CDR), which divide this segment into 4 framework regions (FR). The amino acid sequences of the four FRs are relatively conservative and do not directly participate in the binding reaction. These CDRs form a circular structure, and get close in space structure through the β sheets formed by the FRs in between. The CDRs on the heavy chain and the CDRs on the corresponding light chain constitute the antigen binding site of the antibody. The amino acid sequences of antibodies of the same type can be compared to determine which amino acids constitute the FR or CDR regions.

The present invention includes not only intact antibodies, but also immunologically active fragments of antibody fragments or fusion proteins formed by antibodies and other sequences. Therefore, the present invention also includes fragments, derivatives and analogs of the antibodies.

In the present invention, antibodies include murine, chimeric, humanized, or fully human antibodies prepared by techniques well known to those skilled in the art. Recombinant antibodies, such as chimeric and humanized monoclonal antibodies, including human and non-human parts, can be obtained by standard DNA recombination techniques, and they are all useful antibodies. A chimeric antibody is a molecule in which different parts come from different animal species, such as a chimeric antibody with a variable region of a monoclonal antibody from a mouse and a constant region from a human immunoglobulin (see, for example, US Patent No. 4,816,567 and US Patent 4,816,397, hereby incorporated by reference in its entirety). Humanized antibodies refer to antibody molecules derived from non-human species, having one or more complementary determining regions (CDRs) derived from non-human species and framework regions derived from human immunoglobulin molecules (see US Patent 5,585,089, hereby incorporated by reference in its entirety). These chimeric and humanized monoclonal antibodies can be prepared using recombinant DNA techniques well known in the art.

In the present invention, the antibody may be monospecific, bispecific, trispecific, or more multispecific.

In the present invention, the antibody of the present invention also includes conservative variants thereof, which means that compared with the amino acid sequence of the antibody of the present invention, there are at most 10, preferably at most 8, more preferably at most 5, most preferably at most 3 amino acids replaced by amino acids with the same or similar properties to form a polypeptide. These conservatively variant polypeptides are preferably produced by amino acid substitution according to Table 1.

**Table 1**

| | | |
|---|---|---|
| Initial residue | Representative substitution | Preferred substitution |
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Anti-CD73 antibody

In the present invention, the antibody is an anti-CD73 antibody. The present invention provides an antibody with high specificity and high affinity against CD73, which comprises a heavy chain and a light chain, wherein the heavy chain contains a heavy chain variable region (VH) amino acid sequence, and the light chain contains a light chain variable region (VL) amino acid sequence.

Preferably,
the heavy chain variable region (VH) has complementary determining regions or CDRs selected from the group consisting of:
   VH-CDR1 as shown in SEQ ID NO. 10n +3,
   VH-CDR2 as shown in SEQ ID NO. 10n +4, and
   VH-CDR3 as shown in SEQ ID NO. 10n +5;
wherein, each n is independently 0, 1, 2, 3, 4, 5, 6, 7, 8, or 9;
the light chain variable region (VL) has complementary determining regions or CDRs selected from the group consisting of:
   VL-CDR1 as shown in SEQ ID NO. 10n +8,
   VL-CDR2 as shown in SEQ ID NO. 10n +9, and
   VL-CDR3 as shown in SEQ ID NO. 10n +10;
wherein, each n is independently 0, 1, 2, 3, 4, 5, 6, 7, 8, or 9;
wherein any one of the above amino acid sequences further comprises a derivative sequence that is optionally with at least one amino acid added, deleted, modified and/or substituted, and is capable of retaining the binding affinity to CD73.

Preferably, the heavy chain variable region (VH) comprises the following three complementary determining regions or CDRs:
VH-CDR1 as shown in SEQ ID NO. 10n +3,
VH-CDR2 as shown in SEQ ID NO. 10n +4, and
VH-CDR3 as shown in SEQ ID NO. 10n +5;
the light chain variable region (VL) comprises the following three complementary determining regions or CDRs:
VL-CDR1 as shown in SEQ ID NO. 10n +8,
VL-CDR2 as shown in SEQ ID NO. 10n +9, and
VL-CDR3 as shown in SEQ ID NO. 10n +10;
each n is independently 0, 1, 2 or 3; preferably n is 0 or 1;
wherein any one of the above amino acid sequences further comprises a derivative sequence that is optionally with at least one amino acid added, deleted, modified and/or substituted, and is capable of retaining the binding affinity to CD73.

In another preferred embodiment, the sequence with at least one amino acid added, deleted, modified and/or substituted in any of the above amino acid sequences is preferably an amino acid sequence having a homology or sequence identity of at least 80%, preferably at least 85%, more preferably at least 90%, most preferably at least 95% to the above amino acid sequence.

Methods known to those of ordinary skill in the art for determining sequence homology or identity include, but are not limited to: Computational Molecular Biology, edited by Lesk, A.M., Oxford University Press, New York, 1988; Biocomputing: Informatics and Genome Projects, edited by Smith, D.W., Academic Press, New York, 1993; Computer Analysis of Sequence Data, Part I, edited by Griffin, A.M. and Griffin, H.G., Humana Press, New Jersey, 1994; Sequence Analysis in Molecular Biology, von Heinje, G., Academic Press, 1987, and Sequence Analysis Primer, edited by Gribskov, M. and Devereux, J., Stockton Press, New York, 1991, and Carillo, H. and Lipman, D., SIAM J. Applied Math., 48:1073 (1988). The preferred method of determining identity is to obtain the greatest match between the sequences tested. The method of determining identity is compiled in a publicly available computer program. Preferred computer program methods for determining the identity between two sequences include, but are not limited to: GCG package (Devereux, J. et al., 1984), BLASTP, BLASTN, and FASTA (Altschul, S, F. et al., 1990). The BLASTX program is publicly available from NCBI and other sources (BLAST Manual, Altschul, S. et al., NCBI NLM NIH Bethesda, Md. 20894; Altschul, S. *et al.,* 1990). The well-known Smith Waterman algorithm can also be used to determine identity.

Preferably, the antibody described herein is one or more of an antibody full-length protein, an antigen-antibody binding domain protein fragment, a bispecific antibody, a multispecific antibody, a single chain antibody (scFv), a single domain antibody (sdAb), and a Single-domain antibody, as well as a monoclonal antibody or a polyclonal antibody made from the above antibodies. The monoclonal antibody can be developed by a variety of approaches and technologies, including hybridoma technology, phage display technology, single lymphocyte gene cloning technology, etc. The mainstream is to prepare monoclonal antibodies from wild-type or transgenic mice through hybridoma technology.

The antibody full-length protein is a conventional antibody full-length protein in the art, which comprises a heavy chain variable region, a light chain variable region, a heavy chain constant region, and a light chain constant region. The heavy chain variable region and light chain variable region of the protein and human heavy chain constant region and human light chain constant region constitute a fully human antibody full-length protein. Preferably, the antibody full-length protein is IgG1, IgG2, IgG3 or IgG4.

The antibody in the present invention can be a full-length protein (such as IgG1, IgG2a, IgG2b or IgG2c), or a protein fragment containing an antigen-antibody binding domain (such as Fab, F(ab'), sdAb, ScFv fragments).

The antibody in the present invention (antibody against CD73) can be a wild-type protein, or a mutant protein that has achieved a certain effect through specific mutations, for example, using mutations to eliminate the effector function of the antibody.

The antibody of the present invention may be a double-chain or single-chain antibody, and may be selected from an animal-derived antibody, a chimeric antibody and a humanized antibody, more preferably a humanized antibody and a human-animal chimeric antibody, more preferably a fully humanized antibody.

The antibody derivatives of the present invention may be single chain antibodies, and/or antibody fragments, such as: Fab, Fab', (Fab')2 or other known antibody derivatives in the art, etc., as well as any one or several of IgA, IgD, IgE, IgG and IgM antibodies or other subtypes.

The single-chain antibody is a conventional single-chain antibody in the art, which comprises a heavy chain variable region, a light chain variable region and a short peptide of 15-20 amino acids.

Wherein, the animal is preferably a mammal, such as a mouse.

The antibody of the present invention may be a chimeric antibody, a humanized antibody, a CDR grafted and/or modified antibody targeting CD73 (such as human CD73).

In the above content of the present invention, the number of added, deleted, modified and/or substituted amino acids is preferably not more than 40% of the total number of amino acids in the original amino acid sequence, more preferably not more than 35%, more preferably 1-33%, more preferably 5-30%, more preferably 10-25%, more preferably 15-20%.

In the above content of the present invention, more preferably, the number of added, deleted, modified and/or substituted amino acids may be 1-7, more preferably 1-5, more preferably 1-3, more preferably 1-2.

In another preferred embodiment, the heavy chain variable region of the antibody contains the amino acid sequence shown in SEQ ID NO. 1, 11, 21, 31, 41, 51, 61, 91, or 101.

In another preferred embodiment, the light chain variable region of the antibody contains the amino acid sequence shown in SEQ ID NO. 6, 16, 26, 36, 46, 56, 66, 76, 86, 96, or 103.

In another preferred embodiment, the amino acid sequences of the heavy chain variable region and/or the light chain variable region of the antibody targeting CD73 are shown in the following Table 2:

**Table 2**

| Antibody number | VH Sequence number | VL Sequence number |
|---|---|---|
| 1 | 1 | 6 |
| 2 | 11 | 16 |
| 3 | 21 | 26 |
| 4 | 31 | 36 |
| 5 | 41 | 46 |
| 6 | 51 | 56 |
| 7 | 61 | 66 |
| 8 | 71 | 76 |
| 9 | 81 | 86 |
| 10 | 91 | 96 |
| 11 (Hu030-2) | 101 | 103 |

In another preferred embodiment, the antibodies targeting CD73 are Hu030-2, 42A5A7, 56F12H8, 66H6C12, 24D6B4, 60G1C8, 69C9E12, 71E10B3, 77B9A3, 80H7D6, or 125A4E10.

In another preferred embodiment, the antibodies targeting CD73 are Hu030-2, 42A5A7, 56F12H8, 66H6C12.

In another preferred embodiment, the antibody targeting CD73 is Hu030-2, or 42A5A7.

### Recombinant protein

The present invention also provides a recombinant protein, which comprises one or more of heavy chain CDR1 (VH-CDR1), heavy chain CDR2 (VH-CDR2) and heavy chain CDR3 (VH-CDR3) of a CD73 antibody, and/or one or more of light chain CDR1 (VL-CDR1), light chain CDR2 (VL-CDR2) and light chain CDR3 (VL-CDR3) of a CD73 antibody,
the sequences of the heavy chain CDR1-3 are as follows:
   VH-CDR1 shown in SEQ ID NO: 10n+3,
   VH-CDR2 shown in SEQ ID NO. 10n +4,
   VH-CDR3 shown in SEQ ID NO: 10n+5;
the sequences of the light chain CDR1-3 are as follows:
   VL-CDR1 shown in SEQ ID NO: 10n+8,
   VL-CDR2 shown in SEQ ID NO: 10n+9, and
   VL-CDR3 shown in SEQ ID NO: 10n+10;
each n is independently 0, 1, 2, 3, 4, 5, 6, 7, 8, or 9; preferably n is 0 or 1;
wherein any one of the above amino acid sequences further comprises a derivative sequence that is optionally with at least one amino acid added, deleted, modified and/or substituted, and is capable of retaining the binding affinity to CD73.

In another preferred embodiment, the sequence with at least one amino acid added, deleted, modified and/or substituted in any of the above amino acid sequences is preferably an amino acid sequence having a homology or sequence identity of at least 80%, preferably at least 85%, more preferably at least 90%, most preferably at least 95% to the above amino acid sequence.

In another preferred embodiment, the recombinant protein of the present invention comprises a heavy chain variable region of a CD73 antibody and/or a light chain variable region of a CD73 antibody, the heavy chain variable region of a CD73 antibody comprising the amino acid sequence shown in SEQ ID NO. 1, 11, 21, 31, 41, 51, 61, or 101, and the light chain variable region of a CD73 antibody comprising the amino acid sequence shown in SEQ ID NO. 6, 16, 26, 36, 46, 56, 66, or 103.

In another preferred embodiment, the recombinant protein of the present invention comprises a heavy chain variable region of a CD73 antibody and a light chain variable region of a CD73 antibody, the heavy chain variable region of a CD73 antibody comprising the amino acid sequence shown in SEQ ID NO. 1, 11, 21, 31, 41, 51, 61, or 101, and the light chain variable region of a CD73 antibody comprising the amino acid sequence shown in SEQ ID NO. 6, 16, 26, 36, 46, 56, 66, or 103.

In another preferred embodiment, the recombinant protein and the amino acid sequence numbers of the heavy chain CDR1-3 and light chain CDR1-3 comprised therein are as shown in Table 3:

**Table 3 Amino acid sequence numbers of heavy chain CDR1-3 and light chain CDR1-3**

| Recom binant protein numbe r | Heavy chain protein | | | | Light chain protein | | | |
|---|---|---|---|---|---|---|---|---|
| | Varia ble regio n | VH-C DR1 | VH-CD R2 | VH-CD R3 | Varia ble regio n | VL-C DR1 | VL-CD R2 | VL-CD R3 |
| 1 | 1 | 3 | 4 | 5 | 6 | 8 | 9 | 10 |
| 2 | 11 | 13 | 14 | 15 | 16 | 18 | 19 | 20 |
| 3 | 21 | 23 | 24 | 25 | 26 | 28 | 29 | 30 |
| 4 | 31 | 33 | 34 | 35 | 36 | 38 | 39 | 40 |
| 5 | 41 | 43 | 44 | 45 | 46 | 48 | 49 | 50 |
| 6 | 51 | 53 | 54 | 55 | 56 | 58 | 59 | 60 |
| 7 | 61 | 63 | 64 | 65 | 66 | 68 | 69 | 70 |
| 8 | 71 | 73 | 74 | 75 | 76 | 78 | 79 | 80 |
| 9 | 81 | 83 | 84 | 85 | 86 | 88 | 89 | 90 |
| 10 | 91 | 93 | 94 | 95 | 96 | 98 | 99 | 100 |
| 11 | 101 | 3 | 4 | 5 | 103 | 8 | 9 | 10 |

wherein any one of the above amino acid sequences further comprises a derivative sequence that is optionally with at least one amino acid added, deleted, modified and/or substituted, and is capable of retaining the binding affinity to CD73.

Preferably, the recombinant protein further comprises an antibody heavy chain constant region and/or an antibody light chain constant region, wherein the antibody heavy chain constant region is conventional in the art, preferably a rat antibody heavy chain constant region or a human antibody heavy chain constant region, more preferably a human antibody heavy chain constant region. The antibody light chain constant region is conventional in the art, preferably a rat antibody light chain constant region or a human antibody light chain constant region, more preferably a human antibody light chain constant region.

The recombinant protein is a conventional protein in the art. Preferably, it is one or more of an antibody full-length protein, an antigen-antibody binding domain protein fragment, a bispecific antibody, a multispecific antibody, a single chain antibody fragment (scFv), a single domain antibody (sdAb) and a Single-domain antibody, as well as a monoclonal antibody or a polyclonal antibody made from the above antibodies. The monoclonal antibody can be developed by a variety of approaches and technologies, including hybridoma technology, phage display technology, single lymphocyte gene cloning technology, etc. The mainstream is to prepare monoclonal antibodies from wild-type or transgenic mice through hybridoma technology.

The antibody full-length protein is a conventional antibody full-length protein in the art, which comprises a heavy chain variable region, a light chain variable region, a heavy chain constant region, and a light chain constant region. The heavy chain variable region and light chain variable region of the protein and human heavy chain constant region and human light chain constant region constitute a fully human antibody full-length protein. Preferably, the antibody full-length protein is IgG1, IgG2, IgG3 or IgG4.

The single-chain antibody is a conventional single-chain antibody in the art, which comprises a heavy chain variable region, a light chain variable region and a short peptide of 15-20 amino acids.

The antigen-antibody binding domain protein fragments are conventional antigen-antibody binding domain protein fragments in the art, which comprise a light chain variable region, a light chain constant region, and an Fd segment of heavy chain constant region. Preferably, the antigen-antibody binding domain protein fragments are Fab and F (ab').

The single domain antibody is a conventional single domain antibody in the art, which comprises a heavy chain variable region and a heavy chain constant region.

The single-domain antibody is a conventional single-domain antibody in the art, which only comprises a heavy chain variable region.

Wherein, the preparation method of the recombinant protein is a conventional preparation method in the art. Preferably, the preparation method is: isolating and obtaining the protein from an expression transformant that recombinantly expresses the protein or obtaining the protein by artificially synthesizing a protein sequence. The method of isolating and obtaining the protein from an expression transformant that recombinantly expresses the protein is preferably as follows: cloning a nucleic acid molecule encoding the protein carrying a point mutation into a recombinant vector, and transforming the obtained recombinant vector into a transformant to obtain a recombinant expression transformant, and by culturing the obtained recombinant expression transformant, the recombinant protein can be obtained by separation and purification.

### Nucleic Acid

The present invention also provides a nucleic acid, which encodes the above-mentioned antibody (e.g., anti-CD47 antibody) or the heavy chain variable region or light chain variable region of recombinant protein or anti-CD47 antibody.

The preparation method of the nucleic acid is a conventional preparation method in the art. Preferably, it comprises the following steps: obtaining the nucleic acid molecule encoding the above-mentioned protein by gene cloning technology, or obtaining the nucleic acid molecule encoding the above-mentioned protein by the method of artificial full-length sequence synthesis.

Those skilled in the art know that the base sequence encoding the amino acid sequence of the protein can be replaced, deleted, changed, inserted or added appropriately to provide a polynucleotide homolog. The homolog of the polynucleotide of the present invention can be prepared by replacing, deleting or adding one or more bases of the gene encoding the protein sequence within the scope of maintaining the activity of the antibody.

### Vector

The present invention also provides a recombinant expression vector comprising the nucleic acid.

Wherein the recombinant expression vector can be obtained by conventional methods in the art, that is, by connecting the nucleic acid molecule of the present invention to various expression vectors, thus being constructed. The expression vector is one of a variety of conventional vectors in the art, as long as it can carry the above-mentioned nucleic acid molecule. The vector preferably includes: various plasmids, cosmids, phage or virus vectors and the like.

The present invention also provides a recombinant expression transformant comprising the above-mentioned recombinant expression vector.

Wherein, the preparation method of the recombinant expression transformant is a conventional preparation method in the art, preferably comprising: being obtained by transforming the recombinant expression vector into a host cell. The host cell is one of a variety of conventional host cells in the art, as long as the recombinant expression vector can replicate itself stably and the nucleic acid carried can be effectively expressed. Preferably, the host cell is *E.coli* TGI or *E.coli* BL21 cell (for expressing single-chain antibodies or Fab antibodies), or HEK293 or CHO cell (for expressing full-length IgG antibodies). The above-mentioned recombinant expression plasmid is transformed into a host cell to obtain the preferred recombinant expression transformant of the present invention. Wherein the transformation method is a conventional transformation method in the art, preferably a chemical transformation method, a heat shock method or an electrotransformation method.

### Preparation of Antibodies

The sequence of the DNA molecule for the antibody or a fragment thereof according to the present invention can be obtained by conventional techniques, for example, methods such as PCR amplification or genomic library screening. In addition, the sequences encoding light chain and heavy chain can be fused together, to form a single-chain antibody.

Once a relevant sequence is obtained, recombination methods can be used to obtain the relevant sequence in large quantities. This is usually carried out by cloning the sequence into a vector, transforming a cell with the vector, and then separating the relevant sequence from the proliferated host cell by conventional methods.

In addition, a relevant sequence can be synthesized artificially, especially when the fragment is short in length. Usually, several small fragments are synthesized first, and then are linked together to obtain a fragment with a long sequence.

It has been possible now to obtain a DNA sequence encoding the antibody (or a fragment thereof, or a derivative thereof) according to the present invention completely by chemical synthesis. Then, the DNA sequence can be introduced into various existing DNA molecules (or, for example, vectors) and cells known in the art. In addition, mutations can also be introduced into the protein sequence according to the present invention by chemical synthesis.

The present invention further relates to a vector comprising said suitable DNA sequence and a suitable promoter or a control sequence. These vectors can be used to transform suitable host cells to enable them to express protein.

The host cell can be a prokaryotic cell, such as a bacterial cell; or a lower eukaryotic cell, such as a yeast cell; or a higher eukaryotic cell, such as a mammalian cell. Preferred animal cells include, but are not limited to, CHO-S, HEK-293 cells.

In general, under conditions suitable for expression of the antibody according to the present invention, the host cell obtained is cultured. Then, the antibody according to the present invention is purified by using conventional immunoglobulin purification steps, for example, the conventional separation and purification means well known to those skilled in the art, such as protein A-Sepharose, hydroxyapatite chromatography, gel electrophoresis, dialysis, ion exchange chromatography, hydrophobic chromatography, molecular sieve chromatography or affinity chromatography.

The monoclonal antibody obtained can be identified by conventional means. For example, the binding specificity of a monoclonal antibody can be determined by immunoprecipitation or an *in vitro* binding assay (such as radioimmunoassay (RIA) or enzyme-linked immunosorbent assay (ELISA)). The binding affinity of a monoclonal antibody can be determined by, for example, the Scatchard analysis (Munson et al., Anal. Biochem., 107: 220 (1980)).

The antibody according to the present invention can be expressed in a cell or on the cell membrane, or is secreted extracellularly. If necessary, the recombinant protein can be separated and purified by various separation methods according to its physical, chemical, and other properties. These methods are well known to those skilled in the art. Examples of these methods include, but are not limited to, conventional renaturation treatment, treatment with a protein precipitant (salting out method), centrifugation, osmotic bacteria disruption, ultrasonic treatment, ultracentrifugation, molecular sieve chromatography (gel filtration), adsorption chromatography, ion exchange chromatography, high performance liquid chromatography (HPLC), various other liquid chromatographic techniques, and combinations of these methods.

### Antibody-Drug Conjugate (ADC)

The present invention also provides an antibody-drug conjugate (ADC) based on the antibody according to the present invention.

Typically, the antibody-drug conjugate comprises the antibody and an effector molecule, wherein the antibody is conjugated to the effector molecule, and chemical conjugation is preferred. Preferably, the effector molecule is a therapeutically active drug. In addition, the effector molecule may be one or more of a toxic protein, a chemotherapeutic drug, a small-molecule drug or a radionuclide.

The antibody according to present invention and the effector molecule may be coupled by a coupling agent. Examples of the coupling agent may be any one or more of a non-selective coupling agent, a coupling agent utilizing a carboxyl group, a peptide chain, and a coupling agent utilizing a disulfide bond. The non-selective coupling agent refers to a compound that results in a linkage between an effector molecule and an antibody via a covalent bond, such as glutaraldehyde, etc. The coupling agent utilizing a carboxyl group may be any one or more of cis-aconitic anhydride coupling agents (such as cis-aconitic anhydride) and acyl hydrazone coupling agents (the coupling site is acyl hydrazone).

Certain residues on an antibody (such as Cys or Lys, etc.) are used to link a variety of functional groups, including imaging agents (such as chromophores and fluorophores), diagnostic agents (such as MRI contrast agents and radioisotopes), stabilizers (such as ethylene glycol ploymers) and therapeutic agents. An antibody can be conjugated to a functional agent to form a conjugate of the antibody-functional agent. A functional agent (e.g. a drug, a detection reagent, a stabilizer) is conjugated (covalently linked) to an antibody. A functional agent can be linked to an antibody either directly or indirectly via a linker.

Antibodies can be conjugated to drugs to form antibody-drug conjugates (ADCs). Typically, an ADC comprises a linker between a drug and an antibody. The linker can be a degradable or non-degradable linker. Typically, degradable linkers are easily degraded in an intracellular environment, for example, the linker is degraded at the target site, thereby releasing the drug from the antibody. Suitable degradable linkers include, for example, enzyme-degradable linkers, including peptidyl-containing linkers that can be degraded by protease (e.g. lysosomal protease or endosomal protease) in a cell, or sugar linkers, for example, glucuronide-containing linkers that can be degraded by glucuronidase. Peptidyl linkers may include, for example, dipeptides, such as valine-citrulline, phenylalanine-lysine or valine-alanine. Other suitable degradable linkers include, for example, pH sensitive linkers (e.g. linkers that are hydrolyzed at a pH of below 5.5, such as hydrazone linkers) and linkers that are degraded under reducing conditions (e.g. disulfide-bond linkers). A non-degradable linker typically releases a drug under conditions that the antibody is hydrolyzed by protease.

Prior to linkage to an antibody, a linker has a reactive group capable of reacting with certain amino acid residues, and the linkage is achieved by the reactive group. A thiol-specific reactive group is preferred, and includes, for example, a maleimide compound, a halogenated (e.g. iodo-, bromo- or chloro-substituted) amide; a halogenated (e.g. iodo-, bromo- or chloro-substituted) ester; a halogenated (e.g. iodo-, bromo- or chloro-substituted) methyl ketone, a benzyl halide (e.g. iodide, bromide or chloride); vinyl sulfone, pyridyl disulfide; a mercury derivative such as 3,6-di-(mercurymethyl)dioxane, wherein the counter ion is CH₃COO⁻, Cl⁻ or NO₃⁻; and polymethylene dimethyl sulfide thiosulfonate. The linker may include, for example, a maleimide linked to an antibody via thiosuccimide.

A drug may be any cytotoxic, cytostatic or immunosuppressive drug. In an embodiment, an antibody is linked to a drug via a linker, and the drug has a functional group that can form a bond with the linker. For example, a drug may have an amino group, a carboxyl group, a thiol group, a hydroxyl group, or a ketone group that can form a bond with a linker. When a drug is directly linked to a linker, the drug has a reactive group before being linked to an antibody.

Useful drugs include, for example, anti-tubulin drugs, DNA minor groove binding agents, DNA replication inhibitors, alkylating agents, antibiotics, folic acid antagonists, antimetabolites, chemotherapy sensitizers, topoisomerase inhibitors, vinca alkaloids, etc. Examples of particularly useful cytotoxic drugs include, for example, DNA minor groove binding agents, DNA alkylating agents, and tubulin inhibitors; typical cytotoxic drugs include, for example, auristatins, camptothecins, docamycin/duocarmycins, etoposides, maytansines and maytansinoids (e.g. DM1 and DM4), taxanes, benzodiazepines or benzodiazepine containing drugs (e.g. pyrrolo[1,4]benzodiazepines (PBDs), indolinobenzodiazepines and oxazolidinobenzodiazepines), and vinca alkaloids.

In the present invention, a drug-linker can be used to form an ADC in a simple step. In other embodiments, a bifunctional linker compound can be used to form an ADC in a two-step or multi-step process. For example, a cysteine residue is reacted with the reactive moiety of a linker in a first step, and then the functional group on the linker is reacted with a drug in the subsequent step, so as to form an ADC.

In general, the functional group on a linker is selected so that it can specifically react with the suitable reactive group on a drug moiety. As a non-limiting example, an azide-based moiety can be used to specifically react with the reactive alkynyl group on a drug moiety. The drug is covalently bound to the linker by 1,3-dipolar cycloaddition between the azide and alkynyl group. Other useful functional groups include, for example, ketones and aldehydes (suitable for reacting with hydrazides and alkoxyamines), phosphines (suitable for reacting with azides); isocyanates and isothiocyanates (suitable for reacting with amines and alcohols); and activated esters, for example, N-hydroxysuccinimide esters (suitable for reacting with amines and alcohols). These and other linkage strategies, for example, those described in "Bioconjugation Technology" (2nd Edition (Elsevier)), are well known to those skilled in the art. Those skilled in the art could understand that when a complementary pair of reactive functional groups are selected for a selective reaction between a drug moiety and a linker, each member of the complementary pair can be used for the linker, and can also be used for the drug.

The present invention further provides a method for preparing an ADC, which may further comprise: under conditions sufficient to form an antibody-drug conjugate (ADC), binding an antibody to a drug-linker compound.

In certain embodiments, the method according to the present invention comprises: under conditions sufficient to form an antibody-linker conjugate, binding an antibody to a bifunctional linker compound. In these embodiments, the method according to the present invention further comprises: under conditions sufficient to covalently link the drug moiety to the antibody via a linker, binding the antibody-linker conjugate to the drug moiety.

In some embodiments, an antibody-drug conjugate (ADC) has a formula as follows: 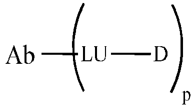 wherein,
Ab is an antibody,
LU is a linker;
D is a drug;
and the subscript p is a value selected from 1 to 8.

### Application

The present invention also provides use of the antibody, the antibody conjugate ADC, the recombinant protein, and/or immune cell of the present invention, for example for the preparation of diagnostic preparations or the preparation of drugs.

Preferably, the drug is used for prevention and/or treatment of diseases associated with abnormal CD73 expression or function.

In the present invention, the diseases associated with abnormal CD73 expression or function are conventional diseases associated with abnormal CD73 expression or function in the art. Preferably, the disease associated with abnormal CD47 expression or function is a tumor/cancer.

In the present invention, the cancer is a conventional cancer in the art, preferably bladder cancer, blood cancer, glioma, malignant glioma, melanoma, ovarian cancer, colon cancer, breast cancer, lung cancer, head and neck cancer, prostate cancer, pancreatic cancer.

Uses of the antibody, the ADC, the recombinant protein, and/or the immune cell of the present invention include (but are not limited to):
(i) for diagnosis, prevention and/or treatment of tumorigenesis, tumor growth and/or metastasis, especially a tumor with high expression of CD73. The tumors include, but are not limited to: bladder cancer, blood cancer, glioma, malignant glioma, melanoma, ovarian cancer, colon cancer, breast cancer, lung cancer, head and neck cancer, prostate cancer, pancreatic cancer.

### Use for detection and the kits

The antibody or ADC of the present invention can be used for detection, for example, for detection of samples to provide diagnostic information.

In the present invention, the samples (specimens) used include cells, tissue samples and biopsy specimens. The term "biopsy" used in the present invention shall include all kinds of biopsy known to those skilled in the art. Therefore, the biopsy used in the present invention may include, for example, excision samples of tumors, tissue samples prepared by endoscopic methods or organ puncture or needle biopsy.

The samples used in the present invention include fixed or preserved cells or tissue samples.

The present invention also provides a kit containing the antibody (or a fragment thereof) of the present invention. In a preferred embodiment of the present invention, the kit further includes a container, instructions for use, buffer, and the like. In a preferred example, the antibody of the present invention can be immobilized on a detection plate.

### Pharmaceutical Composition

The present invention further provides a composition. In the preferred examples, the composition is a pharmaceutical composition comprising the antibody, or an active fragment, a fusion protein or an ADC thereof, or a corresponding immune cell, and a pharmaceutically acceptable carrier. In general, these substances may be formulated in a non-toxic, inert and pharmaceutically acceptable aqueous carrier medium, wherein the pH is generally about 5-8, preferably, pH is about 6-8, though the pH value may be varied depending on the nature of the substances to be formulated and the condition to be treated.

The formulated pharmaceutical composition may be administered by conventional routes, including (but not limited to): intratumoral, intraperitoneal, intravenous, or topical administration. Typically, the administration route of the pharmaceutical composition of the present invention is preferably injection or oral administration. The injection administration preferably includes intravenous injection, intramuscular injection, intraperitoneal injection, intradermal injection, or subcutaneous injection. The pharmaceutical composition is in one of a variety of conventional dosage forms in the art, preferably in solid, semi-solid or liquid form, and can be an aqueous solution, a non-aqueous solution or a suspension, and more preferably tablets, capsules, granules, injection or infusion, etc.

The antibody of the present invention can also be used for cell therapy by expressing the nucleotide sequence in the cell. For example, the antibody is used for chimeric antigen receptor T cell immunotherapy (CAR-T) and the like.

The pharmaceutical composition of the present invention is a pharmaceutical composition for prevention and/or treatment of diseases associated with abnormal CD73 expression or function.

The pharmaceutical composition of the present invention can be directly used for binding to a CD73 protein molecule, and thus can be used for preventing and treating diseases such as tumors.

The pharmaceutical composition according to the present invention comprises a safe and effective amount (e.g. 0.001-99 wt %, preferably 0.01-90 wt %, preferably 0.1-80 wt %) of the monoclonal antibody according to the present invention (or a conjugate thereof) and a pharmaceutically acceptable carrier or excipient. Such carriers include (but are not limited to): saline, buffers, glucose, water, glycerol, ethanol, and a combination thereof. Pharmaceutical preparations should correspond to the administration modes. The pharmaceutical composition according to the present invention can be prepared in the form of an injection, for example, by a conventional method using physiological saline or an aqueous solution containing glucose and other adjuvants. A pharmaceutical composition, for example, an injection and a solution, should be prepared under aseptic conditions. The administration amount of an active ingredient is a therapeutically effective amount, for example, about 1 µg per kilogram of body weight to about 5 mg per kilogram of body weight daily. In addition, the polypeptide according to the present invention may also be used in combination with an additional therapeutic agent.

In the present invention, preferably, the pharmaceutical composition of the present invention further comprises one or more pharmaceutical carriers. The pharmaceutical carrier is a conventional pharmaceutical carrier in the art, and the pharmaceutical carrier can be any suitable physiologically or pharmaceutically acceptable pharmaceutical excipient. The pharmaceutical excipient is a conventional pharmaceutical excipient in the art, and preferably includes pharmaceutically acceptable excipients, fillers or diluents. More preferably, the pharmaceutical composition comprises 0.01-99.99% of the above-mentioned protein and 0.01-99.99% of the pharmaceutically acceptable carrier, wherein the percentage is the mass percentage of the pharmaceutical composition.

In the present invention, preferably, the administration amount of the pharmaceutical composition is an effective amount, and the effective amount is an amount that can alleviate or delay the progression of the disease, and the degenerative or traumatic condition. The effective amount can be determined on an individual basis and will be partly based on consideration of the symptoms to be treated and the results sought. Those skilled in the art can determine the effective amount by using the above-mentioned factors such as individual basis and using no more than conventional experiments.

When a pharmaceutical composition is used, a safe and effective amount of an immunoconjugate is administered to a mammal, wherein the safe and effective amount is generally at least about 10 µg per kilogram of body weight, and in most cases, no more than about 50 mg per kilogram of body weight, preferably, the amount is from about 10 µg per kilogram of body weight to about 20 mg per kilogram of body weight. Of course, a specific amount should also depend on the factors such as administration route and physical conditions of a patient, which falls into the skills of skilled physicians.

The present invention provides use of the above-mentioned pharmaceutical composition in the preparation of a medicine for preventing and/or treating diseases associated with abnormal CD73 expression or function. Preferably, the disease associated with abnormal CD73 expression or function is a tumor/cancer.

### Method and composition for detecting CD73 protein in a sample

The present invention also provides a method for detecting CD73 protein in a sample (for example, detecting over-expressing CD73 cells), which comprises the following steps: contacting the above-mentioned antibody with a sample to be tested *in vitro,* and detecting whether the above-mentioned antibody binds to the sample to be tested, to form an antigen-antibody complex.

The meaning of overexpression is conventional in the art, which refers to the overexpression of RNA or protein of CD73 protein in the sample to be tested (due to increased transcription, post-transcriptional processing, translation, post-translational processing and protein degradation changes), and local overexpression and increased functional activity (such as in the case of increased enzymatic hydrolysis of the substrate) due to changes in protein transport mode (increased nuclear localization).

In the present invention, the detection method for detecting whether an antigen-antibody complex is formed is a conventional detection method in the art, preferably a flow cytometry (FACS) detection.

The present invention provides a composition for detecting CD73 protein in a sample, which comprises the above-mentioned antibody, recombinant protein, antibody conjugate, immune cell, or a combination thereof as an active ingredient. Preferably, it also comprises a compound composed of the functional fragments of the above-mentioned antibody as an active ingredient.

On the basis of conforming to common knowledge in the art, the above-mentioned preferred conditions can be combined arbitrarily to obtain preferred embodiments of the present invention.

### The main advantages of the present invention are:

(1) The antibodies obtained according to the present invention recognize different epitopes from MEDI9447 and BMS anti-CD73;
(2)The antibody obtained according to the present invention can simultaneously have excellent ability to mediate CD73 endocytosis and restore T cell proliferation.

The invention is further illustrated below in conjunction with specific embodiments. It should be understood that the examples are not intended to limit the scope of the invention. The experimental methods without detailed conditions in the following examples are generally in accordance with the conditions described in the conventional conditions such as Sambrook. J et al. "Guide to Molecular Cloning Laboratory", or in accordance with the conditions recommended by the manufacturer. Unless otherwise stated, percentages and parts are calculated by weight. Unless otherwise specified, the experimental materials and reagents used in the following examples are commercially available.

The room temperature described in the examples is a conventional room temperature in the art, and is generally 10-30°C.

Unless otherwise specified, the PBS described in the examples is PBS phosphate buffer, pH 7.2.

### Materials and Methods

The present invention uses an advanced antibody transgenic mouse technology platform to prepare a monoclonal antibody with a fully human sequence.

The anti-CD73 antibody obtained according to the present invention can be prepared by various ways and methods, including:

### (1) Traditional hybridoma preparation technology

The traditional hybridoma preparation technology was established by Kohler and Milstein 40 years ago (Kohler and Milstein 1975, Nature 256: 495), and has now been widely used in the preparation and production of many related monoclonal antibodies in scientific research, diagnosis, and treatment. Although the basic method is still in use today, there have been changes, improvements and innovations in many aspects, including the use of different strains of animals such as genetically modified animals, the introduction of electrofusion technology, and the application of high-efficiency screening technology equipment such as ClonePix equipment, which make the application of tumor technology more diverse and efficient. Monoclonal antibodies prepared from conventional animals such as mice can be cloned by conventional molecular biology methods to clone the antibody heavy chain variable region and light chain variable region genes, and the variable region genes can be grafted to human antibody constant region genes to form human-mouse chimeric antibody (U.S. Pat. No. 4,816,567, Cabilly et al), to greatly reduce the immunogenicity of the human body. Furthermore, the CDR domains of the variable region of the mouse antibody can be grafted onto the framework of the human antibody, thereby reducing the composition of the mouse antibody to less than 5%, greatly increasing the safety of the antibody used in human body. Antibodies obtained through this approach are called humanized antibodies and are the main products in the antibody drug market at present (U.S. Pat. No. 5,225,539 to 55, Winter, and U.S. Pat. No. 5,530,101; 5,585,089; 5,693,762 and 6,180,370 to Queen et al).

In a preferred example of the present invention, a series of human-mouse chimeric monoclonal antibodies are prepared. These anti-human CD73 antibodies are prepared by immunizing Balb/c and SJL mice, optimized hybridoma technology preparation, molecular biology and antibody engineering technology, and have mouse antibody heavy chain and light chain variable regions and human antibody constant regions.

### (2) Immunization of Balb/c and SJL mice

Immunogens including extracellular domain CD73 protein, CD73 recombinant cell line, expression plasmid of CD73 DNA vector and the like were prepared.

Immunogen 1), the C-terminus of the extracellular domain CD73 protein amino acid sequence 27-547 was added with His6 tag, and then cloned into the pTT5 vector to obtain pTT5-hCD73 ECD-His6. It was transiently transfected into CHO cells. After 9 days, the cell culture medium was collected, the cell components were removed by centrifugation, and the supernatant medium was filtered with a 0.22 µm filter. Then, the culture supernatant containing CD73 protein was loaded into nickel affinity chromatography column, and the change in ultraviolet absorption value (A280nm) was monitored by ultraviolet (UV) detector. After the sample was loaded, the column was washed with PBS (phosphate buffer, pH 7.5) and PBS (containing 0.1% Triton X114 and 0.1% Triton X100, pH 7.5) until ultraviolet absorption returned to baseline, and then eluted with PBS containing appropriate amount of imidazole (pH 7.5). The His-tagged CD73 extracellular domain protein (hCD73 ECD-His) eluted from the column was collected and dialyzed with PBS in a 4 degrees refrigerator overnight. The dialyzed protein was aseptically filtered at 0.22 µm and then packed at -80 degrees for storage.

6-8 weeks old Balb/c and SJL mice (provided by Slack (SLAC)) were used for CD73 protein immunization, which were raised under SPF conditions after received. During the first immunization, PD-1 protein was emulsified with Freund's complete adjuvant and injected intraperitoneally with 0.25 ml, 100 micrograms of protein per mouse. During the booster immunization, PD-1 protein was emulsified with Freund's incomplete adjuvant and injected intraperitoneally with 0.25 ml, 50 micrograms of protein per mouse. The interval between the first immunization and the first booster immunization were 2 weeks. After that, the intervals between each subsequent immunization were 3 weeks. Blood was collected 7 days after each booster immunization, and the antibody titer and specificity in the serum were detected by ELISA and FACS.

Immunogen 2), the full-length amino acid sequence of human CD73 was cloned into a pLVX-IRES-puro vector. HEK293 cell line was transfected with plasmid, CHOK1 and BW5147 cell line were infected with lentivirus and selectively cultured in the medium containing puromycin for 2 weeks, and then subcloned in 96-well culture plates by limited dilution method. After about 2 weeks, some monoclonal wells were selected and expanded into 6-well plates. The amplified clones were screened with anti-CD73 specific antibodies by flow cytometry. The monoclonal cell line with better growth, higher fluorescence intensity was selected to continue to be expanding cultured and cryopreserved in liquid nitrogen.

6-8 weeks old female Balb/c and SJL/J (provided by Shanghai Slack breeding) were used for CD73 cells immunization, and the mice were raised under SPF conditions after received. The HEK293/Renca stable cell line transfected with human CD73 was expanded to a 75-90% confluence in a T-75 cell culture flask. The medium was aspirated, washed 1-2 times with DMEM/1640 basal medium, and then treated with trypsin and cells were collected. HEK293 cells were washed 1-2 times with DMEM basal medium, and after cell counting, the cells were diluted with PBS to 1x10⁷ cells per milliliter; Renca cells were treated with mitomycin for 4 hours, washed 2-3 times with PBS, and after cell counting, the cells were diluted with PBS to 1x10⁷ cells per milliliter. Each mouse was intraperitoneally injected with 0.5 ml of cell suspension during each immunization. The interval between the first and the second immunization was 2 weeks. After that, the intervals between each subsequent immunization were 3 weeks. Blood was collected 7 days after each boosted immunization, and the antibody titer and specificity in the serum were detected by FACS.

Immunogen 3), CD73 full-length amino acid sequence cDNA was cloned into a pCP vector, and gene gun immunization or electroporation immunization *in vivo* were used.

Gene gun immunization: the plasmids were coated on 1.0 µM gold colloidal bullets, and immunized with Helios gene gun (Bio-rad). The detailed method was developed according to the instructions of Helios gene gun. 6-8 weeks old female Balb/c and SJL/J (provided by Shanghai Slack breeding) were fed under SPF conditions after reception. All mice were immunized with the gene gun through the abdomen for 3-4 times, 4 shots each time, 1.0 µg cDNA amount per shot. The interval between the first immunization and the first booster immunization, as well as that between booster immunizations, was 2 weeks. Blood was collected 7 days after each booster immunization, and the antibody titer in the serum was detected by ELISA or FACS. Usually, the FACS titer of most mice can reach more than 1:1000 after 2-3 times of immunization.

*In vivo* electroporation immunization:6-8 weeks old female Balb/c and SJL/J (provided by Shanghai Slack Breeding) were fed under SPF conditions after reception. All mice were intradermally injected with CD73 full-length amino acid sequence cDNA 3-4 times on both sides of the tail root of the skin, and 50µg/20µl was injected on each side each time. The AgilePµlse system was used to administer electroporation to the injection site immediately, and the detailed method was carried out according to the AgilePµlse (BTX Harvard apparatus) instructions. The interval between the first immunization and the first booster immunization, as well as that between booster immunizations, was 2 weeks. Blood was collected 7 days after each boosted immunization , and the antibody titer in the serum was detected by ELISA or FACS. Usually, the FACS titer of most mice can reach more than 1:1000 after 3-4 times of immunization.

### (3) Preparation of hybridoma cells and antibody screening

Mice whose titers meet the requirements can be selected for cell fusion and hybridoma preparation. Before cell fusion, protein-immunized and genetically immunized mice were injected intraperitoneally with 50 micrograms of purified hCD73 ECD-His each for the last immunization, and 0.5-1x10⁷ cells per intraperitoneal injection were used for the last immunization of cell-immunized mice. After 3-5 days, the mice were sacrificed and splenocytes or lymphocytes were collected. Adding NH₄OH to the final concentration of 1%, the red blood cells in the cell suspension were washed by centrifugation with DMEM basal medium for 2-3 times, and then mixed with mouse myeloma cells SP2/0 at a ratio of 5: 1. The traditional PEG cell fusion method or high-efficiency electrofusion method was used for cell fusion. The fused cells were diluted into DMEM selective medium containing 20% fetal bovine serum, 1xHAT. The mixture was added to a 96-well cell culture plate at 1x10⁵/20 microliters per well, and was placed in a 5% CO₂, 37°C incubator. After 10-14 days, Acumen (microplate cell detection method) was used to screen the supernatant of cell fusion plate, and the positive clones were amplified to 24-well plate for expansion culture. After 2-3 days, the 24-well plate supernatant was analyzed for antibody subtypes, and FACS was used to determine the binding activity to CD73 positive cells. The inhibitory effect of antibody samples on CD73 metabolism of AMP was confirmed by CD73 enzyme activity assay.

According to the screening results of 24-well plate, the required clones were selected and subcloned on 96-well plate by limited dilution method. 7-10 days after subcloning, Acumen was used for preliminary screening, and 3-4 positive monoclones were selected and amplified into 24-well plates to continue culture. After 2-3 days, FACS was used to confirm antigen binding positive and CD73 enzyme activity assay was used to evaluate biological activity. According to the detection results of 24-well plate samples, an optimal clone was selected for expansion culture, liquid nitrogen cryopreservation, antibody production and purification.

### (4) Production and purification of monoclonal antibodies from mouse hybridoma cells

Hybridoma cells were expanded into T-75 cell culture flasks and production medium (Hybridoma seruM free mediuM, Invitrogen) was used for domestication and passage for 2-3 passages. When the hybridoma cells grew well, they were inoculated into the cell culture spinner flask. 200-500 ml of production medium were added to each 2-liter culture flask, and the inoculated cell density was 0.5-1.0 × 10⁵ cells/ml. The bottle was tightly capped and placed on the spinner in the 37°C incubator, and the rotation speed was adjusted to 3 rpm. After 10-14 days of continuous spinning culture, the cell culture medium was collected, the cells are removed by centrifugation or filtration, and filtered with a 0.22-0.45 µm filter to clarify. The treated cell culture supernatant can be purified immediately or cryopreserved at -30°C.

Monoclonal antibodies in the supernatant of hybridoma cell culture can be purified by protein A affinity chromatography column. According to the amount of the sample volume, the corresponding volume of chromatography column was prepared. For small volume purification of 200-300ml, 1-2ml protein A column was required. The protein A column was first equilibrated with equilibrium buffer (Tris-HCl, pH 7.4), and then the culture supernatant was added to the chromatography column, with a flow rate controlled at 3-4ml/min. After loading the sample, the chromatography column was washed with 3-5 column volumes with equilibrium buffer. IgG1 was eluted with eluent (0.1 M sodium citrate buffer, pH 4.5); and other subclasses of IgG were eluted with eluent (0.1 M sodium citrate buffer, pH 3.5). The antibody bound to the column was monitored for elution with an ultraviolet detector. The eluted antibodies (ultraviolet absorption peak) were collected, and 10% volume of 1.0 M Tris-HCl buffer was added to neutralize pH. Then it was immediately dialyzed with PBS overnight, and the fluid was changed once on the next day and the dialysis was continued for 2-3 hours. The dialyzed antibodies were collected, aseptically filtered with a 0.22µm filter, and stored aseptically. Samples were subpacked for detection and analysis of protein concentration, purity, and internal toxicity.

### (5) Detection of lead antibody after purification

### a) Antigen binding reaction

Flow cytometry (FACS) was used to detect the binding of antibodies to CD73 expressing cells in human, cynomolgus monkey and mouse. The CHOK1 stable cell line transfected with human CD73 was expanded to a 75-90% confluence in a T-75 cell culture flask. The medium was aspirated, washed 1-2 times with PBS, and then was treated with trypsin (Tryple express: Life technology) and cells were collected. The cells were washed with PBS buffer for 1-2 times. After counted, the cells were diluted with PBS to 1-2x10⁶ cells per ml, added with 1% fetal bovine serum (FBS) blocking solution, incubated on ice for 20-30 minutes, and then washed twice with HBSS by centrifugation. The collected cells were suspended in the FACS buffer (PBS+2%FBS) to 2x10⁶ cells/ml, and were added as 100 microliters per well to a 96-well FACS reaction plate. The antibody samples to be tested were added with 100 microliters per well, and the plate was incubated at 4 degrees for 1-2 hours. The plate was washed twice with the FACS buffer by centrifugation, added with 100 microliters of fluorescent (Alexa 488)-labeled secondary antibodies per well, and incubated at 4 degrees for 0.5-1.0 hours. The plate was washed 2-3 times with FACS buffer by centrifugation, added with 100µl fixative solution (4% Paraformaldehyde) per well to suspend the cells. 5-10 minutes later, it was washed 1-2 times with FACS buffer by centrifugation. The cells were suspended with 100 microliters of FACS buffer, and FACS (FACSCalibur, BD) was used for detection and the results analysis.

### b) Biological function analysis

CD73 enzyme activity assay. After digestion of CHOK1-hCD73 cells, they were diluted to 2 × 10⁴ cells per milliliter with TM buffer (25mM Tris, 5mM MgCl₂, pH 7.5). The cells were added to a 96-well reaction plate (Corning Cat # 3799) at 100 milliliters per well and were centrifuged to remove the supernatant. At that same time, the antibody to be tested was prepared as a 4× solution with TM buffer, the cells in the 96-well plate were resuspended at 50 microliters per well and incubated at 37 degrees for 30 min. AMP was prepared as a 4 × solution (800 µM) with TM buffer, added to a 96-well plate at 50 microliters per well, mixed evenly, and incubated at 37 degrees for 30 minutes. The 96-well plate was centrifuged at 300 × g, 50 microliters of the supernatant was taken out (which could not absorb cells) and transfered to a 96-well detection plate (Corning cat # 3903). 50 microliters per well of 2 × ATP solution (130 µM) and 100 microliters per well of CellTiter Glo reaction solution were added and mixed evenly. After being placed in a dark place for 10 minutes, the fluorescence value was read on the microplate reader.

Endocytosis assay. CHOK1-hCD73 cells were digested and suspended to 2x10⁶cells/ml with FACS buffer, added to a 96-well reaction plate at 100 ml per well, and centrifuged to remove the supernatant. 20ug/ml of antibody to be tested was added with 100 microliter per well, incubated at 4 degrees for 1-2 hours, and unbound antibodies were washed off with FACS buffer. After being placed at 37 degrees/4 degrees for 0, 1, 2, 4 hours, the plate was taken out, and 1ug/ml of detection antibody with different recognition epitope from the antibody to be tested was added. The plate was incubated at 4 degrees for 1 hour, and then washed 1-2 times with FACS buffer by centrifugation. The cells were suspended with 100 microliters of FACS buffer, FACS (FACSCalibur, BD) was used for detection and the results were analyzed.

T cell proliferation assay. CD4 positive T cells were isolated from human peripheral blood cells (PBMC) by CD4 + T cell isolation kit. The cells were resuspended to 2×10⁶ cells per ml with PBS+1% BSA, added with the same volume of 2×CFSE solution (4µM), mixed well and placed at 37°C for 10 minutes. 40% by volume of FBS was added, mixed well and placed at 37°C for 10 minutes. The cells were washed twice by centrifugation with a large volume of PBS solution. The cells were resuspended to 1.5×10⁶ cells per milliliter with T cell culture medium containing anti-CD2/CD3/CD28 magnetic beads (Miltenyi Biotec, 130-091-441), added to a 96-well plate at 100 milliliters per well. 4× antibody solution to be tested was added at 50 microliters per well, mixed evenly, and incubated at 37°C for 0.5 h. 50 microliters per well of 4×AMP solution (2mM) was added. CD4+ T cell were placed in a 37°C 5% CO₂ incubator for 3-5 days, the results were detected and analyzed by FACS (FACSCalibur, BD).

### (6) Determination of the variable region genes and amino acid sequences of the antibody heavy chain and light chain and analysis of the CDR domain

Determination of amino acid sequence of variable region of light and heavy chain. Total RNA isolation: After the subclonal culture supernatant was tested for antigen binding, 1-5×10⁷ hybridoma cells were collected by centrifugation. The cells were added with 1mL Trizol, mixed and transfered to a 1.5ml centrifuge tube, standing for 5min at room temperature; and added with 0.2ml chloroform, shaked for 15s, after standing for 2min, centrifuged at 4°C, 12000g×5min. Then the supernatant was taken and transfered to a new 1.5ml centrifuge tube; and added with 0.5ml isopropanol, gently mixed in the tube, standing at room temperature for 10min, and centrifuged at 4°C, 12000g×15min. The the supernatant was discarded; and 1ml 75% ethanol was added, and the precipitate was gently washed. The solution was centrifuged at 4°C, 12000g× 5min, and the supernatant was discarded and dried, added with an appropriate amount of DEPC H₂O for dissolution (55°C water bath to promote dissolution for 10min).

Reverse transcription and PCR: 1µg tRNA was taken, and a 20µl system was configured, added with reverse transcriptase and reacted at 42°C for 60 minutes, and the reaction was terminated at 70°C for 10 minutes. 50µl PCR system was configured, comprising 1µl cDNA, 25pmol of each primer, 1µl DNA polymerase and a matching buffer system, 250µmol dNTPs. PCR program was set, comprising pre-denaturation 95°C for 3min, denaturation 95°C for 30s, annealing 55°C for 30s, and extension 72°C for 35s, and additional extension at 72°C for 5 min after 35 cycles. Note: The extension temperature can be adjusted according to the actual situation.

Cloning and sequencing: 5µl of PCR product was taken for agarose gel electrophoresis detection. Column recovery kit was used to purify the positive samples. Ligation reaction was performed: sample 50ng, T vector 50ng, ligase 0.5µl, and buffer 1µl were in a 10µl reaction system, reacted at 16°C for half an hour. 5µl of the ligation product was taken and added to 100µl of competent cells, ice bath for 5 minutes, then heat shock in a 42°C water bath for 1 minute, and put back on ice for 1 minute, and added with 650µl antibiotic-free SOC medium. The cells were resuscitated on a shaker at 37°C at 200 RPM for 30 min, taken out with 200µl and spreaded on LB solid medium containing antibiotics and incubated overnight at 37°C in an incubator. On the next day, primers M13F and M13R on the T vector were used to configure a 30µl PCR system. Colony PCR was performed, a pipette tip was used to dip the colony into the PCR reaction system and pipette, and 0.5µl was aspirated onto another LB solid petri dish containing antibiotics to preserve the strain. After the PCR reaction was over, 5µl of the reaction solution was take out for agar glycogel electrophoresis detection, and the positive samples were sequenced.

### EXAMPLE 1 Preparation of murine antibody specific to CD73

Immunogens including extracellular domain CD73 protein, CD73 recombinant cell line, CD73 DNA vector expression plasmid and the like were prepared.

Mice were immunized with different immunization strategies (protein immunization, cell immunization and gene immunization). Fusion and screening were performed from mice, and clone screening was performed using supernatants of these hybridoma cells. Clones of particular interest were isolated and purified to obtain murine antibodies including 24D6, 37F8, 42A5, 56F12, 57G8, 60G1, 66H6, 69C9, 47F12, 71E10, 77B9, 78E6, 80H7 and 125A4.

### EXAMPLE 2 Identification of murine anti-CD73 antibody

### 2.1 Flow cytometry (FACS) was used to detect the binding of murine antibodies to CD73 expressing cells in human, cynomolgus monkey and mouse.

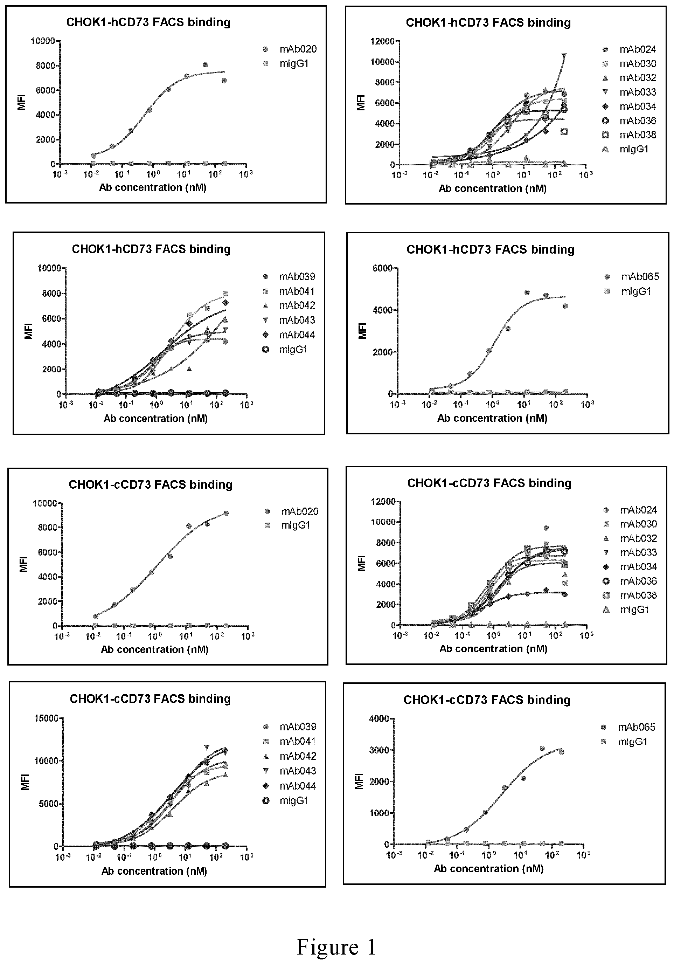

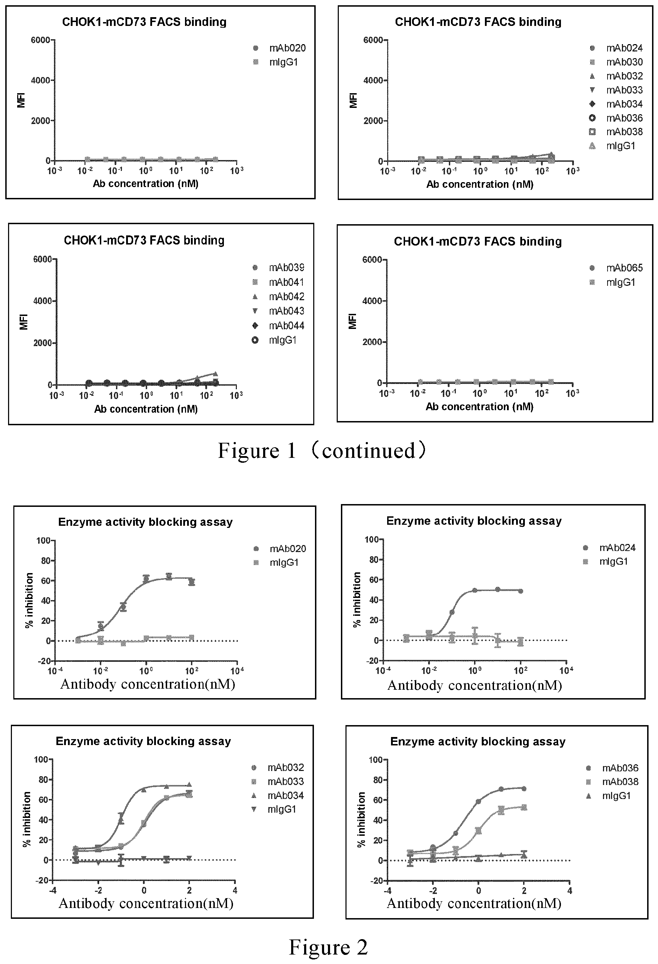

CHOK1-hCD73 (transfected with human CD73), CHOK1-CCD73 (transfected with cynomolgus monkey CD73), CHOK1-mCD73 (transfected with murine CD73) and CHOK1 (human CD73, cynomolgus monkey CD73, murine CD73 negative) cells andCD73 antibody expressed and purified by hybridoma cells were used as the primary antibody, and Alexa Fluor ® 488 donkey anti-mouse IgG (H + L) (Invitrogen, A21202) was used as the secondary antibody. The titration binding curve was produced by the following method:
The CHOK1 stable cell line transfected with human CD73 was expanded to a 75-90% confluence in a T-75 cell culture flask. The medium was aspirated, washed 1-2 times with PBS, and then treated with trypsin (Tryple express: Life technology) and cells were collected. The cells were washed with PBS buffer for 1-2 times. After counted, the cells were diluted with PBS to 1-2x10⁶ cells per ml, added with 1% fetal bovine serum (FBS) blocking solution, incubated on ice for 20-30 minutes, and then washed twice with HBSS by centrifugation. The collected cells were suspended with FACS buffer (PBS+2% FBS) to 2x10⁶ cells/ml, added to a 96-well FACS reaction plate at 100 microliters per well, and centrifuged at 300g for 5 minutes to discard the supernatant. Anti-CD73 antibody was prepared with blocking solution to an initial concentration of 10ug/ml and serially diluted at 8 points. 100 microliter per well of that antibody sample to be tested were added and incubated at 4°C for 1-2 hours. The plate was washed twice with the FACS buffer by centrifugation, added with 100 microliters per well of fluorescent (Alexa 488)-labeled secondary antibodies, and incubated at 4°C for 0.5-1.0 hours. The plate was washed 2-3 times with FACS buffer by centrifugation, added with 100µl fixative solution (4% Paraformaldehyde) per well to suspend the cells. 5-10 minutes later, it was washed 1-2 times with FACS buffer by centrifugation. The cells were suspended with 100 microliters of FACS buffer, FACS (FACSCalibur, BD) was used for detection and the results were analyzed, as shown in Figure 1 and Table 4.

The results in Figure 1 show that: mAb020, 024, 030, 032, 033, 034, 036, 038, 039, 041, 042, 043, 044, 065 antibodies can bind to human CD73 and cynomolgus monkey CD73 on the cell surface, but cannot bind to mouse CD73. The EC50 of binding obtained for each antibody is shown in Table 4.

**Table 4 Flow cytometry (FACS) detects binding of murine antibody to human CD73, cynomolgus monkey CD73 and murine CD73.**

| Antibody number | Clone | FACS | | | | | |
|---|---|---|---|---|---|---|---|
| | | CHOK1-hCD73 | | CHOK1-cCD73 | | CHOK1-mCD73 | |
| | | MAX MFI | EC50 (nM) | MAX MFI | EC50 (nM) | MAX MFI | EC50 (nM) |
| mAb020 | 24D6B4 | 7543.2 | 1.8 | 9961.0 | 3.4 | - | - |
| mAb024 | 37F8B7 | 7242.9 | 0.8 | 11150.6 | 1.5 | - | - |
| mAb030 | 42A5A7 | 6442.8 | 0.9 | 6307.9 | 0.4 | - | - |
| mAb032 | 56F12H8 | 7661.1 | 2.6 | 6053.3 | 0.7 | - | - |
| mAb033 | 57G8H7 | 10600.0 | >10 | 7667.8 | 1.0 | - | - |
| mAb034 | 60G1C8 | 5817.0 | >10 | 3206.4 | 0.2 | - | - |
| mAb036 | 66H6C12 | 5285.8 | 0.3 | 7488.5 | 0.9 | - | - |
| mAb038 | 69C9E12 | 5225.5 | 0.4 | 7395.6 | 0.3 | - | - |
| mAb039 | 47F12C11 | 4379.8 | 1.0 | 10375.9 | 3.3 | - | - |
| mAb041 | 71E10B3 | 8160.6 | 2.9 | 9572.6 | 1.9 | - | - |
| mAb042 | 77B9A3 | 13851.2 | >10 | 8771.5 | 3.9 | - | - |
| mAb043 | 78E6G7 | 4970.9 | 0.8 | 12320.1 | 5.6 | - | - |
| mAb044 | 80H7D6 | 7536.0 | 1.6 | 12349.1 | 3.4 | - | - |
| mAb065 | 125A4E10 | 4653.5 | 1.1 | 3250.8 | 2.5 | - | - |
| mIgG1 | | - | - | - | - | - | - |

### 2.2 Inhibition assay of anti-CD73 mouse antibody on enzyme activity

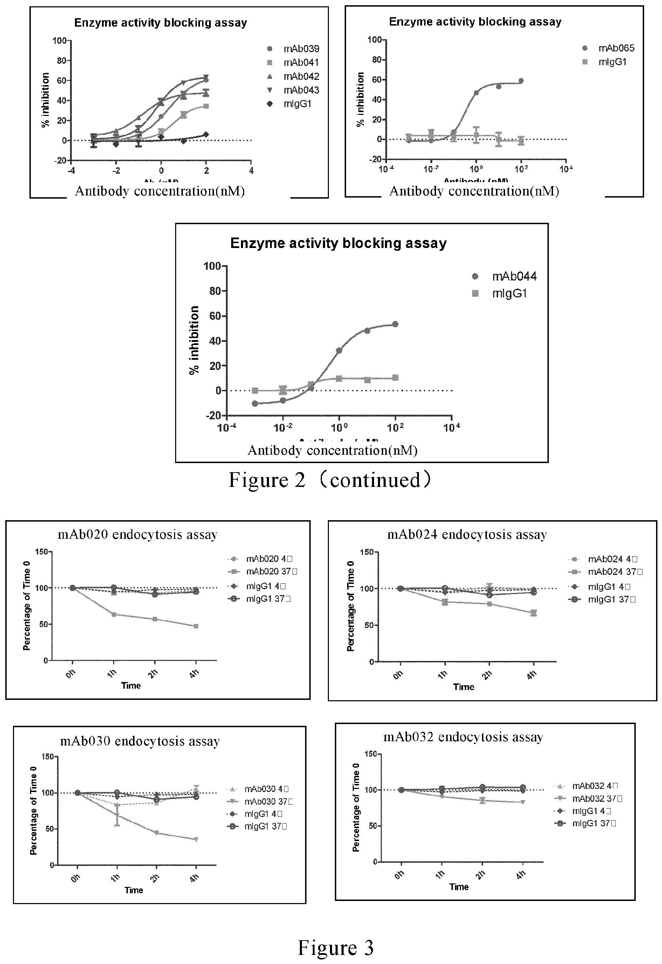

After digestion of CHOK1-hCD73 cells, they were diluted to 2 × 10⁴ cells per milliliter with TM buffer (25mM Tris, 5mM MgCl₂, pH 7.5), added to a 96-well reaction plate (Corning Cat # 3799) at 100 milliliters per well and centrifuged to remove the supernatant. At that same time, the antibody to be tested was prepared as a 4× solution with TM buffer, and serially diluted at 6 points. The cells in the 96-well plate were resuspended at 50 microliters per well and incubated at 37°C for 30 min. AMP was prepared as a 4 × solution (800 µM) with TM buffer, added to a 96-well plate at 50 microliters per well, mixed evenly, and incubated at 37°C for 30 minutes. The 96-well plate was centrifuged at 300 × g, 50 microliters of the supernatant was taken out and transfered to a 96-well detection plate (Corning cat # 3903). 50 microliters per well of 2 × ATP solution (130 µM) and 100 microliters per well of CellTiter Glo reaction solution were added and mixed evenly. After being placed in a dark place for 10 minutes, the fluorescence value was read on the microplate reader. The results are shown in Figure 2 and Table 5.

Figure 2 shows that mAb020, 024, 030, 032, 033, 034, 036, 038, 039, 041, 042, 043, 044, 065 antibodies can all inhibit enzyme activity of human CD73 on the cell surface. The maximum inhibition percentage and IC50 of each antibody are shown in Table 5.

**Table 5 The anti-CD73 murine antibody inhibits the enzyme activity of human CD73.**

| Antibody number | Clone | Enzyme activity blocking assay | |
|---|---|---|---|
| | | MAX % inhibition | IC50 (nM) |
| mAb020 | 24D6B4 | 63.0 | 0.1 |
| mAb024 | 37F8B7 | 49.9 | 0.1 |
| mAb030 | 42A5A7 | 74.6 | 0.1 |
| mAb032 | 56F12H8 | 66.9 | 1.2 |
| mAb033 | 57G8H7 | 64.6 | 1.1 |
| mAb034 | 60G1C8 | 73.7 | 0.1 |
| mAb036 | 66H6C12 | 72.3 | 0.2 |
| mAb038 | 69C9E12 | 53.3 | 1.0 |
| mAb039 | 47F12C11 | 64.6 | 2.2 |
| mAb041 | 71E10B3 | 35.7 | 4.0 |
| mAb042 | 77B9A3 | 47.9 | 0.1 |
| mAb043 | 78E6G7 | 63.7 | 0.6 |
| mAb044 | 80H7D6 | 53.5 | 0.5 |
| mAb065 | 125A4E10 | 56.4 | 0.3 |
| mIgG1 | | - | - |

### 2.3 Endocytosis assay mediated by anti-CD73 murine antibody

CHOK1-hCD73 cells were incubated with CD73 antibody expressed and purified by hybridoma cells at 37°C, and antibody-mediated CD73 endocytosis was detected by FACS.

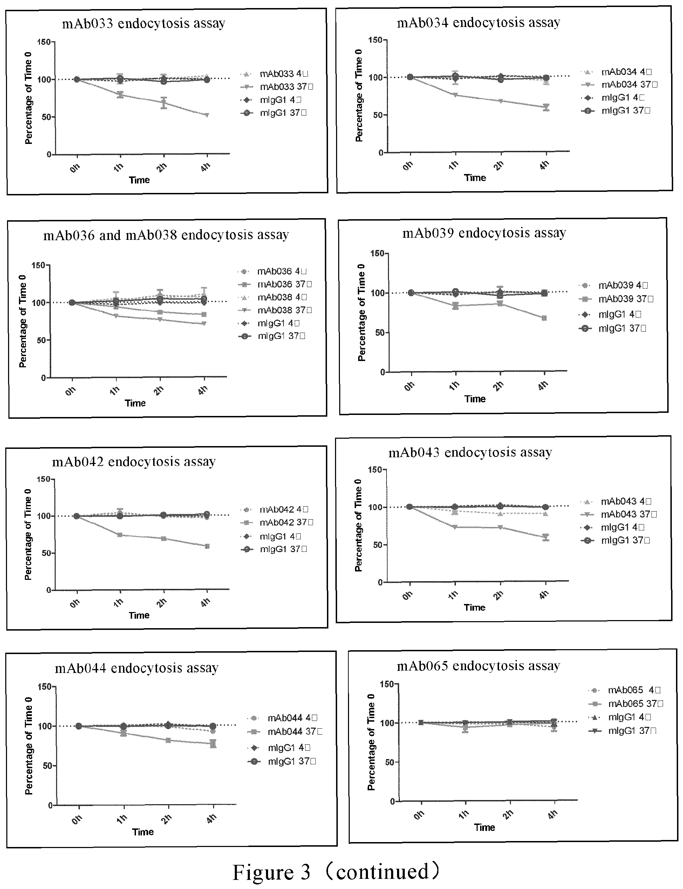

CHOK1-hCD73 cells were digested and suspended to 2x10⁶ cells/ml with FACS buffer, added to a 96-well reaction plate at 100 ml per well, and centrifuged to remove the supernatant. 20ug/ml of antibody to be tested was added with 100 microliter per well, incubated at 4°C for 1-2 hours, and unbound antibodies were washed off with FACS buffer. After being placed at 37°C/4°C for 0, 1, 2, 4 hours, the plate was taken out, and 1ug/ml of detection antibody with different recognition epitope from the antibody to be tested (Alexa 488 labeled) was added.The plate was incubated at 4 °C for 1 hour, and then washed 1-2 times with FACS buffer by centrifugation. The cells were suspended with 100 microliters of FACS buffer, FACS (FACSCalibur, BD) was used for detection and the results were analyzed. The MFI reading value of the data at 4°C for 0h was used as a control, and all calculated values were percentages compared with the control. The results are shown in Figure 3.

Figure 3 shows the time curve of CD73 endocytosis mediated by mAb020, 024, 030, 032, 033, 034, 036, 038, 039, 041, 042, 043, 044, 065 antibodies. The results show that most antibodies can effectively and significantly mediate CD73 endocytosis, such as mab020, 030, 033, 034 and 042.

### 2.4 Anti-CD73 murine antibody restores T cell proliferation

AMP is dephosphorylated by CD73 to form adenosine, which inhibits the proliferation of effector T cells by binding to adenosine receptors on T cells. In this example, the anti-CD73 antibody blocked the action of CD73, inhibited adenosine formation and restored T cell proliferation.

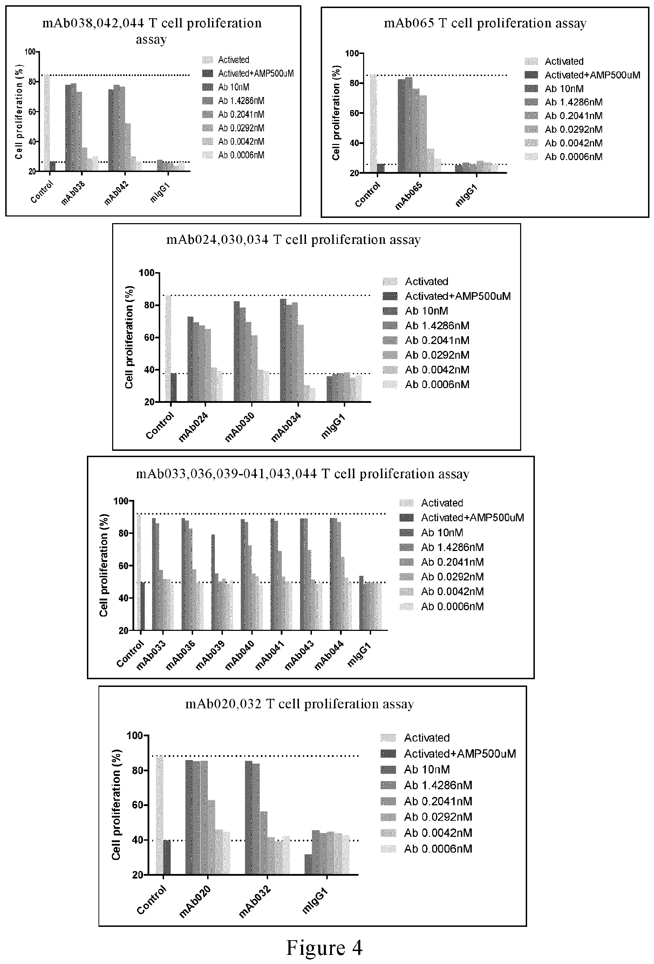

CD4 positive T cells were isolated from human peripheral blood cells (PBMC) by CD4 + T cell isolation kit. The cells were resuspended to 2×10⁶ cells per ml with PBS+1% BSA, added with the same volume of 2×CFSE solution (4µM), mixed well and placed at 37°C for 10 minutes. 40% by volume of FBS was added, mixed well and placed at 37°C for 10 minutes. The cells were washed twice by centrifugation with PBS solution. The cells were resuspended to 1.5×10⁶ cells per milliliter with T cell culture medium (RMPI 1640+10% FBS+1% P/S) containing anti-CD2/CD3/CD28 magnetic beads (Miltenyi Biotec, 130-091-441), added to a 96-well plate at 100 milliliters per well. 4× antibody solution to be tested was added at 50 microliters per well, mixed evenly, and incubated at 37°C for 0.5 h. 50 microliters per well of 4×AMP solution (2mM) was added. CD4+ T cell were placed in a 37°C 5% CO₂ incubator for 3-5 days, the results were detected and analyzed by FACS (FACSCalibur, BD). The results are shown in Figure 4.

The results in Figure 4 show that mAb 020, 024, 030, 032, 033, 034, 036, 038, 039, 041, 042, 043, 044, 065 antibodies all can restore CD4+ T cell proliferation.

### Example 3 Determination of amino acid sequences of light and heavy chain variable regions

Isolation of total RNA: After the supernatant obtained from the subclonal culture of Example 1 was tested for antigen binding (that is, after the verification and activity determination in Examples 2-5), 5×10⁷ hybridoma cells were collected by centrifugation, added with 1mL Trizol and mixed well and transferred to a 1.5mL centrifuge tube, and allowed to stand at room temperature for 5 minutes. The tube was added with 0.2mL chloroform, shaked for 15 seconds, let stand for 2 minutes, and centrifuged at 12000g at 4°C for 5 minutes. The supernatant was taken and transferred to a new 1.5mL centrifuge tube. 0.5 mL of isopropanol was added, and the liquid in the tube was gently mixed. After standing at room temperature for 10 minutes, centrifuged at 12000g for 15 minutes at 4°C, the supernatant was discarded. 1 mL of 75% ethanol (the percentage was volume percentage) was added, and the precipitate was gently washed, centrifuged at 12000g at 4°C for 5 minutes. The supernatant was discarded, and the precipitate was dried, and added with DEPC-treated H₂O for dissolution (55°C water bath to promote dissolution for 10 minutes). The total RNA was obtained.

Reverse transcription and PCR: 1µg of total RNA was taken, and a 20µl system was configured, added with reverse transcriptase and reacted at 42°C for 60 minutes, and the reaction was terminated at 7°C for 10 minutes. 50µl PCR system was configured, comprising 1µl cDNA, 25pmol of each primer, 1µl DNA polymerase and a matching buffer system, 250µmol dNTPs. PCR program was set, comprising pre-denaturation 95°C for 3min, denaturation 95°C for 30s, annealing 55°C for 30s, extension 72°C for 35s, and further extension at 72°C for 5 min after 35 cycles. And the PCR product was obtained. Wherein, the kit used for reverse transcription was PrimeScript RT Master Mix, purchased from Takara, catalog number RR036; the kit used for PCR was Q5 ultra-fidelity enzyme, purchased from NEB, catalog number M0492.

Cloning and sequencing: 5µl of PCR product was taken for agarose gel electrophoresis detection, and the column recovery kit was used to purify the positive samples. Wherein, the recovery kit was NucleoSpin® Gel & PCR Clean-up, purchased from MACHEREY-NAGEL, catalog number 740609. Ligation reaction: 10µl of reaction system containing sample 50ng, T vector 50ng, ligase 0.5µl, and buffer 1µl was reacted for half an hour at 16°C to obtain the ligation product. Wherein, the ligation kit was T4 DNA ligase, purchased from NEB, catalog number M0402. 5µl of ligation product was taken and added into 100µl of competent cells (Ecos 101 competent cells, purchased from Yeastern, catalog number FYE607) in ice bath for 5 minutes. Then heat shock was carried out in a 42°C water bath for 1 minute, and put back on ice for 1 minute, added with 650µl of antibiotic-free SOC medium, resuscitated on a 37°C shaker at 200RPM for 30 minutes. 200µl of the culture was taken and spreaded on LB solid medium containing antibiotics, and incubated overnight at 37°C in an incubator. The next day, the primers M13F and M13R on the T vector were used to configure a 30µl PCR system to perform colony PCR. A pipette tip was used to dip the colony into the PCR reaction system and pipette, and 0.5µl was aspirated onto another piece of 100nM ampicillin LB solid petri dish to save the strain. After the PCR reaction, 5µl was taken out for agarose gel electrophoresis detection, and the positive samples were sequenced. Wherein, the steps of sequencing can be found in Kabat, Sequences of Proteins of Immunological Interest, National Institutes of Health, Bethesda, Md. (1991).

The sequencing results of each antibody are shown in Table A and Table B.

**Table A The sequence numbers (SEQ ID NO.) of VH, VH-CDR1, VH-CDR2, VH-CDR3, VL, VL-CDR1, VL-CDR2, VL-CDR3 of the antibody**

| Clone number | VH Sequence number | VH-CDR1 Sequence number | VH-CDR2 Sequence number | VH-CDR3 Sequence number | VL Sequence number | VL-CDR1 Sequence number | VL-CDR2 Sequence number | VL-CDR3 Sequence number |
|---|---|---|---|---|---|---|---|---|
| | | | | | | | | |
| 42A5A7 | 1 | 3 | 4 | 5 | 6 | 8 | 9 | 10 |
| Hu030-2 | 101 | 3 | 4 | 5 | 103 | 8 | 9 | 10 |
| 56F12H8 | 11 | 13 | 14 | 15 | 16 | 18 | 19 | 20 |
| 66H6C12 | 21 | 23 | 24 | 25 | 26 | 28 | 29 | 30 |
| 24D6B4 | 31 | 33 | 34 | 35 | 36 | 38 | 39 | 40 |
| 60G1C8 | 41 | 43 | 44 | 45 | 46 | 48 | 49 | 50 |
| 69C9E12 | 51 | 53 | 54 | 55 | 56 | 58 | 59 | 60 |
| 71E10B3 | 61 | 63 | 64 | 65 | 66 | 68 | 69 | 70 |
| 77B9A3 | 71 | 73 | 74 | 75 | 76 | 78 | 79 | 80 |
| 80H7D6 | 81 | 83 | 84 | 85 | 86 | 88 | 89 | 90 |
| 125A4E10 | 91 | 93 | 94 | 95 | 96 | 98 | 99 | 100 |

**Table B Sequence Information**

| SEQ ID NO. | Name | Sequence |
|---|---|---|
| 1 | 42A5A7 VH | DIQLQESGPGLVKPSQSLSLTCSVTGYSITSRYYWNWIRQFPGNKLEWM GYITYDDNNNYNPSLKNRISITRDTSKNQFFLKLNSVTPEDTATYYCARG GWDPFLYWGQGTLVTVSA |
| 2 | 42A5A7 VH | DIQLQESGPGLVKPSQSLSLTCSVTGYSITSRYYWNWIRQFPGNKLEWM GYITYDDNNNYNPSLKNRISITRDTSKNQFFLKLNSVTPEDTATYYCARG GWDPFLYWGQGTLVTVSA GATATTCAACTTCAGGAGTCAGGACCTGGCCTCGTGAAACCTTCTCAG TC TCTGTCTCTCACCTGCTCTGTCACTGGCTACTCCATCACCAGTCGTTAT T ACTGGAACTGGATCCGGCAGTTTCCAGGAAACAAACTGGAATGGATG GGCTACATAACCTACGATGATAACAATAACTACAACCCATCTCTCAA AAATCGAATCTCCATCACTCGTGACACTTCTAAGAATCAGTTTTTCCT GAAGTTGAATTCTGTGACTCCTGAGGACACAGCCACATATTACTGTGC AAGAGGGGGCTGGGACCCTTTTCTTTACTGGGGCCAAGGACTCTGG TCACTGTCTCTGCA |
| 3 | VH-CDR1 | SRYYWN |
| 4 | VH-CDR2 | YITYDDNNNYNPSLKN |
| 5 | VH-CDR3 | GGWDPFLY |
| 6 | 42A5A7 VL | DIQMTQSPASLSASVGETVTITCRASDNIYSYLAWYQQKQGKSLQLLVD SAKNLGEGVPSRFSGSGSGTQFYLKINSLQPEDFGVYYCQHYYGTPFTFG GGTKLELK |
| 7 | 42A5A7 VL | DIQMTQSPASLSASVGETVTITCRASDNIYSYLAWYQQKQGKSLQLLVD SAKNLGEGVPSRFSGSGSGTQFYLKINSLQPEDFGVYYCQHYYGTPFTFG GGTKLELK GACATCCAGATGACTCAGTCTCCAGCCTCCCTATCTGCATCTGTGGGA GA AACTGTCACCATCACATGTCGAGCAAGTGACAACATTTACAGTTATTT AGCATGGTATCAACAGAAACAGGGAAAATCTCTTCAGCTCCTGGTCG ATTCTGCAAAAAACTTAGGAGAAGGTGTGCCATCAAGATTCAGTGGC AGTGGATCAGGCACACAGTTTTATCTGAAGATCAACAGCCTGCAGCC TGAGGATTTTGGGGTTTATTACTGTCAACATTATTATGGGACTCCGTT CACGTTCGGTGGTGGGACCAAGCTGGAGCTGAAA |
| 8 | VL-CDR1 | RASDNIYSYLA |
| 9 | VL-CDR2 VL-CDR3 | SAKNLGE |
| 10 | | QHYYGTPFT |
| 11 | 56F12H8 VH | EVQLVESGGDLVKPGGSLKLSCAASGFTFSTHGMSWVRQTPDKRLEWV AIISSDRSDIDYTDNVKGRFTISRDNGKNTLYLQMSSLKSEDTAIYHCAR HRGYDGFYYAMDYWGQGTSVTVSS |
| 12 | 56F12H8 VH | EVQLVESGGDLVKPGGSLKLSCAASGFTFSTHGMSWVRQTPDKRLEWV AIISSDRSDIDYTDNVKGRFTISRDNGKNTLYLQMSSLKSEDTAIYHCAR HRGYDGFYYAMDYWGQGTSVTVSS GAGGTGCAGCTGGTGGAATCTGGGGGAGACTTAGTGAAGCCTGGAGG GTCCCTGAAACTCTCCTGTGCAGCCTCTGGATTCACTTTCAGTACCCA TGGCATGTCTTGGGTTCGTCAGACTCCAGACAAGAGGCTGGAGTGGG TCGCAATTATTAGTAGTGATAGGAGTGACATCGATTATACAGACAAT GTGAAGGGGCGATTCACCATCTCCAGAGACAATGGCAAGAACACCCT GTATTTGCAAATGAGCAGTCTGAAGTCTGAGGACACAGCCATATATC ACTGTGCAAGACATAGGGGTTACGACGGCTTTTACTATGCTATGGACT ACTGGGGTCAAGGAACCTCAGTCACCGTCTCCTCA |
| 13 | VH-CDR1 | THGMS |
| 14 | VH-CDR2 | IISSDRSDIDYTDNVKG |
| 15 | VH-CDR3 | HRGYDGFYYAMDY |
| 16 | 56F12H8 VL | DILMTQSPSSLTVTAGEKVTMSCRSSQSLLNSGNQKNYLTWYQQRPGRP PKLLIYWASTRISGVPDRFAGSGSGTDFTLTISIVQAEDLAVYYCQNDYS YPLTFGSGTKLELR GACATTCTGATGACACAGTCTCCATCCTCCCTGACTGTGACAGCAGGA GAGAAGGTCACTATGAGCTGCAGGTCCAGTCAGAGTCTGTTAAACAG |
| 17 | 56F12H8 VL | GACATTCTGATGACACAGTCTCCATCCTCCCTGACTGTGACAGCAGGA GAGAAGGTCACTATGAGCTGCAGGTCCAGTCAGAGTCTGTTAAACAG |
| | | TGGAAATCAAAAGAACTACTTGACCTGGTATCAGCAGAGACCAGGGC GGCCTCCTAAATTGTTGATCTATTGGGCATCCACTAGGATATCTGGGG TCCCTGATCG CTTCGCAGGCAGTGGATCTGGAACAGATTTCACTCTC ACCATCAGCATTGTGCAGGCTGAGGACCTGGCAGTTTATTACTGTCAG AATGATTATAGTTATCCGCTCACGTTCGGTTCTGGGACCAAACTGGAG CTGAGA |
| 18 | VL-CDR1 | RSSQSLLNSGNQKNYLT |
| 19 | VL-CDR2 | WASTRIS |
| 20 | VL-CDR3 | QNDYSYPLT |
| 21 | 66H6C12 VH | QVQLQQSGAEVVKPGASVKISCKASGYVFSSYWINWVKQRPGKGLEWI GKIFPGNGDTDYNGNFKGKATLTADKSSSTAYMQLSSLTSEDSAVYFCA GFADWGQGTLVTVSA |
| 22 | 66H6C12 VH | CAGGTTCAGCTGCAGCAGTCTGGGGCTGAGGTGGTGAAGCCTGGGGC CTCAGTGAAGATTTCCTGCAAAGCTTCTGGCTACGTATTCAGTAGTTA CTGGATAAACTGGGTGAAGCAGAGGCCTGGAAAGGGTCTTGAGTGGA TTGGAAAGATTTTTCCTGGAAATGGTGATACTGACTATAACGGAAAC TTTAAGGGCAAGGCCACACTGACTGCAGACAAATCCTCCAGCACAGC CTATATGCAGCTCAGTAGCCTGACCTCTGAAGACTCTGCGGTCTATTT CTGTGCTGGCTTTGCTGACTGGGGCCAAGGGACTCTGGTCACTGTCTC TGCA |
| 23 | VH-CDR1 | SYWIN |
| 24 | VH-CDR2 | KIFPGNGDTDYNGNFKG |
| 25 | VH-CDR3 | FAD |
| 26 | 66H6C12 VL | DIQMTQSPSSLSASLGERVSLTCRASQDIGERLIWLQQEPDGTFKRLIYAT SSLDSGVPKRFSGSSSGSDYSLTISRLESEDFVDYYCLQYASSPYTFGGGT KLEIK |
| 27 | 66H6C12 VL | GACATCCAGATGACCCAGTCTCCATCCTCCTTATCTGCCTCGCTGGGA GA AAGAGTCAGTCTCACTTGTCGGGCAAGTCAGGACATTGGTGAAAGGT TAATCTGGCTTCAGCAGGAACCAGATGGAACTTTTAAACGCCTGATCT ACGCCACATCCAGTTTAGATTCTGGTGTCCCCAAAAGGTTCAGTGGCA GTTCGTCTGGGTCAGATTATTCTCTCACCATCAGCAGACTTGAGTCTG AAGATTTTGTTGACTATTACTGTCTACAATATGCTAGTTCTCCGTACA CGTTCGGAGGGGGGACCAAACTGGAAATAAAA |
| 28 | VL-CDR1 | RASQDIGERLI |
| 29 | VL-CDR2 | ATSSLDS |
| 30 | VL-CDR3 | LQYASSPYT |
| 31 | 24D6B4 VH | EVQLQQSGPELVKPGASVKIPCKASGYTFTDYNMDWVKQRHGKSLEWI GDINPNNGGTVYNQKFKGKATLTVDKSSNTAYMELRSLTSEDTAVYYC ARISGTGYWYFDVWGTGTTVTVSS |
| 32 | 24D6B4 VH | GAGGTCCAGCTGCAACAGTCTGGACCTGAGCTGGTGAAGCCTGGGGC TTCAGTGAAGATACCCTGCAAGGCTTCTGGATACACATTCACTGACTA CAACATGGACTGGGTGAAGCAGCGCCATGGAAAGAGCCTTGAGTGG ATTGGAGATATTAATCCTAACAATGGTGGTACTGTCTACAACCAGAA GTTCAAGGGCAAGGCCACATTGACTGTAGACAAGTCCTCCAACACAG CCTACATGGAGCTCCGCAGCCTGACATCTGAGGACACTGCAGTCTATT ACTGTGCAAGAATTTCCGGAACTGGATACTGGTACTTCGATGTCTGGG GCACAGGGACCACGGTCACCGTCTCCTCA |
| 33 | VH-CDR1 | DYNMD |
| 34 | VH-CDR2 | DINPNNGGTVYNQKFKG |
| 35 | VH-CDR3 | ISGTGYWYFDV |
| 36 | 24D6B4 VL | DIQMTQSPASLSVSVGETVTITCRASENIYSNLAWYQQKQGKSPQLLVY GATNLADGVPSRFSGSDSGTQYSLKINSLQSEDFGSYYCQHFWGTPWTF GGGTKLEIK |
| 37 | 24D6B4 | GACATCCAGATGACTCAGTCTCCAGCCTCCCTATCTGTATCTGTGGGA |
| | VL | GAAACTGTCACCATCACATGTCGAGCAAGTGAGAATATTTACAGTAA TTTAGCATGGTATCAGCAGAAACAGGGAAAATCTCCTCAGCTCCTGG TCTATGGTGCAACAAACTTAGCAGATGGTGTGCCATCAAGATTCAGT GGCAGTGATTCAGGCACACAGTATTCCCTCAAGATCAACAGCCTGCA GTCTGAAGATTTTGGGAGTTATTACTGTCAACATTTTTGGGGTACTCC GTGGACGTTCGGTGGAGGCACCAAGCTGGAAATCAAA |
| 38 | VL-CDR1 | RASENIYSNLA |
| 39 | VL-CDR2 | GATNLAD |
| 40 | VL-CDR3 | QHFWGTPWT |
| 41 | 60G1C8 VH | DVQLQESGPGLVKPSQSLSLTCSVTGYSITSRYYWNWIRQFPGNKLEWM GYMTYDGTNNYNPSLTNRISITRDTSKNQFFLKLNSVTAEDTATYFCAR GGWDPFDYWGQGTTLTVSS |
| 42 | 60G1C8 VH | DVQLQESGPGLVKPSQSLSLTCSVTGYSITSRYYWNWIRQFPGNKLEWM GYMTYDGTNNYNPSLTNRISITRDTSKNQFFLKLNSVTAEDTATYFCAR GGWDPFDYWGQGTTLTVSS GATGTACAGCTTCAGGAGTCAGGACCTGGCCTCGTGAAACCTTCTCAGT CTCTGTCTCTCACCTGCTCTGTCACTGGCTACTCCATCACCAGTCGTTATT ACTGGAACTGGATCCGGCAGTTTCCAGGAAACAAACTGGAATGGATGGG GTACATGACCTACGATGGTACCAATAACTACAACCCATCTCTCACAAAT CGAATCTCCATCACTCGTGACACATCTAAGAACCAGTTTTTCCTGAAGTT GAATTCTGTGACTGCTGAGGACACAGCCACATATTTCTGTGCAAGAGGG GGGTGGGACCCTTTTGACTATTGGGGCCAAGGCACCACTCTCACAGTCT CCTCA |
| 43 | VH-CDR1 | SRYYWN |
| 44 | VH-CDR2 | YMTYDGTNNYNPSLTN |
| 45 | VH-CDR3 | GGWDPFDY |
| 46 | 60G1C8 VL | DIQMTQSPAALSASVGETVTITCRASDNIYSYLAWYQQKQGKCPQLLVK NAKTLAEGVPSRFSGSGSGTQFSLRINSLQPDDFGTYYCQHYYGTPLTFG AGTKLELK |
| 47 | 60G1C8 VL | GACATCCAGATGACTCAGTCTCCAGCCGCCCTATCTGCATCTGTGGGAG AGACTGTCACCATCACATGTCGAGCAAGTGACAATATTTACAGTTATTTA GCATGGTATCAACAGAAACAGGGAAAATGTCCTCAGCTCCTGGTCAAAA ATGCAAAAACCTTAGCAGAAGGTGTGCCATCAAGGTTCAGTGGCAGTGG ATCAGGCACACAGTTTTCTCTGAGGATCAACAGCCTGCAGCCTGACGAT TTTGGTACTTATTACTGTCAACATTATTACGGAACTCCACTCACGTTCGG TGCTGGGACCAAACTGGAGCTGAAA |
| 48 | VL-CDR1 | RASDNIYSYLA |
| 49 | VL-CDR2 | NAKTLAE |
| 50 | VL-CDR3 | OHYYGTPLT |
| 51 | 69C9E12 VH | QVQLQQPGTELVNPGASVKLSCKASGYTFTSYWMHWVKQRPGQGLEW IGNINPSNGGTHYNEKFNNKATLTVDKSSSTAYMQLSSLTSEDSAVFYCA RGDYAYDWYFTVWGTGTTVTVSS |
| 52 | 69C9E12 VH | QVQLQQPGTELVNPGASVKLSCKASGYTFTSYWMHWVKQRPGQGLEW IGNINPSNGGTHYNEKFNNKATLTVDKSSSTAYMQLSSLTSEDSAVFYCA RGDYAYDWYFTVWGTGTTVTVSS CAGGTCCAACTGCAGCAGCCTGGGACTGAACTGGTGAACCCTGGGGCTT CAGTGAAGCTGTCCTGCAAGGCTTCTGGCTACACCTTCACCAGCTACTGG ATGCACTGGGTGAAGCAGAGGCCTGGACAAGGCCTTGAGTGGATTGGA AATATTAATCCTAGCAATGGTGGTACTCACTACAATGAGAAATTCAACA ACAAGGCCACACTGACTGTAGACAAATCCTCCAGCACAGCCTACATGCA GCTCAGCAGCCTGACATCTGAGGACTCTGCGGTCTTTTATTGTGCAAGAG GGGACTATGCTTACGACTGGTACTTCACTGTCTGGGGCACGGGGACCAC GGTCACCGTCTCCTCA |
| 53 | VH-CDR1 | SYWMH |
| 54 | VH-CDR2 | NINPSNGGTHYNEKFNN |
| 55 | VH-CDR3 | GDYAYDWYFTV |
| 56 | 69C9E12 VL | DIVLTQSPASLAVSLGQRAIISCKASQSVSFAGTGLMHWYQQKPGQQPK LLIYRVSNLEAGIPTRFSGSGSRTDFTLNIHPVEEDDAATYYCQQNREFP WTFGGGTKLEIK GACATTGTGCTGACCCAATCTCCAGCTTCTTTGGCTGTGTCTCTAGGGCA GAGGGCCATCATCTCCTGCAAGGCCAGCCAAAGTGTCAGTTTTGCTGGT |
| 57 | 69C9E12 VL | GACATTGTGCTGACCCAATCTCCAGCTTCTTTGGCTGTGTCTCTAGGGCA GAGGGCCATCATCTCCTGCAAGGCCAGCCAAAGTGTCAGTTTTGCTGGT |
| | | ACTGGTTTAATGCACTGGTACCAACAGAAACCAGGACAGCAACCCAAAC TCCTCATCTATCGTGTATCCAACCTAGAAGCTGGGATTCCTACCAGGTTT AGTGGCAGTGGGTCTAGGACAGACTTCACCCTCAATATCCATCCTGTGG AGGAAGATGATGCTGCAACCTATTACTGTCAGCAAAATAGGGAATTTCC GTGGACGTTCGGTGGAGGCACCAAGCTGGAAATCAAA |
| 58 | VL-CDR1 | KASQSVSFAGTGLMH |
| 59 | VL-CDR2 | RVSNLEA |
| 60 | VL-CDR3 | QQNREFPWT |
| 61 | 71E10B3 VH | QVQLQQPGTELVKPGASVKLSCKASGYTFTSYWMHWVKQRPGQGLEW IGNINPSNGGTNYNQKFKSKATLTVDKSSNTASMQLSSLTSEDSAVYYC ARGDYGYDWYLDVWGTGTTVTVSS |
| 62 | 71E10B3 VH | QVQLQQPGTELVKPGASVKLSCKASGYTFTSYWMHWVKQRPGQGLEW IGNINPSNGGTNYNQKFKSKATLTVDKSSNTASMQLSSLTSEDSAVYYC ARGDYGYDWYLDVWGTGTTVTVSS CAGGTCCAACTGCAGCAGCCTGGGACTGAACTGGTGAAGCCTGGGGCTT CAGTGAAGCTGTCCTGCAAGGCTTCTGGCTACACCTTCACCAGCTACTGG ATGCACTGGGTGAAGCAGAGGCCTGGACAAGGCCTTGAGTGGATTGGA AATATTAATCCTAGCAATGGTGGAACTAACTACAATCAGAAGTTCAAGA GCAAGGCCACACTGACTGTAGACAAATCTTCCAACACAGCCTCCATGCA GCTCAGCAGCCTGACATCTGAGGACTCTGCGGTCTATTATTGTGCGAGA GGGGACTATGGTTACGACTGGTACCTCGATGTCTGGGGCACAGGGACCA CGGTCACCGTCTCCTCA |
| 63 | VH-CDR1 | SYWMH |
| 64 | VH-CDR2 | NINPSNGGTNYNQKFKS |
| 65 | VH-CDR3 | GDYGYDWYLDV |
| 66 | 71E10B3 VL | DIVLTQSPASLAVSLGQRAIISCKASQSVSFAGPSLMHWYQQKPGQQPKL LIYRTSNLEAGVPTRFSGSGSGTDFTLNIHPVEEDDAATYYCQQNREFPW TFGGGTKLEIK |
| 67 | 71E10B3 VL | DIVLTQSPASLAVSLGQRAIISCKASQSVSFAGPSLMHWYQQKPGQQPKL LIYRTSNLEAGVPTRFSGSGSGTDFTLNIHPVEEDDAATYYCQQNREFPW TFGGGTKLEIK GACATTGTGCTGACCCAATCTCCAGCTTCTTTGGCTGTGTCTCTAGGGCA GAGGGCCATCATCTCCTGCAAGGCCAGCCAAAGTGTCAGTTTTGCTGGT CCTAGTTTAATGCACTGGTACCAACAGAAACCAGGACAGCAACCCAAAC TCCTCATCTATCGTACATCCAACCTAGAAGCTGGGGTTCCTACCAGGTTT AGTGGCAGTGGGTCTGGGACAGACTTCACCCTCAATATCCATCCTGTGG AGGAAGATGATGCTGCAACCTATTACTGTCAGCAAAATAGGGAATTTCC GTGGACGTTCGGTGGAGGCACCAAGCTGGAAATCAAA |
| 68 | VL-CDR1 | KASQSVSFAGPSLMH |
| 69 | VL-CDR2 | RTSNLEA |
| 70 | VL-CDR3 | QQNREFPWT |
| 71 | 77B9A3 VH | QVQLQQSGAGLVKPGASVKLSCKASGYTFTNYWMHWVKQRPGQGLE WIGMIHPNSGSTNNNEKFKNKATLTVDKSSNTAYMQLSSLTSEDSAVYY CARFFFGGYPHYYALDYWGQGTSVTVSS |
| 72 | 77B9A3 VH | QVQLQQSGAGLVKPGASVKLSCKASGYTFTNYWMHWVKQRPGQGLE WIGMIHPNSGSTNNNEKFKNKATLTVDKSSNTAYMQLSSLTSEDSAVYY CARFFFGGYPHYYALDYWGQGTSVTVSS CAGGTCCAACTGCAGCAGTCTGGGGCTGGACTGGTAAAGCCTGGGGCTT CAGTGAAGTTGTCCTGCAAGGCTTCTGGCTACACTTTCACCAACTATTGG ATGCACTGGGTGAAGCAGAGGCCTGGACAAGGCCTTGAGTGGATTGGA ATGATTCATCCTAATAGTGGTAGTACTAATAACAATGAGAAGTTCAAGA ACAAGGCCACACTGACTGTAGACAAATCCTCCAACACAGCCTACATGCA ACTCAGCAGCCTGACATCTGAGGACTCTGCGGTCTATTACTGTGCAAGA TTTTTTTTTGGTGGTTACCCACATTACTATGCTCTGGACTACTGGGGTCA AGGAACCTCAGTCACCGTCTCCTCA |
| 73 | VH-CDR1 | NYWMH |
| 74 | VH-CDR 2 | MIHPNSGSTNNNEKFKN |
| 75 | VH-CDR3 | FFFGGYPHYYALDY |
| 76 | 77B9A3 VL | DIVMTQSQKFMSTTVGDRVSITCKASQNVGTAVAWYQQKPGQSPKLLIY SASDRYTGVPDRLTGSGSGTDFTLTISNVQSEDLADYFCQQYSSFPLFTF GSGTKLVIQ GACATTGTGATGACCCAGTCTCAAAAATTCATGTCCACAACAGTAGGAG ACAGGGTCAGTATCACCTGCAAGGCCAGTCAGAATGTGGGTACTGCTGT AGCCTGGTATCAACAGAAACCAGGACAATCTCCTAAACTACTGATTTAT |
| 77 | 77B9A3 VL | GACATTGTGATGACCCAGTCTCAAAAATTCATGTCCACAACAGTAGGAG ACAGGGTCAGTATCACCTGCAAGGCCAGTCAGAATGTGGGTACTGCTGT AGCCTGGTATCAACAGAAACCAGGACAATCTCCTAAACTACTGATTTAT |
| | | TCAGCATCCGATCGGTACACTGGAGTCCCTGATCGCCTCACAGGCAGTG GATCTGGGACAGATTTCACTCTCACCATTAGCAATGTGCAGTCTGAAGA CCTGGCAGATTATTTCTGTCAGCAATATAGCAGCTTTCCTCTATTCACGT TCGGCTCGGGGACAAAGTTGGTAATACAA |
| 78 | VL-CDR1 | KASQNVGTAVA |
| 79 | VL-CDR2 | SASDRYT |
| 80 | VL-CDR3 | QQYSSFPLFT |
| 81 | 80H7D6 VH | EVKLVESGGGLVQPGGSLRLSCAASGFTFTDYYMSWVRQPPGKALEWL GFIRNKADGSTTEYSASVKGRFTISRDNSQSILYLQMNALRPEDSATYYC TRSPIYFDNWYFDVWGTGTTVTVSS |
| 82 | 80H7D6 VH | EVKLVESGGGLVQPGGSLRLSCAASGFTFTDYYMSWVRQPPGKALEWL GFIRNKADGSTTEYSASVKGRFTISRDNSQSILYLQMNALRPEDSATYYC TRSPIYFDNWYFDVWGTGTTVTVSS GAGGTGAAACTGGTGGAGTCTGGAGGAGGCTTGGTACAGCCTGGGGGTT CTCTGAGACTCTCCTGTGCAGCTTCTGGATTCACCTTCACTGATTACTAC ATGAGCTGGGTCCGCCAGCCTCCAGGGAAGGCACTTGAGTGGTTGGGTT TTATTAGAAACAAAGCTGATGGTTCCACAACAGAGTACAGTGCATCTGT GAAGGGTCGGTTCACCATCTCCAGAGATAATTCCCAAAGCATCCTCTAT CTTCAAATGAATGCCCTGAGACCTGAGGACAGTGCCACTTATTACTGTA CAAGATCCCCTATCTACTTTGATAACTGGTACTTCGATGTCTGGGGCACA GGGACCACGGTCACCGTCTCCTCA |
| 83 | VH-CDR1 | DYYMS |
| 84 | VH-CDR2 | FIRNKADGSTTEYSASVKG |
| 85 | VH-CDR3 | SPIYFDNWYFDV |
| 86 | 80H7D6 VL | DIVMSQSPSSLAVSVGEKVTMTCKSSQSLLYSNNQKNYLAWYQQKPGQ SPKLLIYWASTRESGVPDRFTGSGSGTDFTLTLSSVKAEDLAVYYCQQY YSYPFTFGSGTKLEIK |
| 87 | 80H7D6 VL | DIVMSQSPSSLAVSVGEKVTMTCKSSQSLLYSNNQKNYLAWYQQKPGQ SPKLLIYWASTRESGVPDRFTGSGSGTDFTLTLSSVKAEDLAVYYCQQY YSYPFTFGSGTKLEIK GACATTGTGATGTCACAGTCTCCATCCTCCCTAGCTGTGTCAGTTGGAGA GAAGGTTACTATGACCTGCAAGTCCAGTCAGAGCCTTTTATATAGTAAC AATCAAAAGAACTACTTGGCCTGGTACCAGCAAAAACCAGGGCAGTCTC CTAAACTGCTGATTTACTGGGCATCCACTAGGGAATCTGGGGTCCCTGAT CGCTTCACAGGCAGTGGATCTGGGACAGATTTCACTCTCACCCTCAGCA GTGTGAAGGCTGAAGACCTGGCAGTTTATTACTGTCAGCAATATTATAG CTATCCATTCACGTTCGGCTCGGGGACAAAGTTGGAAATAAAA |
| 88 | VL-CDR1 | KSSQSLLYSNNQKNYLA |
| 89 | VL-CDR2 | WASTRES |
| 90 | VL-CDR3 | QQYYSYPFT |
| 91 | 125A4E10 VH | EVQLQQSGPELVKPGASVKISCKASGYTFTDYYMNWVKQSHGKSLEWI GNINPNNGDTNYNQKFKGKATLTVDKSSSTASMELRSLTSEDSAVYYCA NSGYYYFDYWGQGTTLTVSS |
| 92 | 125A4E10 VH | EVQLQQSGPELVKPGASVKISCKASGYTFTDYYMNWVKQSHGKSLEWI GNINPNNGDTNYNQKFKGKATLTVDKSSSTASMELRSLTSEDSAVYYCA NSGYYYFDYWGQGTTLTVSS GAGGTCCAGCTGCAACAATCTGGACCTGAGCTGGTGAAGCCTGGGGCTT CAGTGAAGATATCCTGTAAGGCTTCTGGATACACGTTCACTGACTACTAC ATGAACTGGGTGAAACAGAGCCATGGAAAGAGCCTTGAGTGGATTGGA AATATTAATCCTAACAATGGTGATACTAACTACAACCAGAAGTTCAAGG GCAAGGCCACATTGACTGTAGACAAGTCCTCCAGCACAGCCTCCATGGA GCTCCGCAGCCTGACATCTGAGGACTCTGCAGTCTATTACTGTGCAAATT CAGGCTACTATTACTTTGACTACTGGGGCCAAGGCACCACTCTCACAGTC TCCTCA |
| 93 | VH-CDR1 | DYYMN |
| 94 | VH-CDR2 | NINPNNGDTNYNQKFKG |
| 95 | VH-CDR3 | ANSGYYYFDY |
| 96 | 125A4E10 VL | DIVMTQSPSSLTVTAGEKVTMSCKSSQSLLNSGNQKNYLTWYQQKPGQP PKVLIYWASTRESGVPDRFTGSGSGTDFTLTISSVQAEDLAVYYCQNDYS YPPTFGGGTNLEIK |
| 97 | 125A4E10 VL | DIVMTQSPSSLTVTAGEKVTMSCKSSQSLLNSGNQKNYLTWYQQKPGQP PKVLIYWASTRESGVPDRFTGSGSGTDFTLTISSVQAEDLAVYYCQNDYS YPPTFGGGTNLEIK GACATTGTGATGACACAGTCTCCATCCTCCCTGACCGTGACAGCAGGAG AGAAGGTCACTATGAGCTGCAAGTCCAGTCAGAGTCTGTTAAACAGTGG AAATCAAAAGAACTACTTGACCTGGTACCAGCAGAAACCAGGGCAGCCT CCTAAAGTATTGATCTACTGGGCATCCACTAGGGAATCTGGGGTCCCTG |
| | | ATCGCTTCACAGGCAGTGGATCTGGAACAGATTTCACTCTCACCATCAG CAGTGTGCAGGCTGAAGACCTGGCAGTTTATTACTGTCAGAATGATTAT AGTTATCCTCCCACGTTCGGAGGGGGGACCAACCTGGAAATAAAA |
| 98 | VL-CDR1 | KSSQSLLNSGNQKNYLT |
| 99 | VL-CDR2 | WASTRES |
| 100 | VL-CDR3 | QNDYSYPPT |
| 101 | Hu030-2 VH | QVQLQESGPGLVKPSETLSLTCTVSGYSITSRYYWNWIRQPPGKGLEWIGYI TYDDNNNYNPSLKNRVTISRDTSKNQFSLKLSSVTAADTAVYYCARGGWD PFLYWGQGTLVTVSS |
| 102 | Hu030-2 VH | QVQLQESGPGLVKPSETLSLTCTVSGYSITSRYYWNWIRQPPGKGLEWIGYI TYDDNNNYNPSLKNRVTISRDTSKNQFSLKLSSVTAADTAVYYCARGGWD PFLYWGQGTLVTVSS CAAGTTCAGCTGCAAGAAAGCGGACCCGGTTTAGTGAAACCCAGCGAG ACTTTATCTTTAACTTGTACCGTGAGCGGCTACAGCATCACCTCTCGTTA CTACTGGAACTGGATCAGACAGCCCCCCGGCAAAGGTTTAGAGTGGATC GGCTACATCACCTACGACGACAACAACAACTACAACCCCTCTTTAAAGA ATCGTGTGACCATCTCTCGTGACACCAGCAAGAACCAGTTCTCTTTAAAG CTGTCCTCCGTGACCGCTGCCGATACCGCCGTGTACTACTGTGCTCGTGG CGGCTGGGATCCCTTTTTATACTGGGGCCAAGGTACACTGGTGACCGTG AGCAGC DIQMTQSPSSLSASVGDRVTITCRASDNIYSYLAWYQQKPGKAPKLLIYSAK NLGEGVPSRFSGSGSGTDFTLTISSLQPEDFATYYCQHYYGTPFTFGGGTKV EIK GACATCCAGATGACCCAGAGCCCTAGCTCTTTAAGCGCCAGCGTGGGCG ATAGGGTGACCATCACTTGTCGTGCCAGCGACAACATCTACAGCTATTT AGCTTGGTACCAGCAGAAGCCCGGCAAGGCCCCCAAGCTGCTGATCTAC AGCGCCAAGAATTTAGGCGAGGGCGTGCCTAGCAGATTCAGCGGAAGC GGCAGCGGCACCGACTTCACTTTAACCATCAGCTCTTTACAGCCCGAGG ACTTCGCCACCTACTACTGCCAGCACTACTACGGCACCCCTTTCACCTTC GGCGGCGGCACCAAGGTGGAGATCAAG |
| 103 | Hu030-2 VL | DIQMTQSPSSLSASVGDRVTITCRASDNIYSYLAWYQQKPGKAPKLLIYSAK NLGEGVPSRFSGSGSGTDFTLTISSLQPEDFATYYCQHYYGTPFTFGGGTKV EIK |
| 104 | Hu030-2 VL | GACATCCAGATGACCCAGAGCCCTAGCTCTTTAAGCGCCAGCGTGGGCG ATAGGGTGACCATCACTTGTCGTGCCAGCGACAACATCTACAGCTATTT AGCTTGGTACCAGCAGAAGCCCGGCAAGGCCCCCAAGCTGCTGATCTAC AGCGCCAAGAATTTAGGCGAGGGCGTGCCTAGCAGATTCAGCGGAAGC GGCAGCGGCACCGACTTCACTTTAACCATCAGCTCTTTACAGCCCGAGG ACTTCGCCACCTACTACTGCCAGCACTACTACGGCACCCCTTTCACCTTC GGCGGCGGCACCAAGGTGGAGATCAAG |

### EXAMPLE 4 Preparation of mouse-human chimeric antibody.

(1) Plasmid construction and preparation: purified CD73 antibody from the culture supernatant of hybridoma cells had been obtained in Example 1, and according to the sequencing results of Example 6, the sequences of heavy chain variable region and light chain variable region of CD73 antibody was identified. The heavy chain variable region sequence of the CD73 antibody was recombined into an expression vector containing the signal peptide and the human heavy chain antibody IgG1-TM constant region (IgG1 contains three site mutations of L234F, L235E and P331S to reduce ADCC and CDC effects) (wherein the IgG1 expression vector was purchased from Invitrogen). Both point mutation modification and recombination steps were conventional steps. The light chain variable region sequence of the CD73 antibody was recombined into an expression vector containing a signal peptide and a human antibody light chain kappa constant region to obtain a recombinant plasmid and verified by sequencing (the sequencing method was the same as that in Example 6). High purity recombinant plasmids with a mass of 500µg or more were extracted using alkaline lysis kit (purchased from MACHEREY-NAGEL), filtered through a 0.22µm filter membrane (purchased from Millopore) for transfection.
(2) Cell transfection:
   293E cells (purchased from Invitrogen) were cultured in Freestyle 293 expression medium (purchased from Invitrogen). The shaker was set to 37°C, 130RPM, and 8% CO₂ (v/v) concentration.
   During transfection, Freestyle 293 expression medium was added with 10% (v/v) F68 (purchased from Invitrogen) to a final concentration of 0.1% (v/v), to obtain Freestyle 293 expression culture containing 0.1% (v/v) F68, that is, medium A.
   5mL of medium A was taken and mixed well with 200µg/mL PEI (purchased from Sigma), to obtain medium B. 5 mL of medium A was taken and mixed well with 100µg/mL of the recombinant plasmid obtained in step (1) to obtain medium C. 5 minutes later, medium B and medium C were combined and mixed, and the mixture was let stand for 15 minutes to obtain a mixture D. 10mL of mixture D was slowly added into 100mL of Freestyle 293 expression medium containing 293E cells, until the cell density of 293E was 1.5×10⁶/mL, and it was shaked while being added, to avoid excessive aggregation of PEI. And the mixture was placed in a shaker for culture. Peptone was added to a final concentration of 0.5% (w/v) on the next day. On days 5-7, the antibody titer of the culture medium was measured. On days 6-7, the supernatant was collected by centrifugation (3500RPM, 30 minutes) and filtered through a 0.22µm filter to obtain the filtered cell supernatant for purification.
(3) Antibody purification: For continuous producted endotoxin-free chromatography columns and Protein A stuffing (purchased from GE), 0.1M NaOH was used for treating for 30 minutes, or 5 column volumes of 0.5M NaOH was used for washing. For column materials and chromatography columns that have not been used for long term, at least 1M NaOH was used for soaking for 1 hour, and non-endotoxic water was used for rinsing to neutrality, and the column material was washed with 10 times the column volume of 1% (v/v) Triton× 100. 5 column volumes of PBS (PBS phosphate buffer, pH 7.2) was used for equilibrate, and the filtered cell supernatant obtained in step (2) was loaded on the column, and the flow-through was collected if necessary. After the samples were loaded, the column was washed with 5 times the column volume of PBS. Elution was performed with 5 times the column volumes of 0.1M pH3.0 Glycine-HCl, and the eluent was collected, and neutralized with 0.5 times the column volume of pH 8.5 1M Tris-HCl (1.5M NaCl). The mouse-human chimeric CD73 antibody was obtained. All the above-mentioned solutions required a new configuration. After the mouse-human chimeric CD73 antibodies harvested, they were dialyzed for 4 hours in 1×PBS to avoid endotoxin contamination. After dialysis, spectrophotometry or a kit was used to determine the concentration, and HPLC-SEC was used to determine the purity of the antibody, and an endotoxin detection kit was used to detect the content of antibody endotoxin.

### EXAMPLE 5 Characterization of chimeric antibody

### 5.1 Identification of the binding of anti-CD73 chimeric antibody to human,cynomolgus monkey and murine CD73 by flow cytometry

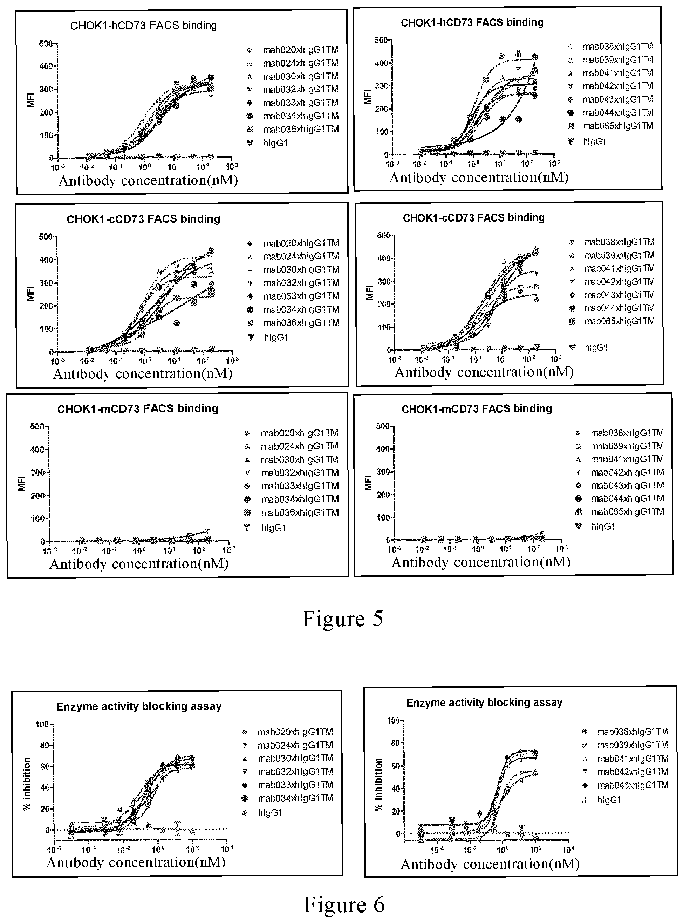

The method was the same as in Example 2. The result is shown in Figure 5.

The results show that mAb020, 024, 030, 032, 033, 034, 036, 038, 039, 041, 042, 043, 044, 065 chimeric antibodies can bind to human CD73 and cynomolgus monkey CD73 on the cell surface, but cannot bind to murine CD73. The EC50 of binding obtained for each antibody is shown in Table 6.

**Table 6 Flow cytometry (FACS) detects the binding of chimeric antibodies to human CD73, cynomolgus monkey CD73 and murine CD73.**

| Antibody Number | **Clone** | **FACS** | | | | | |
|---|---|---|---|---|---|---|---|
| | | **CHOK1-hCD73** | | **CHOK1-cCD73** | | **CHOK1-mCD73** | |
| | | **MAX MFI fold** | **EC50 (nM)** | **MAX MFI fold** | **EC50 (nM)** | **MAX MFI fold** | **EC50 (nM)** |
| mab020xhIgG1 TM | 24D6B4 | 126.4 | 1.6 | 124.1 | 0.7 | - | - |
| mab024xhIgG1 TM | 37F8B7 | 124.4 | 0.9 | 159.4 | 0.9 | - | - |
| mab030xhIgG1TM | 42A5A7 | 113.9 | 1.5 | 138.3 | 0.8 | - | - |
| mab032xhIgG1TM | 56F12H8 | 130.5 | 3.2 | 166.6 | 2.6 | - | - |
| mab033xhIgG1TM | 57G8H7 | 126.1 | 3.5 | 183.1 | 5.2 | - | - |
| mab034xhIgG1TM | 60G1C8 | 153.8 | 5.7 | >150 | >10 | - | - |
| mab036xhIgG1TM | 66H6C12 | 124.9 | 2.0 | 90.2 | 1.5 | - | - |
| mab038xhIgG1TM | 69C9E12 | 119.5 | 1.8 | 167.2 | 2.3 | - | - |
| mab039xhIgG1TM | 47F12C11 | 104.4 | 1.9 | 105.3 | 1.5 | - | - |
| mab041xhIgG1TM | 71E10B3 | 127.1 | 1.3 | 167.4 | 2.0 | - | - |
| mab042xhIgG1TM | 77B9A3 | 135.5 | 2.3 | 132.2 | 5.9 | - | - |
| mab043xhIgG1TM | 78E6G7 | 101.8 | 1.2 | 92.4 | 1.4 | - | - |
| mab044xhIgG1TM | 80H7D6 | >150 | >10 | 174.4 | 7.6 | - | - |
| mab065xhIgG1TM | 125A4E10 | 159.8 | 1.2 | 173.4 | 3.6 | - | - |

### 5.2 Inhibition assay of anti-CD73 chimeric antibody on enzyme activity

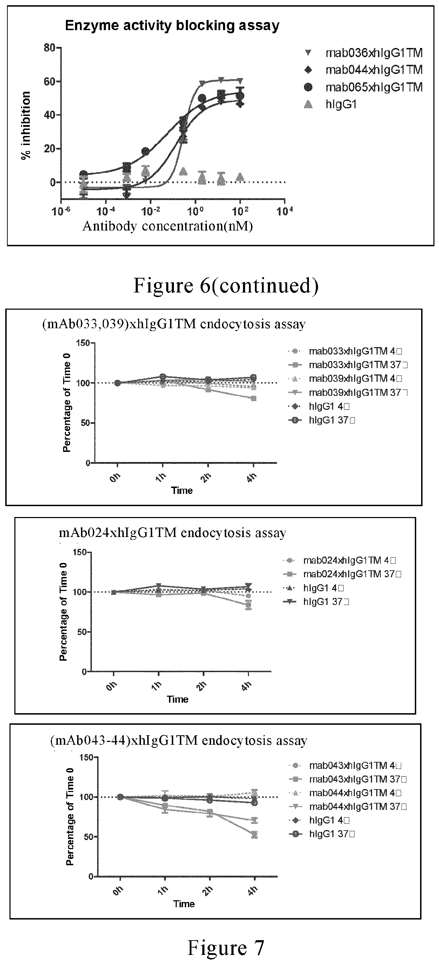

The method was the same as in Example 3. The result is shown in Figure 6.

The result shows that mAb020, 024, 030, 032, 033, 034, 036, 038, 039, 041, 042, 043, 044, 065 chimeric antibodies can all inhibit the enzyme activity of human CD73 on the cell surface. The maximum inhibition percentage and EC50 of each antibody are shown in Table 7.

**Table 7 The anti-CD73 chimeric antibody inhibits the enzyme activity of human CD73.**

| **Antibody number** | **Clone** | **Inhibition assay** of **enzyme activity** | |
|---|---|---|---|
| | | **MAX % inhibition** | **IC50 (nM)** |
| mab020xhIgG1TM | 24D6B4 | 57.7 | 0.6 |
| mab024xhIgG1TM | 37F8B7 | 65.1 | 0.1 |
| mab030xhIgG1TM | 42A5A7 | 67.4 | 0.1 |
| mab032xhIgG1TM | 56F12H8 | 65.1 | 0.3 |
| mab033xhIgG1TM | 57G8H7 | 70.6 | 0.2 |
| mab034xhIgG1TM | 60G1C8 | 61.5 | 0.2 |
| mab036xhIgG1TM | 66H6C12 | 60.6 | 0.3 |
| mab03 8xhIgG1TM | 69C9E12 | 52.4 | 0.8 |
| mab039xhIgG1TM | 47F12C11 | 70.4 | 0.6 |
| mab041xhIgG1TM | 71E10B3 | 54.4 | 0.7 |
| mab042xhIgG1TM | 77B9A3 | 66.3 | 0.5 |
| mab043xhIgG1TM | 78E6G7 | 72.5 | 0.5 |
| mab044xhIgG1TM | 80H7D6 | 49.1 | 0.1 |
| mab065xhIgG1TM | 125A4E10 | 54.7 | 0.1 |
| hIgG1 | | - | - |

### 5.3 Endocytosis assay mediated by anti-CD73 chimeric antibody

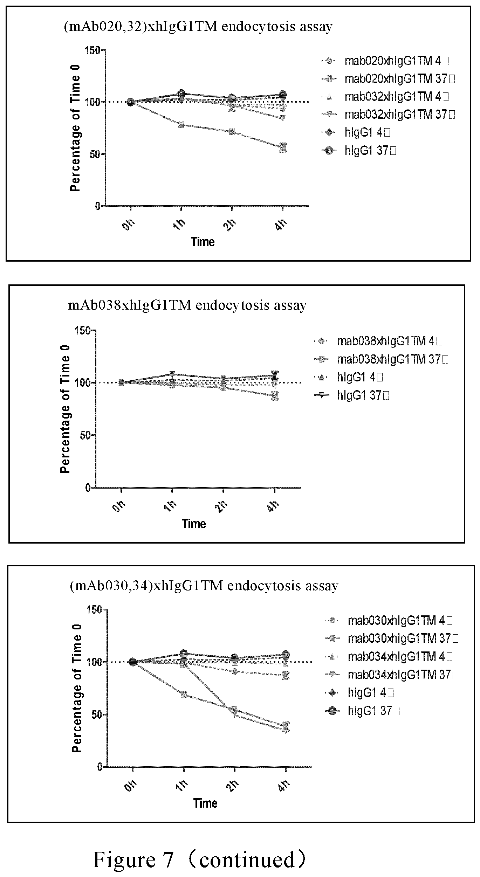

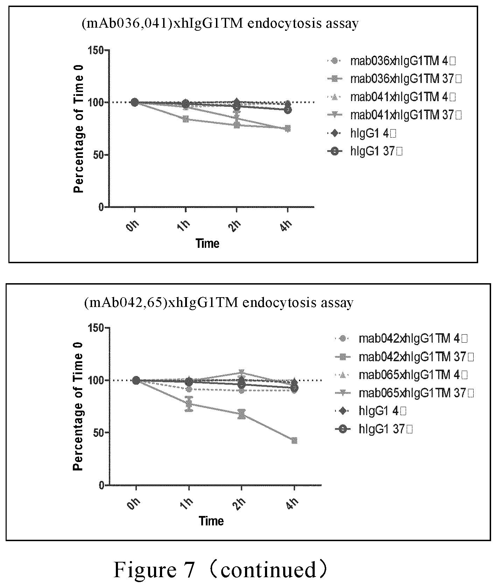

The method was the same as in Example 4. Figure 7 shows the time curve of CD73 endocytosis mediated by mAb020, 024, 030, 032, 033, 034, 036, 038, 039, 041, 042, 043, 044, 065 chimeric antibodies.

The results show that chimeric antibodies mab020, 030, 034 and 042 can effectively and significantly mediate CD73 endocytosis.

### 5.4 Anti-CD73 chimeric antibody restores T cell proliferation.

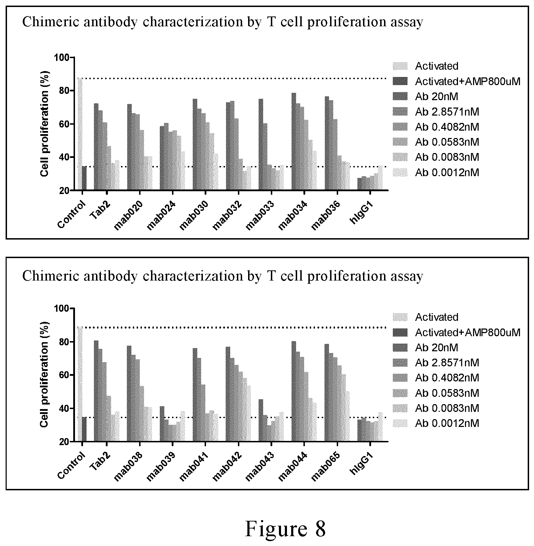

The method was the same as in Example 5. The result is shown in Figure 8.

The results show that mAb 020, 024, 030, 032, 033, 034, 036, 038, 041, 042, 044, 065 chimeric antibodies all can restore the proliferation of CD4+ T cells.

### 5.5 The affinity of anti-CD73 chimeric antibody identified by SPR

Octet Red 96 was selected as the test instrument, and AHC biosensor was selected as the test sensor in this experiment. Anti-human IgG Fc antibody has been immobilized on the AHC sensor, which can be used to directly capture the 15 antibodies in this experiment. Then the sensor was immersed in the analysis sample (antigen). There were five steps in this experiment: 1, Baseline (120s) 2, Loading (capture antibody) (300s) 3, Baseline (120s) 4, Association (binding antigen) (180s) 5, Dissociation (antigen dissociation) (1200s). After the test was completed, the regeneration buffer (Glycine PH1.5) and the neutralization buffer (1*PBS buffer) were alternately immersed for 5 seconds, and the sensor was regenerated by a total of five cycles. The maximum number of sensor regeneration times is 10. The running buffer in this experiment is the sample diluent (1*PBS buffer containing 0.02% Tween20 and 0.1% BSA), that is, the buffer used in the Baseline step, the Dissociation step, and the blank analyte sample. Four sensors are used at one running in this experiment.

Sample treatment: all the antibodies were diluted to the working concentration of 10ug/ml with sample diluent (1*PBS buffer containing 0.02% Tween20 and 0.1% BSA), and the analyte samples (antigens) were diluted to three working concentrations: 200nM, 100nM and 50nM.

For data analysis, Octet Data Analysis (version 7.0) was used to calculate the response signal value (the signal of the coupled analyte sample minus the signal of the blank analyte sample), and a 1:1 binding model was used to fit the data. The results are shown in table 8.

**Table 8 The affinity of anti-CD73 chimeric antibody to hCD73 ECD-His**

| **Antibody number** | **Clone** | **Affinity by Octet** | | |
|---|---|---|---|---|
| | | **Kon (1/Ms)** | **Kdis (1/s)** | **KD (M)** |
| mab020xhIgG1 TM | 24D6B4 | 2.55E+05 | 1.91E-05 | 7.51E-11 |
| mab024xhIgG1TM | 37F8B7 | 4.71E+05 | <1.0 E-05 | <2.12 E-11 |
| mab030xhIgG1TM | 42A5A7 | 3.02E+05 | 1.16E-05 | 3.85E-11 |
| mab032xhIgG1TM | 56F12H8 | 7.00E+04 | 1.95E-05 | 2.79E-10 |
| mab033xhIgG1TM | 57G8H7 | 2.34E+05 | 4.93E-06 | 2.11E-11 |
| mab034xhIgG1TM | 60G1C8 | 4.09E+05 | 2.36E-05 | 5.77E-11 |
| mab036xhIgG1TM | 66H6C12 | 3.01E+05 | 6.42E-06 | 2.13E-11 |
| mab038xhIgG1TM | 69C9E12 | 2.40E+05 | 2.97E-05 | 1.24E-10 |
| mab039xhIgG1TM | 47F12C11 | 2.18E+05 | 5.33E-04 | 2.44E-09 |
| mab041xhIgG!TM | 71E10B3 | 1.48E+05 | 4.41E-05 | 2.98E-10 |
| mab042xhIgG1TM | 77B9A3 | 4.61E+05 | 2.73E-05 | 5.92E-11 |
| mab043xhIgG1TM | 78E6G7 | 7.13E+05 | 1.77E-04 | 2.48E-10 |
| mab044xhIgG1TM | 80H7D6 | 3.83E+05 | 3.29E-05 | 8.60E-11 |
| mab065xhIgG1TM | 125A4E10 | 4.34E+05 | 4.86E-05 | 1.12E-10 |

The results show that the KD values of the tested antibodies are all at the nanomolar level, and are equivalent to or better than MEDI9447, which indicates that these antibodies of the present invention have excellent affinity to human CD73 ECD.

### EXAMPLE 6 Preparation of humanized antibodies

After sequence analysis, the candidate antibody mAb030 has no important hotspot in the heavy chain variable region and light chain variable region. Through sequence alignment (NCBI-Igblast), the germline gene sequence with the highest homology to the heavy chain variable region and light chain variable region of the candidate antibody mAb030 was selected as the variable region transplantation framework: IGHV4-38-2*01 and IGKV1-9 *01. After the human antibody framework was selected, homology modeling was used to predict the key amino acids that may determine the structure in the mouse anti-constant region, and the grafted framework region was designed for back mutation.

According to the above principles, 10 heavy chain variable region sequences (mab030VH. g0, mab030VH. g1, mab030VH. g2, mab030VH. g3) and 4 light chain variable region sequences (mab030VL. g0, mab030VL. g1, mab030VL. g2, mab030VL. g3) were designed. Subsequently, the cross combination was performed for expression, and the following 16 humanized antibodies were obtainedin total, as shown in Table 9.

**Table 9 Design combinations of humanized antibodies**

| | | | | |
|---|---|---|---|---|
| | Hu030VH g0 | Hu030VH g1 | Hu030 VH g2 | Hu030VH g3 |
| Hu030VL g0 | Hu030-1 | Hu030-2 | Hu030-3 | Hu030-4 |
| Hu030VL g1 | Hu030-5 | Hu030-6 | Hu030-7 | Hu030-8 |
| Hu030VL g2 | Hu030-9 | Hu030-10 | Hu030-11 | Hu030-12 |
| Hu030VL g3 | Hu030-13 | Hu030-14 | Hu030-15 | Hu030-16 |

Vector construction: Amplification primers were synthesized by Genewiz, and then the variable regions of light chain and heavy chain were amplified by PCR. A 50 µL reaction system was configured, comprising 50-100ng of heavy chain variable region, light chain variable region, 1ul of forward and reverse primers, 1ul of pfxD enzyme (purchased from invitrogen, 12344-012), 5ul of 10*pfx buff (supplier was identical to pfx enzyme), and water was supplemented to 50 µL. PCR program was set, comprising pre-denaturation 95°C for 5min, denaturation 95°C for 30s, annealing 56°C for 30s, extension 68°C for 30s, and further extension at 68°C for 1 min after 25 cycles. And the PCR product was obtained. 5µl of PCR product was taken for agarose gel electrophoresis detection, and the recovery kit was used to purify the positive samples. Wherein, the recovery kit was PureLink Quick Gel extraction kit, purchased from Qiagen, catalog number 28706.

Expression and purification: ligation reaction was carried out: the reaction system was with a volume of 10µL, containing 20-40ng of fragments to be inserted, 60-100ng of digested expression vector, 1µL of recombinase Exnase (purchased from Vazyme, catalog number C112-01/02), and 2µL of buffer, reacted at 37°C for half an hour to obtain the ligation product, which was the constructed recombinant vector. The buffer was the buffer purchased with the recombinase in set. The heavy chain variable region was directionally cloned into the expression vector containing sequences encoding a signal peptide and human antibody heavy chain IgG4 (S228P) constant region (wherein, the expression vector was purchased from Invitrogen, and the recombination step was a conventional step). The light chain variable region was directionally cloned into the expression vector containing a signal peptide and the human antibody light chain lambda constant region (wherein, the expression vector was purchased from Invitrogen, and the recombination step was a conventional step). 10µL of the ligation product was added to 100µL of competent cells (Ecos 101 competent cells, purchased from Yeastern, catalog number FYE607), and ice bathed for 3 minutes. 80µL was taken out and coated on LB solid medium containing ampicillin, and cultured overnight in incubator at 37°C. The next day, the primers pEFIA and pSV40 for the expression vector were used for configuration of a 30µL PCR system, to perform colony PCR. The colony PCR system was: 1µL of each primer, 15µL of PCR pre-mixture (purchased from Novoprotein), maked up to 30µL. A pipette tip was used to dip the colony into the PCR reaction system and pipette, and 0.5µl was aspirated onto another piece of 100µg/mL ampicillin LB solid petri dish to store the strain. After the PCR reaction, 4.5µl was taken out for agarose gel electrophoresis detection, and the positive samples were sequenced.

The expression vectors with the correct sequences of the recombinant antibody heavy and light chain were amplified, and then transiently transfected into FreeStyleTM 293-F cells (purchased from Invitrogen) to produce antibodies. During transfection, the density of 293-F cells should be 1-1.2×106 cells/mL, and 100mL of cells required 100µg of the above-mentioned constructed recombinant vectors and 200µg of the transfection reagent polyethyleneimine (PEI). The recombinant vector and PEI were added to 5mL culture medium respectively, and the mixture was allowed to stand at room temperature for 5 minutes. After filtration with a 0.22µm filter, the mixture of recombinant vector and PEI was allowed to stand at room temperature for 15 minutes. Then the above mixture was slowly added to the cells, and cultured in a 37°C, 8% (v/v) CO2 incubator at 130 rpm. The culture supernatant and cell pellet were taken every day to detect the expression of antibodies. After 5 days, the cell culture solution was centrifuged at 3000 g for 30 minutes, and the supernatant was collected and filtered with a 0.22µm filter. A 1mL MabSelectTMSuReTM column (purchased from GE Healthcare) was used to purify the monoclonal antibody from 200mL of clear supernatant. MabSelectTMSuReTM column was first equilibrated with equilibration buffer (PBS phosphate buffer, pH 7.2), MabSelectTMSuReTMcolumn. After the sample was loaded, MabSelectTMSuReTM column was washed with the equilibration buffer. The volume of the equilibration buffer was 5 times the volume of the protein A column bed. The monoclonal antibody bound to MabSelectTMSuReTM column was eluted with the eluent (0.1 M glycine hydrochloric acid buffer, pH 3.0). The eluted antibody was collected, added with 10% (v/v) 1.0M Tris-HCl buffer to neutralize the pH. Then immediately dialysis was performed overnight with PBS phosphate buffer. The dialyzed monoclonal antibody was collected, aseptically filtered with a 0.22µm filter, and stored aseptically, thus obtaining purified CD73 humanized antibody. The obtained antibody was tested and analyzed for protein concentration and purity.

The results are shown in Table 10 below. The results show that the yield and purity analysis of humanized antibody are normal.

**Table 10 Expression and purification result of humanized anti-CD73 antibody**

| **Antibody name** | **Volume (mL)** | **Concentration (mg/ml)** | **Yield (mg)** | **Purity (% SEC)** |
|---|---|---|---|---|
| Hu030-1 | 2 | 0.308 | 0.616 | 98.20 |
| Hu030-2 | 2 | 1.61 | 3.22 | 97.54 |
| Hu030-3 | 2 | 1.464 | 2.928 | 97.12 |
| Hu030-4 | 2 | 1.242 | 2.484 | 98.49 |
| Hu030-5 | 2 | 1.478 | 2.956 | 98.42 |
| Hu030-6 | 2 | 1.63 | 3.26 | 97.85 |
| Hu030-7 | 2 | 1.625 | 3.25 | 97.69 |
| Hu030-8 | 2 | 1.775 | 3.55 | 98.84 |
| Hu030-9 | 2 | 1.573 | 3.146 | 97.79 |
| Hu030-10 | 2 | 1.663 | 3.326 | 97.80 |
| Hu030-11 | 2 | 1.504 | 3.008 | 97.44 |
| Hu030-12 | 2 | 1.508 | 3.016 | 98.61 |
| Hu030-13 | 2 | 1.551 | 3.102 | 97.89 |
| Hu030-14 | 2 | 1.548 | 3.096 | 97.68 |
| Hu030-15 | 2 | 1.285 | 2.57 | 97.51 |
| Hu030-16 | 2 | 1.109 | 2.218 | 98.70 |

### EXAMPLE 7 The affinity of humanized anti-CD73 antibodies identified by SPR

The method was the same as in Example 2. The results are shown in Table 11, show that affinity of the humanized antibody was comparable to that of the chimeric antibody.

**Table 11 The affinity of anti-CD73 humanized antibody to hCD73 ECD-His**

| Antibody | KD (M) | kon (1/Ms) | kdis (1/s) | Full R ^ 2 |
|---|---|---|---|---|
| Hu030-6 | < 1.0 E-12 | 7.91E+05 | 6.77E-07 | 0.999 |
| Hu030-3 | 2.18E-12 | 8.33E+05 | 1.81E-06 | 0.9991 |
| Hu030-12 | 6.28E-12 | 5.23E+05 | 3.28E-06 | 0.9991 |
| Hu030-11 | 8.69E-12 | 448100 | 3.90E-06 | 0.9994 |
| Hu030-4 | 6.19E-12 | 7.89E+05 | 4.88E-06 | 0.9992 |
| Hu030-16 | 1.14E-11 | 4.86E+05 | 5.54E-06 | 0.9992 |
| Hu030-13 | 1.19E-11 | 498400 | 5.95E-06 | 0.9982 |
| Hu030-8 | 9.06E-12 | 8.00E+05 | 7.25E-06 | 0.999 |
| Hu030-14 | 2.15E-11 | 4.60E+05 | 9.86E-06 | 0.9994 |
| Hu030-9 | 2.62E-11 | 5.25E+05 | 1.37E-05 | 0.998 |
| Hu030-2 | 2.08E-11 | 7.46E+05 | 1.55E-05 | 0.9988 |
| Hu030-5 | 3.09E-11 | 8.40E+05 | 2.59E-05 | 0.9971 |
| Hu030-1 | 1.82E-10 | 9.28E+05 | 1.69E-04 | 0.9872 |
| Hu030-7 | < 1.0 E-12 | 8.33E+05 | < 1.0 E-07 | 0.999 |
| Hu030-10 | < 1.0 E-12 | 5.00E+05 | < 1.0 E-07 | 0.9992 |
| Hu030-15 | < 1.0 E-12 | 4.51E+05 | < 1.0 E-07 | 0.9994 |
| mab030xhIgG1TM | 3.16E-11 | 7.19E+05 | 2.27E-05 | 0.9991 |

As can be seen from the table, the humanized antibody represented by Hu030-2 has more excellent performance. The amino acid sequence and nucleotide sequence of VH of Hu030-2 are shown in SEQ ID No.: 101 and 102, and the amino acid sequence and nucleotide sequence of VL of Hu030-2 are shown in SEQ ID No.: 103 and 104. The three CDRs of VH and the three CDRs of VL of the Hu030-2 antibody are the same as those of antibody 030 (i.e. clone 42A5A7), which are respectively SEQ ID No.: 3, 4 and 5, and SEQ ID No.: 8, 9 and 10.

### Discussion

At present, the anti-CD73 antibodies of MedImmune and BMS are in the clinical stage. Among them, the antibody MEDI9447 of MedImmune Company is in clinical phase I/II, which is obtained by phage display technology; The antibody BMS-986179 of BMS Company is in clinical phase I/II, which is obtained by immunizing humanized mice. Experimental data in animals show that MEDI9447 alone has no obvious effect on inhibiting CT26 tumor growth, and MEDI9447 combined with anti-PD1 antibody can greatly increase the anti-tumor effect. However, BMS-986179 cannot recognize murine CD73 protein, so there is no reference data *in vivo.* From the experimental data *in vitro,* these two antibodies can inhibit the enzyme activity of CD73 to a certain extent, mediate the endocytosis of CD73, and restore the proliferation of T cells mediated by AMP. But in contrast, MEDI9447 has a weaker effect on promoting endocytosis, and BMS-986179 has a weaker ability to restore the proliferation of T cells mediated by AMP.

From the experimental data *in vitro* provided by MedImmune and BMS, these two antibodies can inhibit the enzyme activity of CD73 to a certain extent, mediate the endocytosis of CD73, and restore the proliferation of T cells mediated by AMP. But in contrast, MEDI9447 has a weaker effect on promoting endocytosis, and BMS-986179 has a weaker ability to restore the proliferation of T cells mediated by AMP. However, the anti-CD73 antibody obtained by the present invention, such as Mab030, can not only show excellent effect on promoting endocytosis, but also provide the possibility for reducing CD73 on the surface of cell membrane. It can also strongly restore the proliferation of T cells mediated by AMP and become an anti-CD73 antibody with excellent performance in all aspects.

All publications mentioned herein are incorporated by reference as if each individual document was cited as a reference in the present application. It should be understood that, after reading the above teachings of the present invention, those skilled in the art can make various modifications and changes. These equivalent forms are also within the scope defined by the claims appended hereto.

## Claims

1. A heavy chain variable region of an antibody, wherein the heavy chain variable region has complementary determining regions or CDRs selected from the group consisting of:
VH-CDR1 as shown in SEQ ID NO. 10n +3,
VH-CDR2 as shown in SEQ ID NO. 10n +4, and
VH-CDR3 as shown in SEQ ID NO. 10n +5;
wherein, each n is independently 0, 1, 2, 3, 4, 5, 6, 7, 8, or 9;
wherein any one of the above amino acid sequences further comprises a derivative sequence that is optionally with at least one amino acid added, deleted, modified and/or substituted, and is capable of retaining the binding affinity to CD73.

2. A heavy chain of an antibody, wherein the heavy chain has the heavy chain variable region of claim 1.

3. A light chain variable region of an antibody, wherein the light chain variable region has complementary determining regions or CDRs selected from the group consisting of:
VL-CDR1 as shown in SEQ ID NO. 10n +8,
VL-CDR2 as shown in SEQ ID NO. 10n +9, and
VL-CDR3 as shown in SEQ ID NO. 10n +10;
wherein, each n is independently 0, 1, 2, 3, 4, 5, 6, 7, 8, or 9;
wherein any one of the above amino acid sequences further comprises a derivative sequence that is optionally with at least one amino acid added, deleted, modified and/or substituted, and is capable of retaining the binding affinity to CD73.

4. A light chain of an antibody, wherein the light chain has the light chain variable region of claim 3.

5. An antibody, wherein the antibody has:
(1) the heavy chain variable region of claim 1; and/or
(2) the light chain variable region of claim 3;
or the antibody has: the heavy chain of claim 2; and/or the light chain of claim 4,
wherein any one of the above amino acid sequences further comprises a derivative sequence that is optionally with at least one amino acid added, deleted, modified and/or substituted, and is capable of retaining the binding affinity to CD73.

6. The antibody of claim 5, wherein the antibody has the heavy chain variable region of claim 1 and the light chain variable region of claim 3;
wherein, the heavy chain variable region and the light chain variable region comprise CDRs selected from the group consisting of:
| VH-CDR 1 Sequence number | VH-CDR 2 Sequence number | VH-CDR 3 Sequence number | VL-CDR 1 Sequence number | VL-CDR 2 Sequence number | VL-CDR 3 Sequence number |
|---|---|---|---|---|---|
| 3 | 4 | 5 | 8 | 9 | 10 |
| 13 | 14 | 15 | 18 | 19 | 20 |
| 23 | 24 | 25 | 28 | 29 | 30 |
| 33 | 34 | 35 | 38 | 39 | 40 |
| 43 | 44 | 45 | 48 | 49 | 50 |
| 53 | 54 | 55 | 58 | 59 | 60 |
| 63 | 64 | 65 | 68 | 69 | 70 |
| 73 | 74 | 75 | 78 | 79 | 80 |
| 83 | 84 | 85 | 88 | 89 | 90 |
| 93 | 94 | 95 | 98 | 99 | 100 |
wherein any one of the above amino acid sequences further comprises a derivative sequence that is optionally with at least one amino acid added, deleted, modified and/or substituted, and is capable of retaining the binding affinity to CD73.

7. The antibody of claim 5, wherein the heavy chain variable region of the antibody has the amino acid sequence shown in SEQ ID NO: 101, and the light chain variable region of the antibody has the amino acid sequence shown in SEQ ID NO: 103.

8. The antibody of claim 6, wherein the antibody is selected from the group consisting of:
| Antibody number | Clone number | VH Sequence number | VL Sequence number |
|---|---|---|---|
| 1 | 42A5A7 | 1 | 6 |
| 2 | 56F12H8 | 11 | 16 |
| 3 | 66H6C12 | 21 | 26 |
| 4 | 24D6B4 | 31 | 36 |
| 5 | 60G1C8 | 41 | 46 |
| 6 | 69C9E12 | 51 | 56 |
| 7 | 71E10B3 | 61 | 66 |
| 8 | 77B9A3 | 71 | 76 |
| 9 | 80H7D6 | 81 | 86 |
| 10 | 125A4E10 | 91 | 96 |
| 11 | Hu030-2 | 101 | 103. |

9. A recombinant protein, wherein the recombinant protein comprises:
(i) the heavy chain variable region of claim 1, the heavy chain of claim 2, the light chain variable region of claim 3, the light chain of claim 4, or the antibody of any one of claims 5-8; and
(ii) an optional tag sequence to assist expression and/or purification.

10. A polynucleotide, wherein the polynucleotide encodes a polypeptide selected from group consisting of:
(1) the heavy chain variable region of claim 1, the heavy chain of claim 2, the light chain variable region of claim 3, the light chain of claim 4, or any one of the antibodies of claims 5-8; and
(2) the recombinant protein of claim 9.

11. The polynucleotide of claim 10, wherein, the polynucleotide encoding the heavy chain variable region is as shown in SEQ ID NO. 2, 12, 22, 32, 42, 52, 62, 72, 82, 92, or 102; and/or, the polynucleotide encoding the light chain variable region is as shown in SEQ ID NO. 7, 17, 27, 37, 47, 57, 67, 77, 87, 97, or 104.

12. The polynucleotide of claim 11, wherein the polynucleotide encoding the heavy chain variable region and the polynucleotide encoding the light chain variable region are selected from the group consisting of:
| Clone number | Sequence number of polynucleotide encoding VH | Sequence number of polynucleotide encoding VL |
|---|---|---|
| 42A5A7 | 2 | 7 |
| 56F12H8 | 12 | 17 |
| 66H6C12 | 22 | 27 |
| 24D6B4 | 32 | 37 |
| 60G1C8 | 42 | 47 |
| 69C9E12 | 52 | 57 |
| 71E10B3 | 62 | 67 |
| 77B9A3 | 72 | 77 |
| 80H7D6 | 82 | 87 |
| 125A4E10 | 92 | 97 |
| Hu030-2 | 102 | 104. |

13. A vector, wherein the vector comprises the polynucleotide according to any one of claims 10-12.

14. A genetically engineered host cell, wherein the host cell contains the vector of claim 13 or the genome thereof is integrated with the polynucleotide of any one of claims 10-12.

15. An antibody conjugate, wherein the antibody conjugate comprises:
(a) an antibody moiety, which is selected from the group consisting of: the heavy chain variable region of claim 1, the heavy chain of claim 2, the light chain variable region of claim 3, the light chain of claim 4, the antibody of any one of claims 5-8, and a combination thereof; and
(b) a coupling moiety coupled to the antibody moiety, which is selected from the group consisting of a detectable label, a drug, a toxin, a cytokine, a radionuclide, an enzyme, or a combination thereof.

16. An immune cell, wherein the immune cell expresses or is exposed outside the cell membrane with the antibody of any one of claims 5-8.

17. A pharmaceutical composition, wherein the pharmaceutical composition comprises:
(i) an active ingredient, wherein the active ingredient is selected from the group consisting of: the heavy chain variable region of claim 1, the heavy chain of claim 2, the light chain variable region of claim 3, the light chain of claim 4, and the antibody of any one of claims 5-8, the recombinant protein of claim 9, the antibody conjugate of claim 15, the immune cell of claim 16, and combinations thereof; and
(ii) a pharmaceutically acceptable carrier.


SEQUENCE LISTING

<110>  SHANGHAI PHARMAEXPLORER CO., LTD.

<120>  CD73 ANTIBODY, PREPARATION METHOD THEREFOR AND APPLICATION THEREOF

<130>  X7-B-63521 EP

<140>  PCT/CN2020/071838
<141>  2020-01-13

<150>  CN201910028562X
<151>  2019-01-11

<160>  104

<170>  PatentIn version 3.5

<210>  1
<211>  117
<212>  PRT
<213>  Artificial Sequence

<400>  1

Asp Ile Gln Leu Gln Glu Ser Gly Pro Gly Leu Val Lys Pro Ser Gln
1               5                   10                  15

Ser Leu Ser Leu Thr Cys Ser Val Thr Gly Tyr Ser Ile Thr Ser Arg
            20                  25                  30

Tyr Tyr Trp Asn Trp Ile Arg Gln Phe Pro Gly Asn Lys Leu Glu Trp
        35                  40                  45

Met Gly Tyr Ile Thr Tyr Asp Asp Asn Asn Asn Tyr Asn Pro Ser Leu
    50                  55                  60

Lys Asn Arg Ile Ser Ile Thr Arg Asp Thr Ser Lys Asn Gln Phe Phe
65                  70                  75                  80

Leu Lys Leu Asn Ser Val Thr Pro Glu Asp Thr Ala Thr Tyr Tyr Cys
                85                  90                  95

Ala Arg Gly Gly Trp Asp Pro Phe Leu Tyr Trp Gly Gln Gly Thr Leu
            100                 105                 110

Val Thr Val Ser Ala
        115

<210>  2
<211>  351
<212>  DNA
<213>  Artificial Sequence

<400>  2

gatattcaac ttcaggagtc aggacctggc ctcgtgaaac cttctcagtc tctgtctctc 60

acctgctctg tcactggcta ctccatcacc agtcgttatt actggaactg gatccggcag 120

tttccaggaa acaaactgga atggatgggc tacataacct acgatgataa caataactac 180

aacccatctc tcaaaaatcg aatctccatc actcgtgaca cttctaagaa tcagtttttc 240

ctgaagttga attctgtgac tcctgaggac acagccacat attactgtgc aagagggggc 300

tgggaccctt ttctttactg gggccaaggg actctggtca ctgtctctgc a 351

<210> 3
<211> 6
<212> PRT
<213> Artificial Sequence

<400> 3

Ser Arg Tyr Tyr Trp Asn
1               5

<210> 4
<211> 16
<212> PRT
<213> Artificial Sequence

<400> 4

Tyr Ile Thr Tyr Asp Asp Asn Asn Asn Tyr Asn Pro Ser Leu Lys Asn
1               5                   10                  15

<210> 5
<211> 8
<212> PRT
<213> Artificial Sequence

<400> 5

Gly Gly Trp Asp Pro Phe Leu Tyr
1               5

<210> 6
<211> 107
<212> PRT
<213> Artificial Sequence

<400> 6

Asp Ile Gln Met Thr Gln Ser Pro Ala Ser Leu Ser Ala Ser Val Gly
1               5                   10                  15

Glu Thr Val Thr Ile Thr Cys Arg Ala Ser Asp Asn Ile Tyr Ser Tyr
            20                  25                  30

Leu Ala Trp Tyr Gln Gln Lys Gln Gly Lys Ser Leu Gln Leu Leu Val
        35                  40                  45

Asp Ser Ala Lys Asn Leu Gly Glu Gly Val Pro Ser Arg Phe Ser Gly
    50                  55                  60

Ser Gly Ser Gly Thr Gln Phe Tyr Leu Lys Ile Asn Ser Leu Gln Pro
65                  70                  75                  80

Glu Asp Phe Gly Val Tyr Tyr Cys Gln His Tyr Tyr Gly Thr Pro Phe
                85                  90                  95

Thr Phe Gly Gly Gly Thr Lys Leu Glu Leu Lys
            100                 105

<210>  7
<211>  321
<212>  DNA
<213>  Artificial Sequence

<400>  7
gacatccaga tgactcagtc tccagcctcc ctatctgcat ctgtgggaga aactgtcacc      60

atcacatgtc gagcaagtga caacatttac agttatttag catggtatca acagaaacag     120

ggaaaatctc ttcagctcct ggtcgattct gcaaaaaact taggagaagg tgtgccatca     180

agattcagtg gcagtggatc aggcacacag ttttatctga agatcaacag cctgcagcct     240

gaggattttg gggtttatta ctgtcaacat tattatggga ctccgttcac gttcggtggt     300

gggaccaagc tggagctgaa a                                               321

<210>  8
<211>  11
<212>  PRT
<213>  Artificial Sequence

<400>  8

Arg Ala Ser Asp Asn Ile Tyr Ser Tyr Leu Ala
1               5                   10

<210>  9
<211>  7
<212>  PRT
<213>  Artificial Sequence

<400>  9

Ser Ala Lys Asn Leu Gly Glu
1               5

<210>  10
<211>  9
<212>  PRT
<213>  Artificial Sequence

<400>  10

Gln His Tyr Tyr Gly Thr Pro Phe Thr
1               5

<210>  11
<211>  122
<212>  PRT
<213>  Artificial Sequence

<400>  11

Glu Val Gln Leu Val Glu Ser Gly Gly Asp Leu Val Lys Pro Gly Gly
1               5                   10                  15

Ser Leu Lys Leu Ser Cys Ala Ala Ser Gly Phe Thr Phe Ser Thr His
            20                  25                  30

Gly Met Ser Trp Val Arg Gln Thr Pro Asp Lys Arg Leu Glu Trp Val
        35                  40                  45

Ala Ile Ile Ser Ser Asp Arg Ser Asp Ile Asp Tyr Thr Asp Asn Val
    50                  55                  60

Lys Gly Arg Phe Thr Ile Ser Arg Asp Asn Gly Lys Asn Thr Leu Tyr
65                  70                  75                  80

Leu Gln Met Ser Ser Leu Lys Ser Glu Asp Thr Ala Ile Tyr His Cys
                85                  90                  95

Ala Arg His Arg Gly Tyr Asp Gly Phe Tyr Tyr Ala Met Asp Tyr Trp
            100                 105                 110

Gly Gln Gly Thr Ser Val Thr Val Ser Ser
        115                 120

<210>  12
<211>  366
<212>  DNA
<213>  Artificial Sequence

<400>  12
gaggtgcagc tggtggaatc tgggggagac ttagtgaagc ctggagggtc cctgaaactc      60

tcctgtgcag cctctggatt cactttcagt acccatggca tgtcttgggt tcgtcagact     120

ccagacaaga ggctggagtg ggtcgcaatt attagtagtg ataggagtga catcgattat     180

acagacaatg tgaaggggcg attcaccatc tccagagaca atggcaagaa caccctgtat     240

ttgcaaatga gcagtctgaa gtctgaggac acagccatat atcactgtgc aagacatagg     300

ggttacgacg gcttttacta tgctatggac tactggggtc aaggaacctc agtcaccgtc     360

tcctca                                                                366

<210>  13
<211>  5
<212>  PRT
<213>  Artificial Sequence

<400>  13

Thr His Gly Met Ser
1               5

<210>  14
<211>  17
<212>  PRT
<213>  Artificial Sequence

<400>  14

Ile Ile Ser Ser Asp Arg Ser Asp Ile Asp Tyr Thr Asp Asn Val Lys
1               5                   10                  15

Gly

<210>  15
<211>  13
<212>  PRT
<213>  Artificial Sequence

<400>  15

His Arg Gly Tyr Asp Gly Phe Tyr Tyr Ala Met Asp Tyr
1               5                   10

<210>  16
<211>  113
<212>  PRT
<213>  Artificial Sequence

<400>  16

Asp Ile Leu Met Thr Gln Ser Pro Ser Ser Leu Thr Val Thr Ala Gly
1               5                   10                  15

Glu Lys Val Thr Met Ser Cys Arg Ser Ser Gln Ser Leu Leu Asn Ser
            20                  25                  30

Gly Asn Gln Lys Asn Tyr Leu Thr Trp Tyr Gln Gln Arg Pro Gly Arg
        35                  40                  45

Pro Pro Lys Leu Leu Ile Tyr Trp Ala Ser Thr Arg Ile Ser Gly Val
    50                  55                  60

Pro Asp Arg Phe Ala Gly Ser Gly Ser Gly Thr Asp Phe Thr Leu Thr
65                  70                  75                  80

Ile Ser Ile Val Gln Ala Glu Asp Leu Ala Val Tyr Tyr Cys Gln Asn
                85                  90                  95

Asp Tyr Ser Tyr Pro Leu Thr Phe Gly Ser Gly Thr Lys Leu Glu Leu
            100                 105                 110

Arg

<210>  17
<211>  339
<212>  DNA
<213>  Artificial Sequence

<400>  17
gacattctga tgacacagtc tccatcctcc ctgactgtga cagcaggaga gaaggtcact      60

atgagctgca ggtccagtca gagtctgtta aacagtggaa atcaaaagaa ctacttgacc     120

tggtatcagc agagaccagg gcggcctcct aaattgttga tctattgggc atccactagg     180

atatctgggg tccctgatcg cttcgcaggc agtggatctg gaacagattt cactctcacc     240

atcagcattg tgcaggctga ggacctggca gtttattact gtcagaatga ttatagttat     300

ccgctcacgt tcggttctgg gaccaaactg gagctgaga                            339

<210>  18
<211>  17
<212>  PRT
<213>  Artificial Sequence

<400>  18

Arg Ser Ser Gln Ser Leu Leu Asn Ser Gly Asn Gln Lys Asn Tyr Leu
1               5                   10                  15

Thr

<210>  19
<211>  7
<212>  PRT
<213>  Artificial Sequence

<400>  19

Trp Ala Ser Thr Arg Ile Ser
1               5

<210>  20

<211>  9
<212>  PRT
<213>  Artificial Sequence

<400>  20

Gln Asn Asp Tyr Ser Tyr Pro Leu Thr
1               5


<210>  21
<211>  112
<212>  PRT
<213>  Artificial Sequence

<400>  21

Gln Val Gln Leu Gln Gln Ser Gly Ala Glu Val Val Lys Pro Gly Ala
1               5                   10                  15


Ser Val Lys Ile Ser Cys Lys Ala Ser Gly Tyr Val Phe Ser Ser Tyr
            20                  25                  30


Trp Ile Asn Trp Val Lys Gln Arg Pro Gly Lys Gly Leu Glu Trp Ile
        35                  40                  45


Gly Lys Ile Phe Pro Gly Asn Gly Asp Thr Asp Tyr Asn Gly Asn Phe
    50                  55                  60


Lys Gly Lys Ala Thr Leu Thr Ala Asp Lys Ser Ser Ser Thr Ala Tyr
65                  70                  75                  80


Met Gln Leu Ser Ser Leu Thr Ser Glu Asp Ser Ala Val Tyr Phe Cys
                85                  90                  95


Ala Gly Phe Ala Asp Trp Gly Gln Gly Thr Leu Val Thr Val Ser Ala
            100                 105                 110


<210>  22
<211>  336
<212>  DNA
<213>  Artificial Sequence

<400>  22
caggttcagc tgcagcagtc tggggctgag gtggtgaagc ctggggcctc agtgaagatt      60

tcctgcaaag cttctggcta cgtattcagt agttactgga taaactgggt gaagcagagg     120

cctggaaagg gtcttgagtg gattggaaag atttttcctg gaaatggtga tactgactat     180

aacggaaact ttaagggcaa ggccacactg actgcagaca aatcctccag cacagcctat     240

atgcagctca gtagcctgac ctctgaagac tctgcggtct atttctgtgc tggctttgct     300

gactggggcc aagggactct ggtcactgtc tctgca                               336

<210>  23
<211>  5
<212>  PRT
<213>  Artificial Sequence

<400>  23

Ser Tyr Trp Ile Asn
1               5

<210>  24
<211>  17
<212>  PRT
<213>  Artificial Sequence

<400>  24

Lys Ile Phe Pro Gly Asn Gly Asp Thr Asp Tyr Asn Gly Asn Phe Lys
1               5                   10                  15

Gly

<210>  25
<211>  3
<212>  PRT
<213>  Artificial Sequence

<400>  25

Phe Ala Asp
1

<210>  26
<211>  107
<212>  PRT
<213>  Artificial Sequence

<400>  26

Asp Ile Gln Met Thr Gln Ser Pro Ser Ser Leu Ser Ala Ser Leu Gly
1               5                   10                  15

Glu Arg Val Ser Leu Thr Cys Arg Ala Ser Gln Asp Ile Gly Glu Arg
            20                  25                  30

Leu Ile Trp Leu Gln Gln Glu Pro Asp Gly Thr Phe Lys Arg Leu Ile
        35                  40                  45

Tyr Ala Thr Ser Ser Leu Asp Ser Gly Val Pro Lys Arg Phe Ser Gly
    50                  55                  60

Ser Ser Ser Gly Ser Asp Tyr Ser Leu Thr Ile Ser Arg Leu Glu Ser
65                  70                  75                  80

Glu Asp Phe Val Asp Tyr Tyr Cys Leu Gln Tyr Ala Ser Ser Pro Tyr
                85                  90                  95

Thr Phe Gly Gly Gly Thr Lys Leu Glu Ile Lys
            100                 105

<210>  27
<211>  321
<212>  DNA
<213>  Artificial Sequence

<400>  27
gacatccaga tgacccagtc tccatcctcc ttatctgcct cgctgggaga aagagtcagt      60

ctcacttgtc gggcaagtca ggacattggt gaaaggttaa tctggcttca gcaggaacca     120

gatggaactt ttaaacgcct gatctacgcc acatccagtt tagattctgg tgtccccaaa     180

aggttcagtg gcagttcgtc tgggtcagat tattctctca ccatcagcag acttgagtct     240

gaagattttg ttgactatta ctgtctacaa tatgctagtt ctccgtacac gttcggaggg     300

gggaccaaac tggaaataaa a                                               321

<210>  28
<211>  11
<212>  PRT
<213>  Artificial Sequence

<400>  28

Arg Ala Ser Gln Asp Ile Gly Glu Arg Leu Ile
1               5                   10

<210>  29
<211>  7
<212>  PRT
<213>  Artificial Sequence

<400>  29

Ala Thr Ser Ser Leu Asp Ser
1               5

<210>  30
<211>  9
<212>  PRT
<213>  Artificial Sequence

<400>  30

Leu Gln Tyr Ala Ser Ser Pro Tyr Thr
1               5

<210>  31

<211>  120
<212>  PRT
<213>  Artificial Sequence

<400>  31

Glu Val Gln Leu Gln Gln Ser Gly Pro Glu Leu Val Lys Pro Gly Ala
1               5                   10                  15

Ser Val Lys Ile Pro Cys Lys Ala Ser Gly Tyr Thr Phe Thr Asp Tyr
            20                  25                  30

Asn Met Asp Trp Val Lys Gln Arg His Gly Lys Ser Leu Glu Trp Ile
        35                  40                  45

Gly Asp Ile Asn Pro Asn Asn Gly Gly Thr Val Tyr Asn Gln Lys Phe
    50                  55                  60

Lys Gly Lys Ala Thr Leu Thr Val Asp Lys Ser Ser Asn Thr Ala Tyr
65                  70                  75                  80

Met Glu Leu Arg Ser Leu Thr Ser Glu Asp Thr Ala Val Tyr Tyr Cys
                85                  90                  95

Ala Arg Ile Ser Gly Thr Gly Tyr Trp Tyr Phe Asp Val Trp Gly Thr
            100                 105                 110

Gly Thr Thr Val Thr Val Ser Ser
        115                 120

<210>  32
<211>  360
<212>  DNA
<213>  Artificial Sequence

<400>  32
gaggtccagc tgcaacagtc tggacctgag ctggtgaagc ctggggcttc agtgaagata      60

ccctgcaagg cttctggata cacattcact gactacaaca tggactgggt gaagcagcgc     120

catggaaaga gccttgagtg gattggagat attaatccta acaatggtgg tactgtctac     180

aaccagaagt tcaagggcaa ggccacattg actgtagaca agtcctccaa cacagcctac     240

atggagctcc gcagcctgac atctgaggac actgcagtct attactgtgc aagaatttcc     300

ggaactggat actggtactt cgatgtctgg ggcacaggga ccacggtcac cgtctcctca     360

<210>  33
<211>  5
<212>  PRT
<213>  Artificial Sequence

<400>  33

Asp Tyr Asn Met Asp
1               5

<210>  34
<211>  17
<212>  PRT
<213>  Artificial Sequence

<400>  34

Asp Ile Asn Pro Asn Asn Gly Gly Thr Val Tyr Asn Gln Lys Phe Lys
1               5                   10                  15

Gly

<210>  35
<211>  11
<212>  PRT
<213>  Artificial Sequence

<400>  35

Ile Ser Gly Thr Gly Tyr Trp Tyr Phe Asp Val
1               5                   10

<210>  36
<211>  107
<212>  PRT
<213>  Artificial Sequence

<400>  36

Asp Ile Gln Met Thr Gln Ser Pro Ala Ser Leu Ser Val Ser Val Gly
1               5                   10                  15

Glu Thr Val Thr Ile Thr Cys Arg Ala Ser Glu Asn Ile Tyr Ser Asn
            20                  25                  30

Leu Ala Trp Tyr Gln Gln Lys Gln Gly Lys Ser Pro Gln Leu Leu Val
        35                  40                  45

Tyr Gly Ala Thr Asn Leu Ala Asp Gly Val Pro Ser Arg Phe Ser Gly
    50                  55                  60

Ser Asp Ser Gly Thr Gln Tyr Ser Leu Lys Ile Asn Ser Leu Gln Ser
65                  70                  75                  80

Glu Asp Phe Gly Ser Tyr Tyr Cys Gln His Phe Trp Gly Thr Pro Trp
                85                  90                  95

Thr Phe Gly Gly Gly Thr Lys Leu Glu Ile Lys

100                 105

<210>  37
<211>  321
<212>  DNA
<213>  Artificial Sequence

<400>  37
gacatccaga tgactcagtc tccagcctcc ctatctgtat ctgtgggaga aactgtcacc      60

atcacatgtc gagcaagtga gaatatttac agtaatttag catggtatca gcagaaacag      120

ggaaaatctc ctcagctcct ggtctatggt gcaacaaact tagcagatgg tgtgccatca      180

agattcagtg gcagtgattc aggcacacag tattccctca agatcaacag cctgcagtct      240

gaagattttg ggagttatta ctgtcaacat ttttggggta ctccgtggac gttcggtgga      300

ggcaccaagc tggaaatcaa a                                                321

<210>  38
<211>  11
<212>  PRT
<213>  Artificial Sequence

<400>  38

Arg Ala Ser Glu Asn Ile Tyr Ser Asn Leu Ala
1               5                   10

<210>  39
<211>  7
<212>  PRT
<213>  Artificial Sequence

<400>  39

Gly Ala Thr Asn Leu Ala Asp
1               5

<210>  40
<211>  9
<212>  PRT
<213>  Artificial Sequence

<400>  40

Gln His Phe Trp Gly Thr Pro Trp Thr
1               5

<210>  41
<211>  117
<212>  PRT
<213>  Artificial Sequence

<400>  41

Asp Val Gln Leu Gln Glu Ser Gly Pro Gly Leu Val Lys Pro Ser Gln

1               5                   10                  15

Ser Leu Ser Leu Thr Cys Ser Val Thr Gly Tyr Ser Ile Thr Ser Arg
            20                  25                  30

Tyr Tyr Trp Asn Trp Ile Arg Gln Phe Pro Gly Asn Lys Leu Glu Trp
        35                  40                  45

Met Gly Tyr Met Thr Tyr Asp Gly Thr Asn Asn Tyr Asn Pro Ser Leu
    50                  55                  60

Thr Asn Arg Ile Ser Ile Thr Arg Asp Thr Ser Lys Asn Gln Phe Phe
65                  70                  75                  80

Leu Lys Leu Asn Ser Val Thr Ala Glu Asp Thr Ala Thr Tyr Phe Cys
                85                  90                  95

Ala Arg Gly Gly Trp Asp Pro Phe Asp Tyr Trp Gly Gln Gly Thr Thr
            100                 105                 110

Leu Thr Val Ser Ser
        115

<210>  42
<211>  351
<212>  DNA
<213>  Artificial Sequence

<400>  42
gatgtacagc ttcaggagtc aggacctggc ctcgtgaaac cttctcagtc tctgtctctc      60

acctgctctg tcactggcta ctccatcacc agtcgttatt actggaactg gatccggcag     120

tttccaggaa acaaactgga atggatgggg tacatgacct acgatggtac caataactac     180

aacccatctc tcacaaatcg aatctccatc actcgtgaca catctaagaa ccagtttttc     240

ctgaagttga attctgtgac tgctgaggac acagccacat atttctgtgc aagagggggg     300

tgggaccctt ttgactattg gggccaaggc accactctca cagtctcctc a              351

<210>  43
<211>  6
<212>  PRT
<213>  Artificial Sequence

<400>  43

Ser Arg Tyr Tyr Trp Asn
1               5

<210>  44
<211>  16

<212>  PRT
<213>  Artificial Sequence

<400>  44

Tyr Met Thr Tyr Asp Gly Thr Asn Asn Tyr Asn Pro Ser Leu Thr Asn
1               5                   10                  15


<210>  45
<211>  8
<212>  PRT
<213>  Artificial Sequence

<400>  45

Gly Gly Trp Asp Pro Phe Asp Tyr
1               5


<210>  46
<211>  107
<212>  PRT
<213>  Artificial Sequence

<400>  46

Asp Ile Gln Met Thr Gln Ser Pro Ala Ala Leu Ser Ala Ser Val Gly
1               5                   10                  15


Glu Thr Val Thr Ile Thr Cys Arg Ala Ser Asp Asn Ile Tyr Ser Tyr
            20                  25                  30


Leu Ala Trp Tyr Gln Gln Lys Gln Gly Lys Cys Pro Gln Leu Leu Val
        35                  40                  45


Lys Asn Ala Lys Thr Leu Ala Glu Gly Val Pro Ser Arg Phe Ser Gly
    50                  55                  60


Ser Gly Ser Gly Thr Gln Phe Ser Leu Arg Ile Asn Ser Leu Gln Pro
65                  70                  75                  80


Asp Asp Phe Gly Thr Tyr Tyr Cys Gln His Tyr Tyr Gly Thr Pro Leu
                85                  90                  95


Thr Phe Gly Ala Gly Thr Lys Leu Glu Leu Lys
            100                 105


<210>  47
<211>  321
<212>  DNA
<213>  Artificial Sequence

<400>  47
gacatccaga tgactcagtc tccagccgcc ctatctgcat ctgtgggaga gactgtcacc        60

atcacatgtc gagcaagtga caatatttac agttatttag catggtatca acagaaacag 120

ggaaaatgtc ctcagctcct ggtcaaaaat gcaaaaacct tagcagaagg tgtgccatca 180

aggttcagtg gcagtggatc aggcacacag ttttctctga ggatcaacag cctgcagcct 240

gacgattttg gtacttatta ctgtcaacat tattacggaa ctccactcac gttcggtgct 300

gggaccaaac tggagctgaa a 321

<210> 48
<211> 11
<212> PRT
<213> Artificial Sequence

<400> 48

Arg Ala Ser Asp Asn Ile Tyr Ser Tyr Leu Ala
1               5                   10

<210> 49
<211> 7
<212> PRT
<213> Artificial Sequence

<400> 49

Asn Ala Lys Thr Leu Ala Glu
1               5

<210> 50
<211> 9
<212> PRT
<213> Artificial Sequence

<400> 50

Gln His Tyr Tyr Gly Thr Pro Leu Thr
1               5

<210> 51
<211> 120
<212> PRT
<213> Artificial Sequence

<400> 51

Gln Val Gln Leu Gln Gln Pro Gly Thr Glu Leu Val Asn Pro Gly Ala
1               5                   10                  15

Ser Val Lys Leu Ser Cys Lys Ala Ser Gly Tyr Thr Phe Thr Ser Tyr
            20                  25                  30

Trp Met His Trp Val Lys Gln Arg Pro Gly Gln Gly Leu Glu Trp Ile
        35                  40                  45

Gly Asn Ile Asn Pro Ser Asn Gly Gly Thr His Tyr Asn Glu Lys Phe
    50                  55                  60

Asn Asn Lys Ala Thr Leu Thr Val Asp Lys Ser Ser Ser Thr Ala Tyr
65                  70                  75                  80

Met Gln Leu Ser Ser Leu Thr Ser Glu Asp Ser Ala Val Phe Tyr Cys
                85                  90                  95

Ala Arg Gly Asp Tyr Ala Tyr Asp Trp Tyr Phe Thr Val Trp Gly Thr
            100                 105                 110

Gly Thr Thr Val Thr Val Ser Ser
        115                 120

<210>  52
<211>  360
<212>  DNA
<213>  Artificial Sequence

<400>  52
caggtccaac tgcagcagcc tgggactgaa ctggtgaacc ctggggcttc agtgaagctg      60

tcctgcaagg cttctggcta caccttcacc agctactgga tgcactgggt gaagcagagg     120

cctggacaag gccttgagtg gattggaaat attaatccta gcaatggtgg tactcactac     180

aatgagaaat tcaacaacaa ggccacactg actgtagaca aatcctccag cacagcctac     240

atgcagctca gcagcctgac atctgaggac tctgcggtct tttattgtgc aagaggggac     300

tatgcttacg actggtactt cactgtctgg ggcacgggga ccacggtcac cgtctcctca     360

<210>  53
<211>  5
<212>  PRT
<213>  Artificial Sequence

<400>  53

Ser Tyr Trp Met His
1               5

<210>  54
<211>  17
<212>  PRT
<213>  Artificial Sequence

<400>  54

Asn Ile Asn Pro Ser Asn Gly Gly Thr His Tyr Asn Glu Lys Phe Asn
1               5                   10                  15

Asn

<210>  55
<211>  11
<212>  PRT
<213>  Artificial Sequence

<400>  55

Gly Asp Tyr Ala Tyr Asp Trp Tyr Phe Thr Val
1               5                   10

<210>  56
<211>  111
<212>  PRT
<213>  Artificial Sequence

<400>  56

Asp Ile Val Leu Thr Gln Ser Pro Ala Ser Leu Ala Val Ser Leu Gly
1               5                   10                  15

Gln Arg Ala Ile Ile Ser Cys Lys Ala Ser Gln Ser Val Ser Phe Ala
            20                  25                  30

Gly Thr Gly Leu Met His Trp Tyr Gln Gln Lys Pro Gly Gln Gln Pro
        35                  40                  45

Lys Leu Leu Ile Tyr Arg Val Ser Asn Leu Glu Ala Gly Ile Pro Thr
    50                  55                  60

Arg Phe Ser Gly Ser Gly Ser Arg Thr Asp Phe Thr Leu Asn Ile His
65                  70                  75                  80

Pro Val Glu Glu Asp Asp Ala Ala Thr Tyr Tyr Cys Gln Gln Asn Arg
                85                  90                  95

Glu Phe Pro Trp Thr Phe Gly Gly Gly Thr Lys Leu Glu Ile Lys
            100                 105                 110

<210>  57
<211>  333
<212>  DNA
<213>  Artificial Sequence

<400>  57
gacattgtgc tgacccaatc tccagcttct ttggctgtgt ctctagggca gagggccatc      60

atctcctgca aggccagcca aagtgtcagt tttgctggta ctggtttaat gcactggtac     120

caacagaaac caggacagca acccaaactc ctcatctatc gtgtatccaa cctagaagct     180

gggattccta ccaggtttag tggcagtggg tctaggacag acttcaccct caatatccat     240

cctgtggagg aagatgatgc tgcaacctat tactgtcagc aaaataggga atttccgtgg     300

acgttcggtg gaggcaccaa gctggaaatc aaa                                    333

<210>  58
<211>  15
<212>  PRT
<213>  Artificial Sequence

<400>  58

Lys Ala Ser Gln Ser Val Ser Phe Ala Gly Thr Gly Leu Met His
1               5                   10                  15

<210>  59
<211>  7
<212>  PRT
<213>  Artificial Sequence

<400>  59

Arg Val Ser Asn Leu Glu Ala
1               5

<210>  60
<211>  9
<212>  PRT
<213>  Artificial Sequence

<400>  60

Gln Gln Asn Arg Glu Phe Pro Trp Thr
1               5

<210>  61
<211>  120
<212>  PRT
<213>  Artificial Sequence

<400>  61

Gln Val Gln Leu Gln Gln Pro Gly Thr Glu Leu Val Lys Pro Gly Ala
1               5                   10                  15

Ser Val Lys Leu Ser Cys Lys Ala Ser Gly Tyr Thr Phe Thr Ser Tyr
            20                  25                  30

Trp Met His Trp Val Lys Gln Arg Pro Gly Gln Gly Leu Glu Trp Ile
        35                  40                  45

Gly Asn Ile Asn Pro Ser Asn Gly Gly Thr Asn Tyr Asn Gln Lys Phe
    50                  55                  60

Lys Ser Lys Ala Thr Leu Thr Val Asp Lys Ser Ser Asn Thr Ala Ser
65                  70                  75                  80

Met Gln Leu Ser Ser Leu Thr Ser Glu Asp Ser Ala Val Tyr Tyr Cys
                85                  90                  95

Ala Arg Gly Asp Tyr Gly Tyr Asp Trp Tyr Leu Asp Val Trp Gly Thr
            100                 105                 110

Gly Thr Thr Val Thr Val Ser Ser
        115                 120

<210>  62
<211>  360
<212>  DNA
<213>  Artificial Sequence

<400>  62
caggtccaac tgcagcagcc tgggactgaa ctggtgaagc ctggggcttc agtgaagctg      60

tcctgcaagg cttctggcta caccttcacc agctactgga tgcactgggt gaagcagagg     120

cctggacaag gccttgagtg gattggaaat attaatccta gcaatggtgg aactaactac     180

aatcagaagt tcaagagcaa ggccacactg actgtagaca aatcttccaa cacagcctcc     240

atgcagctca gcagcctgac atctgaggac tctgcggtct attattgtgc gagaggggac     300

tatggttacg actggtacct cgatgtctgg ggcacaggga ccacggtcac cgtctcctca     360

<210>  63
<211>  5
<212>  PRT
<213>  Artificial Sequence

<400>  63

Ser Tyr Trp Met His
1               5

<210>  64
<211>  17
<212>  PRT
<213>  Artificial Sequence

<400>  64

Asn Ile Asn Pro Ser Asn Gly Gly Thr Asn Tyr Asn Gln Lys Phe Lys
1               5                   10                  15

Ser

<210>  65
<211>  11
<212>  PRT
<213>  Artificial Sequence

<400>  65

Gly Asp Tyr Gly Tyr Asp Trp Tyr Leu Asp Val
1               5                   10


<210>  66
<211>  111
<212>  PRT
<213>  Artificial Sequence

<400>  66

Asp Ile Val Leu Thr Gln Ser Pro Ala Ser Leu Ala Val Ser Leu Gly
1               5                   10                  15


Gln Arg Ala Ile Ile Ser Cys Lys Ala Ser Gln Ser Val Ser Phe Ala
            20                  25                  30


Gly Pro Ser Leu Met His Trp Tyr Gln Gln Lys Pro Gly Gln Gln Pro
        35                  40                  45


Lys Leu Leu Ile Tyr Arg Thr Ser Asn Leu Glu Ala Gly Val Pro Thr
    50                  55                  60


Arg Phe Ser Gly Ser Gly Ser Gly Thr Asp Phe Thr Leu Asn Ile His
65                  70                  75                  80


Pro Val Glu Glu Asp Asp Ala Ala Thr Tyr Tyr Cys Gln Gln Asn Arg
                85                  90                  95


Glu Phe Pro Trp Thr Phe Gly Gly Gly Thr Lys Leu Glu Ile Lys
            100                 105                 110


<210>  67
<211>  333
<212>  DNA
<213>  Artificial Sequence

<400>  67
gacattgtgc tgacccaatc tccagcttct ttggctgtgt ctctagggca gagggccatc      60

atctcctgca aggccagcca aagtgtcagt tttgctggtc ctagtttaat gcactggtac     120

caacagaaac caggacagca acccaaactc ctcatctatc gtacatccaa cctagaagct     180

ggggttccta ccaggtttag tggcagtggg tctgggacag acttcaccct caatatccat     240

cctgtggagg aagatgatgc tgcaacctat tactgtcagc aaaataggga atttccgtgg     300

acgttcggtg gaggcaccaa gctggaaatc aaa                                  333


<210>  68
<211>  15
<212>  PRT

<213>  Artificial Sequence

<400>  68

Lys Ala Ser Gln Ser Val Ser Phe Ala Gly Pro Ser Leu Met His
1               5                   10                  15


<210>  69
<211>  7
<212>  PRT
<213>  Artificial Sequence

<400>  69

Arg Thr Ser Asn Leu Glu Ala
1               5


<210>  70
<211>  9
<212>  PRT
<213>  Artificial Sequence

<400>  70

Gln Gln Asn Arg Glu Phe Pro Trp Thr
1               5


<210>  71
<211>  123
<212>  PRT
<213>  Artificial Sequence

<400>  71

Gln Val Gln Leu Gln Gln Ser Gly Ala Gly Leu Val Lys Pro Gly Ala
1               5                   10                  15


Ser Val Lys Leu Ser Cys Lys Ala Ser Gly Tyr Thr Phe Thr Asn Tyr
            20                  25                  30


Trp Met His Trp Val Lys Gln Arg Pro Gly Gln Gly Leu Glu Trp Ile
        35                  40                  45


Gly Met Ile His Pro Asn Ser Gly Ser Thr Asn Asn Asn Glu Lys Phe
    50                  55                  60


Lys Asn Lys Ala Thr Leu Thr Val Asp Lys Ser Ser Asn Thr Ala Tyr
65                  70                  75                  80


Met Gln Leu Ser Ser Leu Thr Ser Glu Asp Ser Ala Val Tyr Tyr Cys
                85                  90                  95


Ala Arg Phe Phe Phe Gly Gly Tyr Pro His Tyr Tyr Ala Leu Asp Tyr
            100                 105                 110

Trp Gly Gln Gly Thr Ser Val Thr Val Ser Ser
        115                 120

<210>  72
<211>  369
<212>  DNA
<213>  Artificial Sequence

<400>  72
caggtccaac tgcagcagtc tggggctgga ctggtaaagc ctggggcttc agtgaagttg        60

tcctgcaagg cttctggcta cactttcacc aactattgga tgcactgggt gaagcagagg       120

cctggacaag gccttgagtg gattggaatg attcatccta atagtggtag tactaataac       180

aatgagaagt tcaagaacaa ggccacactg actgtagaca aatcctccaa cacagcctac       240

atgcaactca gcagcctgac atctgaggac tctgcggtct attactgtgc aagatttttt       300

tttggtggtt acccacatta ctatgctctg gactactggg gtcaaggaac ctcagtcacc       360

gtctcctca                                                               369

<210>  73
<211>  5
<212>  PRT
<213>  Artificial Sequence

<400>  73

Asn Tyr Trp Met His
1               5

<210>  74
<211>  17
<212>  PRT
<213>  Artificial Sequence

<400>  74

Met Ile His Pro Asn Ser Gly Ser Thr Asn Asn Asn Glu Lys Phe Lys
1               5                   10                  15

Asn

<210>  75
<211>  14
<212>  PRT
<213>  Artificial Sequence

<400>  75

Phe Phe Phe Gly Gly Tyr Pro His Tyr Tyr Ala Leu Asp Tyr
1               5                   10

<210>  76
<211>  108
<212>  PRT
<213>  Artificial Sequence

<400>  76

Asp Ile Val Met Thr Gln Ser Gln Lys Phe Met Ser Thr Thr Val Gly
1               5                   10                  15

Asp Arg Val Ser Ile Thr Cys Lys Ala Ser Gln Asn Val Gly Thr Ala
            20                  25                  30

Val Ala Trp Tyr Gln Gln Lys Pro Gly Gln Ser Pro Lys Leu Leu Ile
        35                  40                  45

Tyr Ser Ala Ser Asp Arg Tyr Thr Gly Val Pro Asp Arg Leu Thr Gly
    50                  55                  60

Ser Gly Ser Gly Thr Asp Phe Thr Leu Thr Ile Ser Asn Val Gln Ser
65                  70                  75                  80

Glu Asp Leu Ala Asp Tyr Phe Cys Gln Gln Tyr Ser Ser Phe Pro Leu
                85                  90                  95

Phe Thr Phe Gly Ser Gly Thr Lys Leu Val Ile Gln
            100                 105

<210>  77
<211>  324
<212>  DNA
<213>  Artificial Sequence

<400>  77
gacattgtga tgacccagtc tcaaaaattc atgtccacaa cagtaggaga cagggtcagt      60

atcacctgca aggccagtca gaatgtgggt actgctgtag cctggtatca acagaaacca     120

ggacaatctc ctaaactact gatttattca gcatccgatc ggtacactgg agtccctgat     180

cgcctcacag gcagtggatc tgggacagat ttcactctca ccattagcaa tgtgcagtct     240

gaagacctgg cagattattt ctgtcagcaa tatagcagct ttcctctatt cacgttcggc     300

tcggggacaa agttggtaat acaa                                            324

<210>  78
<211>  11
<212>  PRT
<213>  Artificial Sequence

<400>  78

Lys Ala Ser Gln Asn Val Gly Thr Ala Val Ala

1               5                   10


<210>  79
<211>  7
<212>  PRT
<213>  Artificial Sequence

<400>  79

Ser Ala Ser Asp Arg Tyr Thr
1               5


<210>  80
<211>  10
<212>  PRT
<213>  Artificial Sequence

<400>  80

Gln Gln Tyr Ser Ser Phe Pro Leu Phe Thr
1               5                   10


<210>  81
<211>  123
<212>  PRT
<213>  Artificial Sequence

<400>  81

Glu Val Lys Leu Val Glu Ser Gly Gly Gly Leu Val Gln Pro Gly Gly
1               5                   10                  15

Ser Leu Arg Leu Ser Cys Ala Ala Ser Gly Phe Thr Phe Thr Asp Tyr
            20                  25                  30

Tyr Met Ser Trp Val Arg Gln Pro Pro Gly Lys Ala Leu Glu Trp Leu
        35                  40                  45

Gly Phe Ile Arg Asn Lys Ala Asp Gly Ser Thr Thr Glu Tyr Ser Ala
    50                  55                  60

Ser Val Lys Gly Arg Phe Thr Ile Ser Arg Asp Asn Ser Gln Ser Ile
65                  70                  75                  80

Leu Tyr Leu Gln Met Asn Ala Leu Arg Pro Glu Asp Ser Ala Thr Tyr
                85                  90                  95

Tyr Cys Thr Arg Ser Pro Ile Tyr Phe Asp Asn Trp Tyr Phe Asp Val
            100                 105                 110

Trp Gly Thr Gly Thr Thr Val Thr Val Ser Ser
        115                 120

<210>  82
<211>  369
<212>  DNA
<213>  Artificial Sequence

<400>  82
gaggtgaaac tggtggagtc tggaggaggc ttggtacagc ctgggggttc tctgagactc      60

tcctgtgcag cttctggatt caccttcact gattactaca tgagctgggt ccgccagcct     120

ccagggaagg cacttgagtg gttgggtttt attagaaaca aagctgatgg ttccacaaca     180

gagtacagtg catctgtgaa gggtcggttc accatctcca gagataattc ccaaagcatc     240

ctctatcttc aaatgaatgc cctgagacct gaggacagtg ccacttatta ctgtacaaga     300

tcccctatct actttgataa ctggtacttc gatgtctggg gcacagggac cacggtcacc     360

gtctcctca                                                             369


<210>  83
<211>  5
<212>  PRT
<213>  Artificial Sequence

<400>  83

Asp Tyr Tyr Met Ser
1               5


<210>  84
<211>  19
<212>  PRT
<213>  Artificial Sequence

<400>  84

Phe Ile Arg Asn Lys Ala Asp Gly Ser Thr Thr Glu Tyr Ser Ala Ser
1               5                   10                  15


Val Lys Gly



<210>  85
<211>  12
<212>  PRT
<213>  Artificial Sequence

<400>  85

Ser Pro Ile Tyr Phe Asp Asn Trp Tyr Phe Asp Val
1               5                   10


<210>  86
<211>  113
<212>  PRT
<213>  Artificial Sequence

<400>  86

Asp Ile Val Met Ser Gln Ser Pro Ser Ser Leu Ala Val Ser Val Gly
1               5                   10                  15

Glu Lys Val Thr Met Thr Cys Lys Ser Ser Gln Ser Leu Leu Tyr Ser
            20                  25                  30

Asn Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Leu Ser Ser Val Lys Ala Glu Asp Leu Ala Val Tyr Tyr Cys Gln Gln
                85                  90                  95

Tyr Tyr Ser Tyr Pro Phe Thr Phe Gly Ser Gly Thr Lys Leu Glu Ile
            100                 105                 110

Lys

<210>  87
<211>  339
<212>  DNA
<213>  Artificial Sequence

<400>  87
gacattgtga tgtcacagtc tccatcctcc ctagctgtgt cagttggaga gaaggttact      60

atgacctgca agtccagtca gagcctttta tatagtaaca atcaaaagaa ctacttggcc     120

tggtaccagc aaaaaccagg gcagtctcct aaactgctga tttactgggc atccactagg     180

gaatctgggg tccctgatcg cttcacaggc agtggatctg ggacagattt cactctcacc     240

ctcagcagtg tgaaggctga agacctggca gtttattact gtcagcaata ttatagctat     300

ccattcacgt tcggctcggg gacaaagttg gaaataaaa                            339

<210>  88
<211>  17
<212>  PRT
<213>  Artificial Sequence

<400>  88

Lys Ser Ser Gln Ser Leu Leu Tyr Ser Asn Asn Gln Lys Asn Tyr Leu
1               5                   10                  15

Ala

<210>  89
<211>  7
<212>  PRT
<213>  Artificial Sequence

<400>  89

Trp Ala Ser Thr Arg Glu Ser
1               5

<210>  90
<211>  9
<212>  PRT
<213>  Artificial Sequence

<400>  90

Gln Gln Tyr Tyr Ser Tyr Pro Phe Thr
1               5

<210>  91
<211>  117
<212>  PRT
<213>  Artificial Sequence

<400>  91

Glu Val Gln Leu Gln Gln Ser Gly Pro Glu Leu Val Lys Pro Gly Ala
1               5                   10                  15

Ser Val Lys Ile Ser Cys Lys Ala Ser Gly Tyr Thr Phe Thr Asp Tyr
            20                  25                  30

Tyr Met Asn Trp Val Lys Gln Ser His Gly Lys Ser Leu Glu Trp Ile
        35                  40                  45

Gly Asn Ile Asn Pro Asn Asn Gly Asp Thr Asn Tyr Asn Gln Lys Phe
    50                  55                  60

Lys Gly Lys Ala Thr Leu Thr Val Asp Lys Ser Ser Ser Thr Ala Ser
65                  70                  75                  80

Met Glu Leu Arg Ser Leu Thr Ser Glu Asp Ser Ala Val Tyr Tyr Cys
                85                  90                  95

Ala Asn Ser Gly Tyr Tyr Tyr Phe Asp Tyr Trp Gly Gln Gly Thr Thr
            100                 105                 110

Leu Thr Val Ser Ser
        115

<210>  92
<211>  351
<212>  DNA
<213>  Artificial Sequence

<400>  92
gaggtccagc tgcaacaatc tggacctgag ctggtgaagc ctggggcttc agtgaagata      60

tcctgtaagg cttctggata cacgttcact gactactaca tgaactgggt gaaacagagc     120

catggaaaga gccttgagtg gattggaaat attaatccta acaatggtga tactaactac     180

aaccagaagt tcaagggcaa ggccacattg actgtagaca agtcctccag cacagcctcc     240

atggagctcc gcagcctgac atctgaggac tctgcagtct attactgtgc aaattcaggc     300

tactattact ttgactactg gggccaaggc accactctca cagtctcctc a              351

<210>  93
<211>  5
<212>  PRT
<213>  Artificial Sequence

<400>  93

Asp Tyr Tyr Met Asn
1               5

<210>  94
<211>  17
<212>  PRT
<213>  Artificial Sequence

<400>  94

Asn Ile Asn Pro Asn Asn Gly Asp Thr Asn Tyr Asn Gln Lys Phe Lys
1               5                   10                  15

Gly

<210>  95
<211>  10
<212>  PRT
<213>  Artificial Sequence

<400>  95

Ala Asn Ser Gly Tyr Tyr Tyr Phe Asp Tyr
1               5                   10

<210>  96
<211>  113
<212>  PRT

<213>  Artificial Sequence

<400>  96

Asp Ile Val Met Thr Gln Ser Pro Ser Ser Leu Thr Val Thr Ala Gly
1               5                   10                  15

Glu Lys Val Thr Met Ser Cys Lys Ser Ser Gln Ser Leu Leu Asn Ser
            20                  25                  30

Gly Asn Gln Lys Asn Tyr Leu Thr Trp Tyr Gln Gln Lys Pro Gly Gln
        35                  40                  45

Pro Pro Lys Val Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Asp Tyr Ser Tyr Pro Pro Thr Phe Gly Gly Gly Thr Asn Leu Glu Ile
            100                 105                 110

Lys

<210>  97
<211>  339
<212>  DNA
<213>  Artificial Sequence

<400>  97
gacattgtga tgacacagtc tccatcctcc ctgaccgtga cagcaggaga gaaggtcact      60

atgagctgca agtccagtca gagtctgtta aacagtggaa atcaaaagaa ctacttgacc     120

tggtaccagc agaaaccagg gcagcctcct aaagtattga tctactgggc atccactagg     180

gaatctgggg tccctgatcg cttcacaggc agtggatctg gaacagattt cactctcacc     240

atcagcagtg tgcaggctga agacctggca gtttattact gtcagaatga ttatagttat     300

cctcccacgt tcggaggggg gaccaacctg gaaataaaa                            339

<210>  98
<211>  17
<212>  PRT
<213>  Artificial Sequence

<400>  98

Lys Ser Ser Gln Ser Leu Leu Asn Ser Gly Asn Gln Lys Asn Tyr Leu

1               5                   10                  15

Thr

<210>  99
<211>  7
<212>  PRT
<213>  Artificial Sequence

<400>  99

Trp Ala Ser Thr Arg Glu Ser
1               5

<210>  100
<211>  9
<212>  PRT
<213>  Artificial Sequence

<400>  100

Gln Asn Asp Tyr Ser Tyr Pro Pro Thr
1               5

<210>  101
<211>  117
<212>  PRT
<213>  Artificial Sequence

<220>
<223>  Hu030-2 VH amino acid sequence

<400>  101

Gln Val Gln Leu Gln Glu Ser Gly Pro Gly Leu Val Lys Pro Ser Glu
1               5                   10                  15

Thr Leu Ser Leu Thr Cys Thr Val Ser Gly Tyr Ser Ile Thr Ser Arg
            20                  25                  30

Tyr Tyr Trp Asn Trp Ile Arg Gln Pro Pro Gly Lys Gly Leu Glu Trp
        35                  40                  45

Ile Gly Tyr Ile Thr Tyr Asp Asp Asn Asn Asn Tyr Asn Pro Ser Leu
    50                  55                  60

Lys Asn Arg Val Thr Ile Ser Arg Asp Thr Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Gly Gly Trp Asp Pro Phe Leu Tyr Trp Gly Gln Gly Thr Leu
            100                 105                 110

Val Thr Val Ser Ser
        115

<210>  102
<211>  351
<212>  DNA
<213>  Artificial Sequence

<220>
<223>  Hu030-2 VH nucleotide sequence

<400>  102
caagttcagc tgcaagaaag cggacccggt ttagtgaaac ccagcgagac tttatcttta      60

acttgtaccg tgagcggcta cagcatcacc tctcgttact actggaactg gatcagacag     120

ccccccggca aaggtttaga gtggatcggc tacatcacct acgacgacaa caacaactac     180

aacccctctt taaagaatcg tgtgaccatc tctcgtgaca ccagcaagaa ccagttctct     240

ttaaagctgt cctccgtgac cgctgccgat accgccgtgt actactgtgc tcgtggcggc     300

tgggatccct ttttatactg gggccaaggt acactggtga ccgtgagcag c              351

<210>  103
<211>  107
<212>  PRT
<213>  Artificial Sequence

<220>
<223>  Hu030-2 VL amino acid sequence

<400>  103

Asp Ile Gln Met Thr Gln Ser Pro Ser Ser Leu Ser Ala Ser Val Gly
1               5                   10                  15

Asp Arg Val Thr Ile Thr Cys Arg Ala Ser Asp Asn Ile Tyr Ser Tyr
            20                  25                  30

Leu Ala Trp Tyr Gln Gln Lys Pro Gly Lys Ala Pro Lys Leu Leu Ile
        35                  40                  45

Tyr Ser Ala Lys Asn Leu Gly Glu Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln His Tyr Tyr Gly Thr Pro Phe
                85                  90                  95

Thr Phe Gly Gly Gly Thr Lys Val Glu Ile Lys
            100                 105

<210>  104
<211>  321
<212>  DNA
<213>  Artificial Sequence

<220>
<223>  Hu030-2 VL nucleotide sequence

<400>  104
gacatccaga tgacccagag ccctagctct ttaagcgcca gcgtgggcga tagggtgacc      60

atcacttgtc gtgccagcga caacatctac agctatttag cttggtacca gcagaagccc     120

ggcaaggccc ccaagctgct gatctacagc gccaagaatt taggcgaggg cgtgcctagc     180

agattcagcg gaagcggcag cggcaccgac ttcactttaa ccatcagctc tttacagccc     240

gaggacttcg ccacctacta ctgccagcac tactacggca cccctttcac cttcggcggc     300

ggcaccaagg tggagatcaa g                                               321